(19) Europäisches Patentamt
European Patent Office
Office européen des brevets

(11) EP 1 447 444 A1

(12) EUROPEAN PATENT APPLICATION
published in accordance with Art. 158(3) EPC

(43) Date of publication:
**18.08.2004 Bulletin 2004/34**

(21) Application number: **02777889.3**

(22) Date of filing: **18.10.2002**

(51) Int Cl.[7]: **C12N 15/00**, G06F 17/30

(86) International application number:
**PCT/JP2002/010851**

(87) International publication number:
**WO 2003/035863 (01.05.2003 Gazette 2003/18)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **19.10.2001 JP 2001322896**

(71) Applicant: **KYOWA HAKKO KOGYO CO., LTD.**
**Chiyoda-ku, Tokyo 100-8185 (JP)**

(72) Inventor: **NISHI, Tatsunari**
**Ohta-ku, Tokyo 145-0074 (JP)**

(74) Representative: **Casalonga, Axel**
**Bureau D.A. Casalonga - Josse,**
**Paul-Heyse-Strasse 33**
**80336 München (DE)**

# (54) METHOD OF IDENTIFYING PROKARYOTIC GENE STRUCTURE

(57) A method of determining a genetic structure which includes a process of, after having predicted coding regions which create a transcription unit, proceeding to determine a translation start codon; a method of determining a genetic structure which includes a process of selecting a plurality of pairs of codons of which the difference between the appearance frequencies and those of the codons which have the reverse complementary sequence within the nucleotide sequences of a plurality of coding regions which have already been determined is great, and of deciding that those coding regions which have a large number of codons for which the frequency at which each pair appears is high are true coding regions; a method of determining a genetic structure where the GC content of a nucleotide sequence exceeds 50%, including a process of deciding as false one for which the first and the third GC content of the codons within a coding region are less than a predetermined value; a program for performing these; a recording medium which can be read in by a computer, upon which this program has been recorded; and furthermore a genetic structure determination system which is based upon a computer which executes this program.

EP 1 447 444 A1

## Description

Technical Field

The present invention relates to a method of determining a genetic structure based upon a nucleotide sequence of a prokaryote (nucleotide sequence means a sequence of DNA or of RNA), to a program for executing the method, to a computer-readable recording medium on which the program is recorded, and to a system for determining a genetic structure based upon a computer holding the recording medium.

Background Art

Various microbes and various enzymes which are produced by the various microbes are utilized in wide fields of industry, and there is a great demand to improve these microbes and to discover new enzymes. The decoding of the nucleotide sequence information of living organisms has accelerated due to the progress of the automatic fluorescence sequencers in the latter part of the 1980's, and the study of genomes has been greatly made. Since the entire genome sequence of a bacterium (Haemophilus influenza) was determined for the first time in 1995, at the present time the entire genome sequences of about 50 varieties of microbes have been determined. Furthermore, there are about 200 varieties of microbes whose genome sequence is being determined at the present time, so that the genome sequences of more than 250 varieties of microbes are being clarified soon. The field of microbial biotechnology is breaking through into the post-genome era. In promotion of studies utilizing a massive amount of genetic information, the development of technology for analyzing colossal amounts of microbial genome information at high accuracy and at high speed has become a great demand for research and development activities.

A first step in the decoding of genome information is to determine a genetic structure from the determined nucleotide sequence information. If it is possible to determine the genetic structure, in particular the position of the coding regions (Coding sequence: CDS) (hereinafter a coding region of a gene is referred to as a coding region), then it becomes possible to predict functions of the gene products, since it is possible to predict their amino acid sequences. Furthermore, if it is possible to predict the structure of a transcription unit such as a polycistron, then it is possible to predict an expression control mechanism for a group of genes which are upon the same transcription unit. Methods of predicting the genetic structure from the nucleotide sequence information are basic and important techniques, and various methods have been developed up till now. When predicting a genetic structure from nucleotide sequence information using a computer, if the total number of "correct" structures, in other words "true" structures, is termed $N_T$, the total number of structures predicted by the computer is termed $N_S$, and the number of structures which are predicted by the computer and identical with "correct" structures, in other words "true" structures, is termed $N_{TP}$, then $N_{TP}/N_T$ is termed the sensitivity, and $N_{TP}/N_S$ is termed the specificity. The closer both the sensitivity and the specificity approach to a numerical value "1", the more excellent is the performance of the program considered to be. Accordingly, it is a great demand for development of a program which exhibits excellent performance in both sensitivity and specificity.

The characteristics of the coding regions of a prokaryote were extracted by using a stochastic process such as Markov model and the like, and programs for determining the coding regions by using these extracted characteristics were developed, for example, GenMark [Borodovsky, M. & McIninch, J.: Computers Chem. Vol. 17, 123-133 (1993)], GenMark.hmm [Lukashin, A.V. & Borodovsky, M.: Nucleic Acids Research, Vol. 26, 1107-1115 (1998), and Besemer, J. & Borodovsky, M.: Nucleic Acids Research Vol. 27, 3911-3920 (1999)], and Glimmer [Salzberg, S. et al.: Nucleic Acids Research Vol. 26, p. 544-548 (1998), and Delcher, A.L. et al.: Nucleic Acids Research Vol. 27, p. 4636-4641 (1999)], and the like. Among these, Glimmer is the most widely used program in the world. Furthermore, the programs CRITICA [Badger, J.H. & Losen, G.J. et al.: Mol. Biol. Evol. Vol. 16, p. 512-524 (1999)] which determines the coding regions based upon homology analysis, and ORPHELUS [Frishman, M. et al.: Nucleic Acids Research Vol. 26, p. 2941-2947 (1998)] which determines the coding regions based upon the existence of ribosome binding sequences and codon usage analysis, were developed. However, the accuracy of coding region determination of these programs is not sufficiently high, and it is desired to develop a technique for determining coding regions with higher accuracy. Furthermore, very recently, the program GenMarkS [Besemer, J., Lomsadze, A. & Borodovsky, M.: Nucleic Acids Research Vol. 29, 2607-2618 (2001)], which determines the coding regions at high accuracy by enhancing the accuracy of determination of the translation start codons, was also developed. By combination with the above described GenMark.hmm, in the determination of the coding regions from 8 microbal genomes, this program has an accuracy with sensitivity of 0.969 or more and specificity of 0.865 or more. However, a program whose accuracy in specificity is even higher is required, and also there is a great demand of development of a program with high accuracy in both sensitivity and specificity as a single program.

Up till now the living organism which has been analyzed in the greatest detail by various experimental methods is Escherichia coli K-12 strain, the American study group and the Japanese study group who analyzed the Escherichia coli genome respectively announced its total number of CDSs to be 4289 and 4359. When the Escherichia coli genome is analyzed using the CDS determination program Glimmer which is most often utilized, it is possible to find about 4158 of the coding regions from 4289 CDSs. On the other hand, the total number of CDSs predicted by Glimmer is 5026, which greatly differs from 4289 [Delcher, A.L. et al.: Nucleic Acids Research Vol. 27, p. 4636-4641 (1999)]. Also, in the actual process of annotation of the microbial genome sequence, it takes a long time to examine the coding regions closely which are tentatively determined by using these CDS determination programs to determine a genetic structure. In this process of annotation, there is a strong demand for the development of a technique which has higher accuracy and can shorten the time for annotation.

The existing programs for determination of the coding regions for prokaryotes have the problem that the accuracy of determination of the coding regions is insufficient in practice, since the accuracy of prediction of the position of the translation start codon (hereinafter referred to as the start codon) is low, and the accuracy of determination of the coding regions is low. Existing programs also have the problem that they can not predict the structure of the polycistronic transcription units. Furthermore, when existing programs for determination of the coding regions are utilized, a preprocess is required before the execution of the programs for determining the coding regions, since correct coding regions are determined in advance by homology analysis and the like, and the programs use a method for prediction based upon the information about these coding regions. Due to this, they have the problem that processes are complicated and the time required to determine the coding regions is long. Furthermore, most of the programs for determining the coding regions which have been developed up till now have the problem that the accuracy of the determination of the coding regions become low when the coding regions are predicted from a nucleotide sequence in which the content of G residues and C residues (hereinafter abbreviated as "GC content") is high. Even the combination of the two previously described programs GenMarkS and GenMark.hmm can not determine the coding regions at high accuracy from a microbial genome with the high (for example, 65% or more) GC content.

In order to show these problems specifically, the genetic structure of a prokaryote is explained using FIGs. 1 and 2 (refer to FIGs. 1 and 2). As shown in FIG. 1, the basic structure of a prokaryote gene consists of a promoter which start the synthesis of mRNA, a ribosome binding site which participate in the binding between mRNA and ribosomes and in the translation initiation, a start codon, a translation stop codon (hereinafter referred to as a stop codon), and a terminator which terminate the synthesis of mRNA. AUG codon is the most appropriate as a start codon, and next GUG codon is used. UUG codon is used rarely. It was reported that CUG codon is used very rarely.

Since the start codons and coding regions are determined usually based upon a DNA sequence, in the present specification, the sequences of start codons and stop codons and sequences involved in the binding of ribosomes and mRNA are expressed as DNA sequences appropriately as well as RNA sequences, unless mentioned specifically. Ribosome binding site is also called Shine Dalgarno sequence (hereinafter abbreviated as SD sequence), and generally has a sequence complementary to the 3' terminal of 16S rRNA. In particular, the SD sequence which appears at high frequency is AGGAGG or AAGGAGG (hereinafter these sequences are referred to as "consensus SD sequences"), or a sequence homologous with "consensus SD sequence". Although these sequences appear at various sites of genes, it is understood that the SD sequences appear at high frequency in regions upstream of start codons. In the present specification, in order to specify a "position upstream of a start codon" clearly, the distance between AGGA (or a sequence which positionally identical with AGGA) within a consensus SD sequence (AGGAGG or AAGGAGG) and a start codon (hereinafter, referred to as the distance between SD-ATG) is used in the following description. It is known that the distance between SD-ATG exerts a great influence upon the translation starting efficiency of genes [Shepard, H.M. et al.: DNA Vol. 2, p. 125-131 (1982), Itoh, S. et al.: DNA Vol. 2, p. 157-165 (1982)].

Next, as shown in FIG. 2, many reading frames of translation which have possibility to encode proteins (Open Reading Frames: hereinafter abbreviated as "ORF"s) are present. In the present specification, a reading frame of translation which has possibility to encode a protein is termed "ORF", and, among the ORFs, a coding region from which a protein is actually translated is termed "CDS" (Coding Sequence). Furthermore, individual ORF, CDS, coding region or transcription unit is expressed as ORF-1, ORF-AN ORF-B, CDS-1, CDS-A, CDS-B, coding region A, coding region B, transcription unit P, transcription unit Q, and the like, wherein a number or symbol is added respectively. In the present specification, ORF means the longest ORF in length in many cases. In the present specification, as shown in FIG. 2, a DNA strand on the upper side on the figure, in other words a DNA strand upon which genes translated rightwards are present tandemly, is termed a plus strand, on the other hand, a DNA strand on the lower side of the figure is termed a minus strand.

In many cases, as shown in FIG. 2, a CDS (a region encoding a true protein) is identical with an ORF, or is a region which is a portion of an ORF. Furthermore, many candidates for start codons also exist, and it is not easy to determine the true start codon. In the case of the genes of a prokaryote, a plurality of CDSs exists upon the same mRNA, and moreover the individual CDSs, in other words cistrons, are adjacent and form a linked structure. This structure of the transcription unit is termed "polycistronic mRNA". It is difficult to predict the transcription unit structure of the polycistrons with approaches of information science, and no program for determination of translation which can predict the transcription units with good efficiency and can output the results thereof has been developed. Furthermore, if the CDS or the transcription unit overlaps with other CDS or transcription unit, or contains other CDS or transcription unit (in the present specification, expressed as "include" or "be in an inclusion relationship"), it is necessary to decide on the truth or the falsity of each of the CDSs or transcription units.

As described above, there is the problem that it is difficult to determine the correct CDSs for the given nucleotide sequence information, due to reasons such as "the existence of many candidates for CDS", "the existence of many candidates for the start codon", and "the overlapping of two or more candidates for CDS".

DISCLOSURE OF THE INVENTION

In order to solve the above described problems, the present invention is made, wherein its goal is to develop a method of determining a genetic structure with enhanced accuracy, of which the chief aims are: to make it possible to predict the structure of polycistronic transcription units; to enhance the accuracy of determination of the positions of start codons; to require no necessity for providing information in advance; and to handle nucleotide sequences whose GC content is high. Thus, the objective of the present invention is to provide a method of determining a genetic structure of a prokaryote, which achieves these aims, a program for executing the method, a computer-readable recording medium on which the program is recorded, and a system for determining a genetic structure based upon a computer holding the recording medium.

A method of determining a genetic structure of the present invention is directed to a method of determining at least one of the following members: a coding region, the position of a translation start codon, the position of a translation stop codon, and a transcription unit.

The present inventor has principally found a "method of determining a genetic structure from a viewpoint of a transcription unit structure", a "method of determining a genetic structure using a shadow discrimination function", and a "method of determining a genetic structure from a viewpoint of the GC content of the bases in the codons", has produced programs for executing these methods on a computer, and has found that it is possible to attain these goals by executing the programs.

In other words, the present invention is a "method of determining a genetic structure from the viewpoint of a transcription unit structure" according to (1) - (6) below.

(1) A method of determining a genetic structure of a prokaryote, which comprises the steps (a) to (g) described below:

(a) setting a translation stop codon from information about the nucleotide sequence of a prokaryote (a nucleotide sequence is a sequence of DNA or RNA), and setting a provisional translation start codon which yields the longest open reading frame (hereinafter abbreviated as ORF) based upon said translation stop codon;

(b) deciding that the ORF-A and the ORF-B have a possibility to form a single transcription unit if the provisional start codon of the ORF-A is upstream of the translation stop codon of the ORF-B, or is within $D_S$ bases downstream of said translation stop codon [herein $D_S$ is an integer from 20 to 100], wherein any two neighboring ORFs which are obtained in the step (a) and present on the same strand are termed ORF-A and ORF-B from downstream;

(c) determining that the candidate for the translation start codon is the translation start codon of ORF-A if the ORF-A and the ORF-B are decided to have a possibility to form a single transcription unit in the step (b) and if the candidate for the translation start codon is present within a region (hereinafter termed the "vicinity of the translation stop codon") between $D_B$ bases downstream from the first T (thymidine) residue of the translation stop codon of the ORF-B and $U_B$ bases upstream from said T residue [herein $D_B$ is an integer between 10 and 20, and $U_B$ is an integer between 3 and 15], and determining the translation start codon of the ORF-A from a priority ranking determined by using the distance between each candidate and the translation stop codon of the ORF-B as an indicator if there is a plurality of candidates;

(d) examining whether a candidate for the translation start codon of the ORF-A is present within a region (hereinafter termed the "region around the vicinity of the translation stop codon") between $R_D$ bases downstream from the first T residue of the translation stop codon of the ORF-B and $R_U$ bases upstream from said T residue and excluding said "vicinity of the translation stop codon" [herein $R_D$ is an integer from 30 to 120, and $R_U$ is an integer from 20 to 120] if the translation start codon of the ORF-A can not be determined in the step (c);

(e) examining whether a ribosome binding site is present from 1 to 30 bases upstream of a candidate for the translation start codon of the ORF-A if the candidate is present in the region around the vicinity of the translation stop codon in the step (d), determining its ribosome binding sequence if such a ribosome binding site is present, and determining that the candidate which corresponds to said ribosome binding sequence is the translation start codon of the ORF-A;

(f) searching for up to the number N of candidates for the translation start codon including the provisional start codon which yields the longest ORF from the 5' terminal of an ORF-A which is not decided to have a possibility to form a single transcription unit in the step (b) or whose translation start codon is not determined in the step (e), investigating whether a ribosome binding site is present from 1 to 30 bases upstream of each candidate, determining its ribosome binding sequence if such a ribosome binding site is present, and determining that the candidate which corresponds to said ribosome binding sequence is the translation start codon [herein N is an integer from 5 to 20];
(g) confirming the positions of the translation start codon and the translation stop codon, the coding region, and the transcription units from the results of determination by the step (c), the step (e) or the step (f) to determine a genetic structure.

(2) The method of determining a genetic structure according to (1), wherein the step (e) is a step of determining the translation start codon of an ORF-A by the following steps:

determining that a mRNA sequence whose ribosome binding score exceeds a threshold value $V_3$, described below, is a ribosome binding sequence [herein $V_3$ is an integer from 4 to 12], wherein the paired state between a mRNA sequence of 4 to 17 bases upstream of a candidate for the translation start codon of the ORF-A and a sequence (3'-UUCCUCC-5') involved in the binding to mRNA in a 16S rRNA 3' terminal sequence, or between a mRNA sequence of 4 to 16 bases upstream of said candidate and a sequence (3'-UCCUCC-5') involved in the binding to mRNA in a 16S rRNA 3' terminal sequence, is expressed as a numerical value, which is termed a "score which shows the binding state between mRNA and a ribosome" (hereinafter termed a ribosome binding score), according to the four rules described below:

(i) A pairing of G and C yields +4;
(ii) A pairing of A and U yields +2;
(iii) A pairing of G and U yields +1;
(iv) When no pairing is present at a base pair which is adjacent to a base pair where a pairing is present, then this yields -1;

determining that the candidate which corresponds to said ribosome binding sequence is the translation start codon;
dividing the "region of an ORF-B around the vicinity of the stop codon" into the two of "the region downstream of said vicinity" and "the region upstream of said vicinity" if there is a plurality of said translation start codons, and
determining the one of said translation start codons which has the highest priority is the true translation start codon based on the priority of "the region downstream of said vicinity" and "the region upstream of said vicinity" in that order;
determining the translation stop codon of the ORF-A from a priority ranking defined by using the distance from the translation stop codon of the ORF-B as an indicator if a plurality of translation start codons is present within the respective regions.

(3) The method of determining a genetic structure according to (1) or (2), wherein the step (f) is a step of determining the translation start codon of an ORF-A by the following steps:

determining that the mRNA sequence whose ribosome binding score exceeds a threshold value V, described below,$_1$ is a ribosome binding sequence, wherein the paired state between a mRNA sequence of from 4 to 17 bases upstream of a candidate for the translation start codon of the ORF-A and a sequence (3'-UUCCUCC-5') involved in the binding to mRNA in a 16S rRNA 3' terminal sequence, or between a mRNA sequence of 4 to 16 bases upstream of said candidate and a sequence (3'-UCCUCC-5') involved in the binding to mRNA in a 16S rRNA 3' terminal sequence, is expressed as a numerical value, termed "ribosome binding score", according to the four rules described below:

(i) A pairing of G and C yields +4;
(ii) A pairing of A and U yields +2;
(iii) A pairing of G and U yields +1;
(iv) When no pairing is present at a base pair which is adjacent to a base pair where a pairing is present, then this yields -1;

determining that the candidate which corresponds to said ribosome binding sequence is the translation start codon;
determining that the translation start codon corresponding to the ribosome binding sequence which yields the highest score is the true translation start codon if there is a plurality of said translation start codons;
setting one or more threshold value(s) smaller than $V_1$, which include the threshold value $V_3$, if there is no candidate which exceeds the threshold value $V_1$, and
determining the translation start codon of the ORF-A in a stepwise manner if said threshold value is exceeded [herein $V_1$ is an integer which is greater than the $V_3$ of (2), and which is between 7 and 14].

(4) The method of determining a genetic structure according to (2) or (3), wherein the "ribosome binding score" is calculated by deducting a numerical value $P_G$ if the translation start codon is GTG, or by deducting a numerical value $P_T$ if the translation start codon is TTG [herein $P_G$ is an integer from 1 to 4, and $P_T$ is an integer from 2 to 6].
(5) A method of determining a genetic structure, wherein a transcription unit P, a coding region A, a transcription unit Q, and a coding region B is determined by utilizing the method according to any one of (1) to (4), which further comprises the steps (h) to (j) described below if the transcription unit P or the coding region A overlaps with the transcription unit Q or the coding region B:

(h) deciding that the transcription unit Q or the coding region B is a "false transcription unit" or a "false coding region" if a transcription unit Q or a coding region B which is present upon the same strand as a transcription unit P or a coding region A is included in the transcription unit P or the coding region A;
(i) deciding that the transcription unit Q or the coding region B is a "false transcription unit" or a "false coding region" if a transcription unit Q or a coding region B which is present upon the complementary strand to a transcription unit P or a coding region A is included in the transcription unit P or the coding region A;
(j) deciding that the transcription unit or coding region whose length is shorter is a "false transcription unit" or a "false coding region" when a transcription unit P or a coding region A overlaps with a transcription unit Q or a coding region B which is present upon the complementary strand.

(6) A method of determining a genetic structure, wherein the method of determining a genetic structure according to any one of (1) to (5) is utilized repeatedly.
Furthermore, the present invention is a "method of determining a genetic structure using a shadow discrimination function" as described in (7) - (11) below.
(7) A method of determining a genetic structure of a prokaryote, which comprises the steps (k) and (l) described below:

(k) selecting k types of combination of codons wherein "the frequency of appearance of one codon is high and the frequency of appearance of a codon which has the complementary sequence to the 3-base sequence of said codon is low" in a plurality (the number T) of determined coding regions of the prokaryote;
(l) comparing the "number of times of the k types of codons whose frequency of appearance is high appearing in a coding region A which is assumed to be a coding region" with the "number of times of the k types of codons whose frequency of appearance is low appearing in said coding region A", and deciding on the truth or falsity of said coding region A [herein k is an integer greater than or equal to 5 and less than or equal to 20].

(8) The method of determining a genetic structure according to (7), wherein the method for comparing the "number of times of the k types of codons whose frequency of appearance is high appearing in a coding region A which is assumed to be a coding region" with the "number of times of the k types of codons whose frequency of appearance is low appearing in said coding region A" is a method which involves using "the reciprocal of the sum of 1 and the ratio of the number of the latter to the number of the former" as a calculation formula and which involves deciding that said coding region A is a "false coding region" if the value of said reciprocal is less than a fixed value.
(9) The method of determining a genetic structure according to (7), which is based on the nucleotide sequence of the number T of determined coding regions of the prokaryote and comprises the steps (m) to (p) described below:

(m) arranging the 64 types of codons so that the 3-base sequence of the i-th codon has the complementary sequence to the nucleotide sequence of the (i+32)-th codon;
(n) obtaining $y_i$ from the formula (2) below and $y_{i+32}$ from the formula (3) below:

$$y_i = \left(\sum_{t=1}^{T} C_i^t - \sum_{t=1}^{T} C_{i+32}^t\right) \Big/ \sum_{t=1}^{T}\sum_{j=1}^{64} C_j^t \qquad (2)$$

$$y_{i+32} = \left(\sum_{t=1}^{T} C_{i+32}^t - \sum_{t=1}^{T} C_i^t\right) \Big/ \sum_{t=1}^{T}\sum_{j=1}^{64} C_j^t \qquad (3)$$

wherein the number of appearances of the i-th codon in the t-th coding region is expressed as $C_j^t$

(o) rearranging the 64 types of codon in the step (m) in descending order of the $y_i$ and the $y_{i+32}$, selecting top k types of codons for which the value of $y_i$ or of $y_{i+32}$ is large, and obtaining the value of $Sd_A$ for a coding region A by the following formula (4):

$$Sd_A = 2 \times \sum_{i=1}^{k} C_i^A \Big/ \left(\sum_{i=1}^{k} C_i^A + \sum_{i=65-k}^{64} C_i^A\right) \qquad (4)$$

[herein the value of $Sd_A$ is defined as 1 if

$$\left(\sum_{i=1}^{k} C_i^A + \sum_{i=65-k}^{64} C_i^A\right)$$

is zero].

(p) deciding that a coding region A is a true coding region if the value of $Sd_A$ of said coding region calculated in the process (o) is greater than or equal to a threshold value $S_1$, and that it is a false coding region if said value of $Sd_A$ is less than the threshold value $S_1$ [herein T is an integer greater than or equal to 2, i is a positive integer less than or equal to 32, j is a positive integer less than or equal to 64, t is a positive integer less than or equal to T, k is an integer from 5 to 20, and $S_1$ is a value from 0.8 to 1.8].

(10) A method of determining a genetic structure of a prokaryote, which comprises the steps (q) and (r) described below, wherein a coding region of the prokaryote or a coding region A which is assumed to be a coding region overlaps with a coding region B which is assumed to be a coding region and present upon the complementary strand, and said coding region B is included in said coding region A:

(q) comparing the length $L_B$ (in base pairs) of said coding region B with the length $L_A$ (in base pairs) of said coding region A, and deciding that said coding region B is a "false coding region" if $L_B$ is less than or equal to $T_P$% of $L_A$;

(r) deciding on the truth or falsity of said coding region A and of said coding region B by the method according to any one of (7) to (9) if $L_B$ exceeds $T_P$% of $L_A$ [herein, $T_P$ is a positive integer from 30 to 95].

(11) A method of determining a genetic structure, characterized by removing the translation stop codons from the coding regions which form a transcription unit, and linking up the resulting coding regions into a single coding region, before utilizing the method according to any one of (7) to (10).

Furthermore, the present invention, as specified by (12) below, enhances the accuracy of determination of the coding regions of a prokaryote by combining a "method of determining a genetic structure from the viewpoint of a transcription unit structure" and a "method of determining a genetic structure using a shadow discrimination function".

12) A method of determining a genetic structure, which comprises:

deciding on the truth or falsity of a coding region or of a transcription unit which is determined by the method of determining a genetic structure according to any one of (1) to (6), by utilizing the method of determining a genetic structure according to any one of (7) to (11).

Furthermore, the present invention is specified by (13) below.
(13) A method of determining a genetic structure, which comprises:

deciding on the truth or falsity of a coding region which encodes a polypeptide of $L_M$ amino acids or more in length, by using the method of determining a genetic structure according to any one of (7) to (12), based on the nucleotide sequence of a coding region which is determined by using the method of determining a genetic structure according to any one of (1) to(12) and which encodes a polypeptide of $L_F$ amino acids or more in length [herein $L_F$ is a positive integer greater than or equal to 100, and $L_M$ is a positive integer greater than or equal to 20].

Furthermore, the present invention is a "method of determining a genetic structure from the viewpoint of the GC content of the bases in the codons", as shown in (14) to (18) below.
(14) A method of determining a genetic structure of a prokaryote, characterized by deciding that a coding region in the nucleotide sequence of the prokaryote is a "false coding region" if the GC content of said nucleotide sequence is greater than 50% and if a content, calculated by utilizing a calculation formula which yields a content of the first and third G residues and C residues of the codons in said nucleotide sequence, is less than a fixed value.
(15) The method of determining a genetic structure according to (14), wherein the following formula (5) is used as a calculation formula, the value of $GC_i$ described below is used as a calculated content, and one value which is selected from 0.6 to 0.75 is used as a fixed value:

$$GC_i = (y^{i(1)} + y^{i(3)}) \Big/ \sum_{r=1}^{3} y^{i(r)} \quad \text{wherein} \quad y^{i(r)} = \sum_{n=1}^{N_i} \sum_{b=1}^{4} x_{n(b)}^{i(r)} \qquad (5)$$

[herein when the r-th base (r is 1, 2, or 3) of the n-th codon of the i-th coding region is b (b is 1, 2, 3, or 4), then

$$\chi_{n|b|}^{i|r|} \text{ is } \chi_{n|b|}^{i|r|} = 1 \text{ (b= 1 or 2)}$$

$$\chi_{n|b|}^{i|r|} = 0 \text{ (b= 3 or 4)}$$

and, as for b, when the r-th base of the n-th codon of the i-th coding region is G, C, A, or T, b is 1, 2, 3, or 4, respectively, i and n are positive integers, and $N_i$ denotes the total number of the codons (excluding the translation stop codon) of the i-th coding region].
(16) A method of determining a genetic structure of a prokaryote, which comprises:

deciding that a coding region in the nucleotide sequence of the prokaryote is a "false coding region" if the GC content of said nucleotide sequence is greater than 50%, and if a content, calculated by utilizing a calculation formula which yields a content of the first and third G residues and C residues of the codons in said nucleotide sequence, is less than a fixed value; and
re-searching for a translation start codon which is present downstream of said translation start codon which is decided to be false.

(17) The method of determining a genetic structure according to (16), wherein the following formula (5) is used as a calculation formula, the value of $GC_i$ described below is used as a calculated content, and one value which is selected from 0.6 to 0.75 is used as a fixed value:

$$GC_i = (y^{i(1)} + y^{i(3)}) \Big/ \sum_{r=1}^{3} y^{i(r)} \quad \text{wherein} \quad y^{i(r)} = \sum_{n=1}^{N_i} \sum_{b=1}^{4} x_{n(b)}^{i(r)} \qquad (5)$$

[herein, when the r-th base (r is 1, 2, or 3) of the n-th codon of the i-th coding region is b (b is 1, 2, 3, or 4), then

$$\chi_{n|b|}^{i|r|} \text{ is } \chi_{n|b|}^{i|r|} = 1 \text{ (b= 1 or 2)}$$

$$\chi_{n|b|}^{i|r|} = 0 \text{ (b= 3 or 4)}$$

and, as for b, when the r-th base of the n-th codon of the i-th coding region is G, C, A, or T, b is 1, 2, 3, or 4, respectively, i and n are positive integers, and $N_i$ denotes the total number of the codons (excluding the translation stop codon) of the i-th coding region].

(18) A method of determining a genetic structure of a prokaryote whose GC content in the nucleotide sequence exceeds 50%, wherein the method of determining a genetic structure according to any one of (1) to (13) and the method of determining a genetic structure according to any one of (14) to (17) are utilized.

Furthermore, the present invention is specified by (19) to (24) below.

(19) A method of determining a genetic structure of a prokaryote, wherein the method of determining a genetic structure according to any one of (1) to (18) and a "method of deciding on the truth or falsity of a coding region by utilizing a coding potential" are utilized.

(20) The method of determining a genetic structure according to (19), wherein said "method of deciding on the truth or falsity of a coding region by utilizing a coding potential" is a method of deciding on the truth or falsity of the coding region A described below by, based upon the nucleotide sequences of the number T of the determined coding regions of the prokaryote, comparing the "number of times of m types of codons whose frequency of appearance is high appearing in the coding region A which is assumed to be the coding region" with the "number of times of m types of codons whose frequency of appearance is low appearing in the coding region A" for the number T of coding regions [herein, T is an integer greater than or equal to 2, and m is an integer greater than or equal to 5 and less than or equal to 20].

(21) The method of determining a genetic structure according to (20), wherein the method of comparing the "number of times of m types of codons whose frequency of appearance is high appearing in the coding region A which is assumed to be the coding region" and the "number of times of m types of codons whose frequency of appearance is low appearing in the coding region A" is a method which involves utilizing the "reciprocal of the sum of 1 and the ratio of the number of the latter to the number of the former" as a calculation formula, and which decides that said coding region A is a "false coding region" if the value of said reciprocal is less than a fixed value [herein m is an integer greater than or equal to 5 and less than or equal to 20].

(22) The method of determining a genetic structure according to (20), which comprises the steps (s) to (u) described below:

(s) obtaining $y_i$ from the following formula (6):

$$y_i = \sum_{t=1}^{T} C_i^t \Big/ \sum_{t=1}^{T} \sum_{j=1}^{64} C_j^t \qquad (6)$$

wherein the number of times of the i-th codon appearing in the t-th coding region is expressed as $C_j^t$

(t) rearranging the 64 types of codon in descending order of $y_i$, selecting "top m codons for which the value of $y_i$ is large" and "bottom m codons for which the value of $y_i$ is large, excluding the translation stop codon", and obtaining the value of $Cd_A$ for the coding region A which is assumed to be the coding region from the following formula (7):

$$Cd_A = 2\times\sum_{i=1}^{m}C_i^A \Big/ \left(\sum_{i=1}^{m}C_i^A + \sum_{i=62-m}^{61}C_i^A\right) \quad (7)$$

[herein the value of $Cd_A$ is defined as 1 if

$$\left(\sum_{i=1}^{m}C_i^A + \sum_{i=62-m}^{61}C_i^A\right)$$

is zero];

(u) deciding that said coding region A is a true coding region if the value of $Cd_A$ for said coding region A which is calculated in the step (t) is greater than or equal to a threshold value CV, and deciding that it is a false coding region if said value of $Cd_A$ is less than the threshold value CV [herein T is an integer greater than or equal to 2; i is a positive integer less than or equal to 64; j is a positive integer less than or equal to 64; t is a positive integer less than or equal to T, m is an integer from 5 to 20; and CV is a value from 0.8 to 1.8].

(23) A method of determining a genetic structure, which comprises the steps (v) and (w) described below if a coding region of the prokaryote or a coding region A which is assumed to be a coding region overlaps with a coding region B which is assumed to be a coding region and present upon the complementary strand, and if said coding region B is included in said coding region A:

(v) comparing the length $L_B$ (in base pairs) of said coding region B with the length $L_A$ (in base pairs) of said coding region A, and deciding that said coding region B is a "false coding region" if $L_B$ is less than or equal to $T_P$% of $L_A$;

(w) deciding on the truth or falsity of said coding region A and of said coding region B by the method of determining a genetic structure according to any one of (18) to (22) if $L_B$ exceeds $T_P$% of $L_A$ [herein TP is a positive integer from 30 to 95].

(24) A method of determining a genetic structure, characterized by removing the translation stop codons from the coding regions which form a transcription unit, and linking up the resulting coding regions into a single coding region, before utilizing the method of determining a genetic structure according to any one of (18) to (23).

Furthermore, the present invention is specified by (25) to (45) below.

(25) A program for executing the following steps on a computer:

(a) finding a translation stop codon in the nucleotide sequence of a prokaryote from the information of said nucleotide sequence inputted via an input device, searching for a provisional translation start codon which yields the longest open reading frame (ORF) for all the obtained translation stop codons to make a candidate for ORF which is the combination of the said translation stop codon and provisional translation start codon, and storing the position of these codons in said nucleotide sequence in a memory;

(b) calling up from the memory two adjacent candidates for ORF which are present upon the same strand, investigating the positions of the provisional translation start codon of the downstream side ORF (termed ORF-A) and of the translation stop codon of the upstream side ORF (termed ORF-B) and the distance between the ORF-A and the ORF-B; and

deciding that the two adjacent ORFs have a possibility to form a single transcription unit if the provisional translation start codon of the ORF-A is upstream of the translation stop codon of the ORF-B, or is within $D_S$ bases downstream of said translation stop codon [herein $D_S$ is an integer from 20 to 100], and proceeding to the step (c); or

deciding that the two adjacent ORFs do not form a single transcription unit if the distance between the positions of the provisional translation start codon of the ORF-A and of the translation stop codon of the ORF-B does not satisfy the above described condition, and proceeding to the step (f);

(c) calling up the above described nucleotide sequence data for the two ORFs which are decided to have a possibility to form a single transcription unit in the step (b), and searching for a candidate for the translation start codon of the ORF-A from a region (hereinafter termed the "vicinity of the translation stop codon") between $D_B$ bases downstream from the first T (thymidine) residue of the translation stop codon of the ORF-B and $U_B$ bases upstream from said T residue [here $D_B$ is an integer between 10 and 20, and $U_B$ is an integer between 3 and 15]; and

determining that the ORF-A whose translation start codon is said candidate is a true coding region if there is a single candidate for the translation start codon,

determining that said ORF-A and ORF-B form a single transcription unit, and writing the results of this determination into the memory; or

selecting the candidate whose priority is the highest if there is a plurality of candidates for the translation start codon, wherein the distance between each candidate and the translation stop codon of the ORF-B is used as an indicator of priority, determining that the ORF-A whose translation start codon is said candidate is a true coding region, and

determining that said ORF-A and ORF-B constitute a single transcription unit, and writing the results of the determination into the memory;

(d) calling up the above described nucleotide sequence data if the translation start codon of the ORF-A can not be determined in the step (c), examining whether a candidate for the translation start codon of the ORF-A is present within a region (hereinafter termed the "coding region around the vicinity of the translation stop codon") between $R_D$ bases downstream from the first T residue of the translation stop codon of the ORF-B and $R_U$ bases upstream from said T residue [here $R_D$ is an integer from 30 to 120, and $R_U$ is an integer from 20 to 120] and excluding the "vicinity of the translation stop codon"; and

proceeding to the step (e) if a candidate for the translation start codon of the ORF-A is present in said region, or proceeding to the step (f) if no such candidate is present;

(e) calling up the above described nucleotide sequence data for a candidate for the translation start codon of the ORF-A found in the step (d), examining whether a ribosome binding site is present from 1 to 30 bases upstream of each candidate, and determining its ribosome binding sequence if such a ribosome binding site is present, or determining that the ORF-A, whose translation start codon is the candidate which corresponds to said ribosome binding sequence, is a true coding region, determining that said ORF-A and ORF-B form a single transcription unit, and writing the results of the determination into the memory;

(f) calling up the above described nucleotide sequence data for an ORF-A which is not decided to form a single transcription unit in the step (b) or for an ORF-A whose translation start codon can not be determined in the step (e), searching for up to the number N of candidates [here N is an integer from 5 to 20] for the translation start codon, including the provisional start codon which yields the longest ORF, from the 5' terminal, examining whether a ribosome binding site is present from 1 to 30 bases upstream of each candidate, determining its ribosome binding sequence if such a ribosome binding site is present, determining that the ORF-A whose translation start codon is the candidate corresponding to said ribosome binding sequence is a true coding region, and writing the results of the determination into the memory;

(g) repeating the above steps until all of the ORFs stored in the memory are processed;

outputting, via an output device, the results of determination of transcription units and coding regions in step (c), (e) or (f), which have been stored in the memory.

(26) The program according to (25), wherein the above described step (e) is:

calling up the above described nucleotide sequence data;

calculating a "ribosome binding score" which express the paired state between a mRNA sequence of 4 to 17 bases upstream of a candidate for the translation start codon of the ORF-A and a sequence (3'-UUCCUCC-5') involved in the binding to mRNA in a 16S rRNA 3' terminal sequence, or between a mRNA sequence of 4 to 16 bases upstream of said candidate and a sequence (3'-UCCUCC-5') involved in the binding to mRNA within a 16S rRNA 3' terminal sequence as a numerical value according to the four rules described below:

(1) A pairing of G and C yields +4;
(2) A pairing of A and U yields +2;
(3) A pairing of G and U yields +1;
(4) When no pairing is present at a base pair which is adjacent to a base pair where a pairing is present, then this yields -1;

maintaining a threshold value $V_3$ [herein $V_3$ is an integer from 4 to 12] for said ribosome binding score, determining that the above described mRNA sequence whose ribosome binding score exceeds a threshold value V3 is a ribosome binding sequence, and selecting the translation start codon which corresponds to said ribosome binding sequence as the translation start codon of the ORF-A;
dividing the "region around the vicinity of the translation stop codon of the ORF-B" into the two "the region downstream of said vicinity" and "the region upstream of said vicinity" if there is a plurality of said translation start codons for the ORF-A, and selecting the candidate whose priority is highest, wherein the order of priority is the first "the region downstream of said vicinity" and the second "the region upstream of said vicinity";
selecting the candidate whose priority is highest if a plurality of translation start codons is present within the respective regions, wherein the distance from the translation stop codon of the ORF-B is used as an indicator of priority; and
determining that the ORF-A whose translation start codon is the selected candidate is a true coding region, determining that said ORF-A and ORF-B form a single transcription unit, and writing the results of the determination into the memory.

(27) The program according to (25) or (26), wherein the above described step (f) is:

calling up the above described nucleotide sequence data;
calculating a "ribosome binding score" which express the paired state between a mRNA sequence of 4 to 17 bases upstream of a candidate for the translation start codon of the ORF-A and a sequence (3'-UUCCUCC-5') involved in the binding to mRNA in a 16S rRNA 3' terminal sequence, or between a mRNA sequence of 4 to 16 bases upstream of said candidate and a sequence (3'-UCCUCC-5') involved in the binding to mRNA in a 16S rRNA 3' terminal sequence as a numerical value, according to the four rules described below:

(1) A pairing of G and C yields +4;
(2) A pairing of A and U yields +2;
(3) A pairing of G and U yields +1;
(4) When no pairing is present at a base pair which is adjacent to a base pair where a pairing is present, then this yields -1;

maintaining a threshold value $V_1$ for said ribosome binding score, determining that the above described mRNA sequence which exceeds the threshold value $V_1$ is the ribosome binding sequence, and selecting a candidate for the translation start codon which corresponds to said ribosome binding sequence as the translation start codon of the ORF-A;
selecting the translation start codon corresponding to the ribosome binding sequence which yields the highest score as the translation start codon of ORF-A if there is a plurality of said translation start codons;
setting one or more threshold value(s) which is smaller than $V_1$ and include the threshold value $V_3$ in a stepwise manner if there is no candidate which exceeds the threshold value $V_1$, searching for the above described mRNA sequence whose score exceeds said threshold value in a stepwise manner, determining the ribosome binding sequence, and selecting the translation start codon which corresponds to said ribosome binding sequence as the translation start codon of the ORF-A; and
determining that the ORF-A whose translation start codon is the selected candidate is a true coding region, and

writing the results of the determination into the memory [herein $V_1$ is an integer which is greater than the $V_3$ of (2), and which is between 7 and 14].
(28) The program according to (26) or (27), characterized in that the above described "ribosome binding score" is calculated by deducting a numerical value $P_G$ if the translation start codon is GTG, and by deducting a numerical value $P_T$ if the translation start codon is TTG [herein, $P_G$ is an integer from 1 to 4, and $P_T$ is an integer from 2 to 6].
(29) A program for executing the following steps on a computer: calling up the data for transcription units and coding regions stored in the memory after the above described step (g) in the program according to (25) to (28);

(h) deciding that the transcription unit Q or the coding region B is a "false transcription unit" or a "false coding region" if a transcription unit Q or a coding region B which is present upon the same strand as a transcription unit P or a coding region A is included in the transcription unit P or the coding region A;
(i) deciding that the transcription unit Q or the coding region B is a "false transcription unit" or a "false coding region" if a transcription unit Q or a coding region B which is present upon the complementary strand to a transcription unit P or a coding region A is included in the transcription unit P or the coding region A;
(j) deciding that the transcription unit or coding region whose length is shorter is a "false transcription unit" or a "false coding region" if a transcription unit P or a coding region A overlaps with a transcription unit Q or a coding region B which is present upon the complementary strand; and

outputting the results of the above described decision via an output device.

(30) A program for executing the following steps on a computer:

(k) investigating the type of the codons and the number thereof, which are utilized in a plurality (T) of the coding regions of the prokaryote which regions are determined and inputted via an input means, selecting k types of combination of codons among them wherein "the frequency of appearance of one codon is high, and the frequency of appearance of a codon which has the complementary sequence of the 3-base sequence of said codon is low", and storing the codons in the memory;

(l) measuring the frequency of appearance of the selected codons in a coding region A which is assumed to be the coding region from the data of said coding region A inputted via an input means, comparing the "number of times of the k types of codons whose frequency of appearance is high appearing in a coding region A which is assumed to be a coding region" with the "number of times of the k types of codons whose frequency of appearance is low appearing in said coding region A", and deciding on the truth or falsity of said coding region A [herein k is an integer greater than or equal to 5 and less than or equal to 20]; and

displaying the results of the above described decision via an output device.

(31) The program according to (30), wherein the step (1) is comparing the "number of times of the k types of codons whose frequency of appearance is high appearing in a coding region A which is assumed to be the coding region" and the "number of times of the k types of codons whose frequency of appearance is low appearing in said coding region A" by using "the reciprocal of the sum of 1 and the ratio of the number of the latter to the number of the former" as a calculation formula, and deciding that said coding region A is a "false coding region" if the value of said reciprocal is less than a fixed value.

(32) The program for executing the following steps on a computer according to (30):

(m) constructing a codon table by arranging the 64 types of codons so that the 3-base sequence of the i-th codon has the complementary sequence to the nucleotide sequence of the (i+32)-th codon, and storing the codon table in the memory;

(n) inputting the nucleotide sequence of the number T of determined coding regions of a prokaryote, and obtaining yi from the formula (2) below and yi+32 from the formula (3) below:

$$y_i = \left(\sum_{t=1}^{T} C_i^t - \sum_{t=1}^{T} C_{i+32}^t\right) \Big/ \sum_{t=1}^{T}\sum_{j=1}^{64} C_j^t \qquad (2)$$

$$y_{i+32} = \left(\sum_{t=1}^{T} C_{i+32}^t - \sum_{t=1}^{T} C_i^t\right) \Big/ \sum_{t=1}^{T}\sum_{j=1}^{64} C_j^t \qquad (3)$$

wherein the number of times the i-th codon appear in the t-th coding region is expressed as $C_i^t$

(o) calling up the codon table which was obtained in the step (m) from the memory, setting up a correspondence between the $y_i$ and $y_{i+32}$ for the codons in the table, rearranging the sequence of the codons in the table in descending order of the $y_i$ and the $y_{i+32}$, selecting top k codons for which the value of $y_i$ or of $y_{i+32}$ is large, and obtaining the value of $Sd_A$ for a coding region A by the following formula (4):

$$Sd_A = 2 \times \sum_{i=1}^{k} C_i^A \Big/ \left(\sum_{i=1}^{k} C_i^A + \sum_{i=65-k}^{64} C_i^A\right) \qquad (4)$$

[herein the value of $Sd_A$ is defined as 1 if

$$\left(\sum_{i=1}^{k} C_i^A + \sum_{i=65-k}^{64} C_i^A\right)$$

is zero];

(p) deciding that said coding region is a true coding region if the value of $Sd_A$ of a coding region A obtained in the above described step is greater than or equal to a threshold value $S_1$, and deciding that it is a false coding region if said value of $Sd_A$ is less than the threshold value $S_1$ [herein T is an integer greater than or equal to 2, i is a positive integer less than or equal to 32, j is a positive integer less than or equal to 64, t is a positive integer less than or equal to T, k is an integer from 5 to 20, and $S_1$ is a value from 0.8 to 1.8].

(33) A program for executing the following steps on a computer:

examining whether there is mutual overlapping and inclusion between coding regions of a prokaryote which are inputted via an input device:

(q) calling up the above described nucleotide sequence data if a coding region or a coding region A which is assumed to be a coding region overlaps with a coding region B which is assumed to be a coding region and present upon the complementary strand, and if said coding region B is included in said coding region A, comparing the length $L_B$ (in base pairs) of said coding region B with the length $L_A$ (in base pairs) of said coding region A, and deciding that said coding region B is a "false coding region" if $L_B$ is less than or equal to $T_P$% of $L_A$;

(r) deciding on the truth or falsity of said coding region A and of said coding region B by the steps of the program according to any one of (30) to (32) if $L_B$ exceeds $T_P$% of $L_A$ [herein $T_P$ is a positive integer from 30 to 95].

(34) The program according to (33), characterized by rewriting the data for the determined coding regions to a single coding region constructed by removing the translation stop codons from the coding regions which form a transcription unit and by linking up the resulting coding regions from said data, before executing the steps (k) and (l) described above.

(35) A program for deciding on the truth or falsity of a coding region or of a transcription unit which is determined and stored in the memory in any one of (25) to (35), by the steps of the program according to any one of (30) to (34).

(36) A program for executing the steps:

calling up the data for coding regions which is determined as true coding regions by the steps of the program according to any one of (25) to (35) from the memory, calculating the length of the polypeptide encoded by each coding region, and deciding on the truth or falsity of the coding regions which encode the polypeptides of $L_M$ amino acids or more in length, by using the program according to any one of (7) to (12), based upon the nucleotide sequences of the coding regions encoding the polypeptide of $L_F$ amino acids or more in length [herein $L_F$ is a positive integer greater than or equal to 100, and $L_M$ is a positive integer greater than or equal to 20].

(37) A program for executing the following steps on a computer:

calculating the content of the first and third G residues and C residues of the codons in a coding region of a prokaryote whose GC content exceeds 50% by using a predetermined calculation formula from the data for said coding region inputted via an input device; deciding that said coding region is a "false coding region" if the calculated content is less than a fixed value; and outputting the results of the decision via an output device.

(38) The program according to (37), wherein the following formula (5) is used as a calculation formula, the value of $GC_i$ described below is used as a calculated content, and one value which is selected from 0.6 to 0.75 is used as a fixed value:

$$GC_i = (y^{i(1)} + y^{i(3)}) \Big/ \sum_{r=1}^{3} y^{i(r)} \quad \text{wherein} \quad y^{i(r)} = \sum_{n=1}^{N_i} \sum_{b=1}^{4} x_{n(b)}^{i(r)} \qquad (5)$$

[herein when the r-th base (r is 1, 2, or 3) of the n-th codon of the i-th coding region is b (b is 1, 2, 3, or 4), then

$\chi_{n|b|}^{i|r|}$ is $\chi_{n|b|}^{i|r|} = 1$ (b= 1 or 2)

$\chi_{n|b|}^{i|r|} = 0$ (b= 3 or 4)

and, as for b, when the r-th base of the n-th codon of the i-th coding region is G, C, A, or T, then b is 1, 2, 3, or 4, respectively, i and n are positive integers, and $N_i$ denotes the total number of the codons (excluding the translation stop codon) of the i-th coding region].

(39) A program for executing the following steps on a computer:

calculating the content of the first and third G residues and C residues of the codons of the 5' terminal region of a coding region of a prokaryote whose GC content exceeds 50% by using a predetermined calculation formula, from the data for said coding region inputted via an input device;
deciding that the translation start codon of said coding region is a "false translation start codon" if the calculated content is less than a fixed value, and
outputting the results of this decision via an output device;
calling up the nucleotide sequence data of the above described coding region which is inputted via an input device, and re-searching for a translation start codon which is present downstream of said translation start codon decided to be false.

(40) The program according to (39), wherein the following formula (5) is used as an calculation formula, the value of $GC_i$ described below is used as a calculated content and one value selected from 0.6 to 0.75 is used as a fixed value:

$$GC_i = (y^{i(1)} + y^{i(3)}) \Big/ \sum_{r=1}^{3} y^{i(r)} \quad \text{wherein} \quad y^{i(r)} = \sum_{n=1}^{N_i} \sum_{b=1}^{4} x_{n(b)}^{i(r)} \qquad (5)$$

[herein, when the r-th base (r is 1, 2, or 3) of the n-th codon of the i-th coding region is b (b is 1, 2, 3, or 4), then

$\chi_{n|b|}^{i|r|}$ is $\chi_{n|b|}^{i|r|} = 1$ (b= 1 or 2)

$\chi_{n|b|}^{i|r|} = 0$ (b= 3 or 4)

and, as for b, when the r-th base of the n-th codon of the i-th coding region is G, C, A, or T, b is 1, 2, 3, or 4, respectively, i and n are positive integers, and $N_i$ denotes the total number of the codons (excluding the translation stop codon) of the i-th coding region].

(41) A program for executing the following steps on a computer:

selecting m types of codons whose frequency of appearance is high and m types of codons whose frequency of appearance is low in the number T of the coding regions of a prokaryote which are determined by the steps of the program according to any one of (25) to (40), and storing the codons in the memory;
measuring the "number of times of the m types of codons whose frequency of appearance in the number T of the coding regions is high appearing in the coding region A which is assumed to be the coding region" and the "number of times of the m types of codons whose frequency of appearance in the T coding regions is low appearing in said coding region A" from the data for coding regions which are not determined, different from the number T of the coding regions and inputted; deciding on the truth or the falsity of said coding region A by comparing both numbers; and outputting the results of the decision via an output device. [herein T is an integer greater than or equal to 2, and m is an integer greater than or equal to 5 and less than or equal to 20].

(42) The program according to (41), wherein the method of comparing the "number of times of the m types of codons whose frequency of appearance is high appearing in the coding region A which is assumed to be the coding region" with the "number of times of the m types of codons whose frequency of appearance is low appearing in said coding region A" is the method which utilizes the "reciprocal of the sum of 1 and the ratio of the number of the latter to the number of the former" as a calculation formula, and which decides that said coding region A is a "false coding region" if the value of said reciprocal is less than a fixed value [herein m is an integer greater than or equal to 5 and less than or equal to 20].

(43) The program for executing the following steps on a computer according to (41):

(m) constructing a codon table in which the 64 types of codons are arranged so that the 3-base sequence of the i-th codon has a complementary sequence to the nucleotide sequence of the (i+32)-th codon, and storing the codon table in the memory;

(s) obtaining $y_i$ by the following formula (6):

$$y_i = \sum_{t=1}^{T} C_i^t \Big/ \sum_{t=1}^{T} \sum_{j=1}^{64} C_j^t \qquad (6)$$

wherein the number of times of the i-th codon appearing in the t-th coding region is expressed as $C_j^t$

(t) calling up the codon table from the memory, rearranging the 64 types of codon in descending order of $y_i$, selecting "top m codons for which the value of $y_i$ is large" and "bottom m codons for which the value of $y_i$ is large, excluding the translation stop codon", and obtaining the value of $Cd_A$ for the coding region A which is assumed to be the coding region from the following formula (7):

$$Cd_A = 2 \times \sum_{i=1}^{m} C_i^A \Big/ \left(\sum_{i=1}^{m} C_i^A + \sum_{i=62-m}^{61} C_i^A\right) \qquad (7)$$

[herein the value of $Cd_A$ is defined as 1 if

$$\left(\sum_{i=1}^{m} C_i^A + \sum_{i=62-m}^{61} C_i^A\right)$$

is zero, ] and

(u) deciding said coding region A to be a true coding region if the value of $Cd_A$ for said coding region A which is calculated by the step (t) is greater than or equal to a threshold value CV, deciding said coding region A to be a false coding region if said value of $Cd_A$ is less than the threshold value CV, and outputting the decision results via an output device [herein T is an integer greater than or equal to 2; i is a positive integer less than or equal to 64; j is a positive integer less than or equal to 64; t is a positive integer less than or equal to T, m is an integer from 5 to 20; and CV is a value from 0.8 to 1.8].

44. A program for executing the following steps on a computer, wherein a coding region of the prokaryote or a coding region A which is assumed to be a coding region overlaps with a coding region B which is assumed to be a coding region and present upon the complementary strand, and said coding region B is included in said coding region A:

(v) comparing the length $L_B$ (in base pairs) of the coding region B with the length $L_A$ (in base pairs) of the coding region A, and deciding that the coding region B is a "false coding region" if $L_B$ is less than or equal to $T_P$% of $L_A$;
(w) deciding on the truth or falsity of said coding region A and of said coding region B by the method of determining a genetic structure according to any one of (41) to (43) if $L_B$ exceeds $T_P$% of $L_A$, [herein, $T_P$ is a positive integer from 30 to 95]; and

outputting the results of the decision via an output device.

(45) A program executing the following steps:

removing translation stop codons from the coding regions which form a transcription unit from the data for determined coding regions; linking up the resulting coding regions into a single coding region; and rewriting the data for determined coding regions to the resulting single coding region; and executing the steps of the program according to any one of (41) to (44).

46) A computer-readable recording medium on which the program according to any one of Claim 25 to Claim 45 is recorded.

(47) A system for determining a genetic structure which comprises:

(i) an input means for inputting nucleotide sequence data;
(ii) a means for executing the program according to any one of Claim 25 to Claim 45, using the inputted data; and
(iii) an output device for outputting the results which is obtained by (ii).

Brief Description of the Drawings

FIG. 1 is a figure showing the genetic structure of a prokaryote. In the present specification, the distance between the AGGA sequences which appear at high frequency in the ribosome binding sequences in the drawing and the start codon, is defined as the distance between the SD sequence and the start codon.

FIG. 2 is a figure showing the structure of the transcription units of a prokaryote. As shown in the figure, the fact that many false CDS, i.e. so called "gene shadows", appear in the opposite strand of a true CDS, is a cause of deterioration of the accuracy of CDS determination. The ORFs are shown by "white boxes" or by "white and gray colored" boxes, while the CDS are shown by gray colored boxes. Furthermore, the SD sequences are shown by small black boxes, while the candidates for start codons (normally ATG, GTG, and TTG) are shown by black triangles, and the stop codons are shown by black dots.

FIG. 3 is a figure showing the flow chart of an algorithm of a "method of determining a genetic structure from the viewpoint of transcription unit structure" according to the present invention.

FIG. 4 is a figure showing, in the "method of determining a genetic structure from the viewpoint of transcription unit structure" according to the present invention, the value of a rank function which determines a priority ranking of the position of a start codon, when two coding regions constitute an operon. The value $R_{UP}$ in the figure is an integer which is greater than the distance between the stop codon for creating a polycistron for which a value of three times ($R_{UP}$-2) has been set in advance and the start codon, and moreover is an integer greater than 10. $R_{DN}$ is an integer which is not greater than a value (for example 30) which has been set in advance, and which is greater than ($R_{UP}$+2).

FIG. 5 is a figure showing, as a flow chart, the process of computation of the "ribosome binding score" of a coding region A (CDS-A).

FIG. 6 is a figure showing a paired state between a SD sequence of a Escherichia coli trpL gene and a 16S rRNA 3' terminal sequence.

FIG. 7 is a figure showing a flow chart of an algorithm of a variant of a "method of CDS determination aimed at transcription unit structure" of the present invention, and is continued in FIG. 8.

FIG. 8 continues from FIG. 7, and is a figure showing a flow chart of the algorithm of the variant of the "method of CDS determination aimed at transcription unit structure" of the present invention.

FIG. 9 is a figure showing as a flow chart a process for, after having determined a plurality of CDSs and transcription units from the nucleotide sequences of the plus strand and of the minus strand using the "method of CDS determination aimed at transcription unit structure" of the present invention, deciding upon the truth or the falsity of transcription units which overlap.

FIG. 10 is a figure showing as a flow chart a example of a method for determining a plurality of CDSs by repeatedly using the "method of CDS determination aimed at transcription unit structure". In the figure, $X_1$ (%) normally takes a value from 5 to 20.

FIG. 11 is a figure showing as a flow cart a process for enhancing the accuracy of CDS determination by deciding upon the truth or the falsity of a coding region A (CDS-A) using a shadow discrimination function. In the figure, $P_1$ (%) normally takes a value from 5 to 20.

FIG. 12 is a structural figure of hardware for desirably implementing the method of the present invention.

FIG. 13 is an overall processing flow diagram of one embodiment of the method of determining a genetic structure of the present invention.

FIG. 14 is an overall processing flow diagram of one embodiment of the method of determining a genetic structure of the present invention.

FIG. 15 is an overall processing flow diagram of one embodiment of the method of determining a genetic structure of the present invention.

ILLUSTRATIVE Mode for Carrying Out the Invention

Although the method of determining a genetic structure of the present invention is not limited to a method which involves utilizing a computer, it is desirable to carry out the method using a computer from the viewpoint that the computer can process a large amount of nucleotide sequence data at high speed.

In FIG. 12 shows a hardware structure for operating the method of determining a genetic structure of the present invention in a desirable manner, and, basically, it consists of an input device 1, a storage device 2, a central processing unit (CPU) 3, and an output device 4, and these are connected together by bus lines 5.

The CPU 3 is a device which executes a program to search for sequences, to calculate, compare, and store various indicators, and to designate data to be outputted, and the like.

The input device 1 is a device for inputting the nucleotide sequence data which must be analyzed, and for inputting designation commands for executing various types of processing, and the like.

The storage device 2 is a memory for storing inputted information and the results of calculation, and memory access means for accessing the memory; and is mainly consists of an external storage means 6, a data storage means 7, a program file 8, and the like.

The external storage means 6 is a recording medium such as a floppy disk, an magneto-optical disk, a hard disk, and a memory or the like, in which the data of the nucleotide sequence to be analyzed is stored.

The data storage means 7 (hereinafter also termed the memory) is a device for storing data which is obtained by various types of processing for operating the method of determining a genetic structure of the present invention. Data of ORFs, CDSs, and transcription units, which are determined or whose truth or falsity is decided by the method of determining a genetic structure of the present invention, is also stored therein.

In the program file 8, a program for executing the method of the present invention, a table for the rank function described hereinafter and the like are stored.

The output device 4 is a device for outputting the results of determination or decision of ORFs, CDSs, and transcription units by the method of determining a genetic structure of the present invention and utilizes a display, a printer, a recording medium or the like. In other words, the information about the positions of the translation start codon and the stop codon and about the CDSs which are included in each of the transcription units can be outputted by displaying it upon the display, by printing it by outputting it to the printer, or by recording it upon the recording medium,

Furthermore, it is desirable that the hardware for operating the present invention is connected to a transmission network 9 to transmit an external nucleotide sequence database via said network and to store it in an external storage means.

1. A method to determining a genetic structure from the viewpoint of a transcription unit structure

The "method of determining a genetic structure from the viewpoint of a transcription unit structure" of the present invention is method which employs a method for predicting the positions of start codons and the structure of polycistronic transcription units at high accuracy, and a method of determining a genetic structure of a prokaryote at a high accuracy. The structure of the polycistronic transcription units can be determined by the method. In the method of determining a genetic structure, the determination of the position of a start codon, the determination of CDS, and the determination of the structure of a polycistronic transcription unit are intimately mutually related. In other words, in case of the determination of the position of the start codon of a coding region CDS-A, it is examined whether CDS-A and other coding region CDS-B form a polycistronic transcription unit, and if the CDS-A and the CDS-B have a possibility to form a polycistronic transcription unit, then the positional relationship between the stop codon of CDS-B and the candidates for a start codon of CDS-A is examined to decide the truth or falsity of the start codon. Furthermore, by expressing the paired state of the mRNA sequence and the ribosome binding sequence within the 3' terminal sequence of the 16S rRNA as a numerical value, the position of the start codon is determined and the truth or falsity of the CDS is decided.

FIG. 13 gives an overall processing flow diagram when carrying out the method of determining a genetic structure according to an embodiment of the present invention using the device structured as shown in FIG. 12.

In FIG. 13, the nucleotide sequence information of a prokaryote which is to be analyzed is inputted via the input device at the step S11.

In the method of the present invention, the nucleotide sequence data can be inputted to the computer by inputting the data directly from a keyboard, by reading the data which is transmitted via the internet or the like, or by reading the data recorded on a recording medium (an externally stored file) such as a floppy disk, an magneto-optical disk, a hard disk, a memory and the like.

In the step S12, the CPU searches for a stop codon from the nucleotide sequence data which is inputted in the step S11, searches for a provisional start codon which yields the longest ORF in length based upon said stop codon, and stores a set of the stop codon and the provisional start codon as an candidate for ORF in the memory.

In the step S13, the CPU calls up from the memory two adjacent ORFs which are present upon the same strand, and decides on the possibility to form a single transcription unit by investigating the positional relationship between the translation start codon on the downstream side and the translation stop codon on the upstream side.

In the step S14, for ORFs which are decided to have possibility to form a single transcription unit in the step S13, candidates for a translation start codon are searched for from the region around the translation start codon on the downstream side, and the translation start codon on the downstream side is determined based upon the positional relationship with the translation stop codon on the upstream side and a priority order which has been determined in advance, and the determined transcription unit and CDS are stored in the memory. For other ORFs, candidates for translation start codon are searched for from the region around the above described provisional translation start codon, from the found candidates, a candidate which has a ribosome binding region in an appropriate position upstream thereof is selected to determine a translation start codon, and the determined CDSs are stored in the memory.

In the step S17, the data for determined transcription units and CDSs, which is stored in the memory, is outputted via the output device.

the position of a start codon is expressed as the position of the first base of 3 bases of the codon in the case of the start codon upon a plus strand, expressed as the position of the first base or the last base of 3 bases of the codon in the case of the start codon upon a minus strand during the output. The position of a stop codon is expressed as the position of the first base or the last base of 3 bases of the codon in the case of the stop codon upon a plus strand, and expressed as the position of the first base of 3 bases of the codon in the case of the stop codon upon a minus strand during the output.

The method of determining a genetic structure shown in FIG. 13 is described in further detail using the flow chart of FIG. 3.

The process (a) of FIG. 3 corresponds to the step S12 of FIG. 13.

The information about a nucleotide sequence which can be utilized in the present invention is information about the nucleotide sequence of the DNA or the RNA of a prokaryote. It is possible to utilize a nucleotide sequence of either a single strand or double strands. In the case of double strands, information about the nucleotide sequence of either of the strands is utilized. It is possible that the nucleotide sequence information of the plus strand of the double strands is utilized, and then the nucleotide sequence information of the minus strand is utilized. When a nucleotide sequence of RNA is utilized, it is desirable to utilize it by replacing the U residues by T residues. It is possible to predict the CDSs, not only for a nucleotide sequence of about 100 bases, but for a nucleotide sequence of genome DNA of more than a million bases.

A nucleotide sequence of a prokaryote includes the genome sequence of Escherichia coli K-12 strain 4639221bp (GenBank accession number: U00096), the genome sequence of Bacillus subtilis strain 168 of 4214814bp (GenBank accession number: AL009126), the nucleotide sequences of DNA fragments including ribosomal operons of Escherichia coli K-12 strain (GenBank accession numbers: AE000408 and AE000472, DNA fragments of 10944bp and 14659bp respectively in length), and the like. It is possible to predict the regions for RNA genes in a nucleotide sequence in advance using a homology search program or a tRNA prediction program such as tRNA-Scan or the like, and to eliminate said regions in the determination of CDS by replacing the bases of said regions with x and the like.

In the process (a), translation stop codons are searched for from the nucleotide sequence information of the prokaryote which was inputted via the input device, and the provisional translation start codon which yields the longest ORF is set based upon said stop codon.

The longest ORF means, for the i-th stop codon and the (i+1)th stop codon from the 5' terminal, the region from the ATG codon, the GTG codon, or the TTG codon (termed the provisional start codon) which appears first in the 3' direction from the i-th stop codon to the (i+1)th stop codon (herein i is an integer greater than or equal to 1). A TAA codon, a TAG codon, or a TGA codon is utilized as the stop codon.

The sets of the stop codon and the provisional translation start codon (the provisional ORFs) are arranged in the order of the position of the stop codon from the 5' terminal towards the 3' terminal, and stored in the memory with the positions of the stop codons and the provisional start codons in the above described nucleotide sequence.

ATG, GTG, and TTG are used as the start codon in the present invention unless mentioned specifically.

The process (b) is a process which corresponds to the step S13 of FIG. 13, wherein the CPU predicts the polycistronic transcription units. When a plurality of CDSs, in other words, cistrons, forms polycistron, the translation of these cistrons (CDSs) is often performed by utilizing the same ribosome binding site. In other words, the translational coupling between adjacent CDSs may occur. As a condition of occur the translational coupling between a CDS-A and a CDS-B which is present upstream of the CDS-A, it is necessary that the provisional start codon of the longest ORF which includes the CDS-A is upstream of the stop codon of the CDS-B, or that said provisional start codon is present in a close position downstream of said stop codon at least. More desirably, when the start codon of the CDS-A is present upstream of the stop codon of the CDS-B, or is present within $D_S$ bases downstream of said stop codon (herein $D_S$ is an integer from 20 to 100), it is decided that the translational coupling between the CDS-A and the CDS-B can occur, in other words, that CDS-A and the CDS-B have a possibility to form a polycistronic transcription unit. Accordingly, in this process, it is decided that the ORF-A and the ORF-B have a possibility to form a polycistronic transcription unit if the provisional start codon of the ORF-A is present upstream of the stop codon of the ORF-B (S302), or if it is present within $D_S$ bases downstream of said stop codon (S303), and the decision is stored in the memory; otherwise, it is decided that the set of the two adjacent ORFs (ORF-A and ORF-B) does not form a single transcription unit.

Next, the steps of determination of the start codon of the ORF-A [the step S14 of FIG. 13; the processes (c) to (f)] is explained.

Generally, in the expression of genes of a prokaryote, when a start codon of ORF-A is present in the vicinity of the stop codon of the ORF-B and a ribosome finished the translation of an ORF-B, the translation of ORF-A is started even if there is no ribosome binding sequence at the upstream of said start codon. Accordingly, when a start codon is present in the vicinity of the stop codon of ORF-B, it is determined that said start codon is the start codon of CDS-A. In the process (c) of the present invention, two adjacent ORF candidates which was stored in the memory are called up, and it is desirable to search for the start codon in the region within $D_B$ bases downstream from the first T residue of the stop codon of ORF-B (herein $D_B$ is an integer from 10 to 20) and within $U_B$ bases upstream from said T residue (herein $U_B$ is an integer from 3 to 15) as the "vicinity of the stop codon" of ORF-B where the translation of ORF-A is started. More desirably the search is performed in the region wherein $D_B$ is 14 and $U_B$ is 11 (S304) as shown in FIG. 4.

It is desirable to limit the start codon, which is searched for in this "vicinity of the stop codon" of ORF-B, to ATG or GTG. This is due to the prediction that it is difficult to start the translation from a TTG codon, when a suitable ribosome binding sequence does not exist.

If a single candidate for the translation start codon is obtained, then it is determined that the ORF-A whose translation start codon is said candidate is a true coding region, and that said ORF-A and ORF-B form a single transcription unit, and the results of determination are written into the memory (S312).

On the other hand, if a plurality of coding start candidates is present in the search of S304, then, the start codon whose priority is the highest is selected as the distance between each candidate and the translation stop codon of ORF-B as an indicator of the priority.

If the start codon can not be determined in the process (c), it is examined whether a candidate for the start codon at which translation of ORF-A can start is present in "the region around the vicinity of the stop codon" of ORF-B (S305) as the process (d). In the present invention, "the region around the vicinity of the stop codon" of ORF-B is a region within $R_D$ bases downstream from the first T (thymidine) residue of said stop codon and within $R_U$ bases upstream from said T residue and desirably said "vicinity of the stop codon" is excluded from the region [herein $R_D$ is an integer from 30 to 120, and $R_U$ is an integer from 20 to 120].

If a single translation start codon is determined, then it is determined that the ORF-A whose translation start codon is said candidate is a true coding region and that the ORF-A and the ORF-B form a single transcription unit, and the results of this determination are written into the memory.

In the above described processes (c) and (d), if a plurality of candidates for the start codon of the ORF-A is obtained, then, "region around the vicinity of the stop codon" of ORF-B is divided into the two of "the region downstream of said vicinity" and "the region upstream of said vicinity", it is determined that translation start codon which has the highest priority is the true translation start codon according to the priority of the three regions, "said vicinity", "the region downstream of said vicinity", and "the region upstream of said vicinity" in that order. Furthermore, when a plurality of translation start codons is present within each region, it is determined that the start codon which has the highest priority is the true start codon of ORF-A, according to a priority ranking determined in advance by using the distance from the translation stop codon of ORF-B as an indicator.

FIG. 4 shows an example in which the priority ranking of a translation stop codon which exists in the vicinity of the stop codon of the ORF-B is expressed as the "rank function". FIG. 4 shows an example in which the region within 14 bases downstream from the first T residue of the stop codon of the ORF-B and within 11 bases upstream from said T residue is set to the "vicinity of the stop codon of the ORF-B". When the priority ranking is determined, generally, priority ranking between the above described three regions is determined, and the rule that the closer to the stop codon of ORF-B, the higher is the priority ranking is applied.

In the present invention, "determining the priority ranking using the distance as an indicator" is applying this rule, desirably a priority ranking of the start codon is determined by utilizing, as shown in FIG. 4, a value expressing the positional relationship of the candidate for the start codon of the ORF-A with the stop codon of the ORF-B (in the present specification, termed "rank function"). The value of the "rank function" is shown in FIG. 4, and the lower is this value, the higher is the priority. In each of the regions "the vicinity of the stop codon of the ORF-B", "the region downstream of said vicinity", and "the region upstream of said vicinity", the closer is the start codon of the ORF-A to the stop codon of the ORF-B, the smaller is the value of the rank function.

Specifically, in FIG. 4, the "vicinity of the stop codon of the ORF-B" whose priority is the highest is the region within 14 bases in the downstream from the first T residue of the stop codon of the ORF-B and within 11 bases upstream from said T residue, and a value of the rank function of this region is a value from 1 to 8, as shown in FIG. 4.

As described above, the translation starts if the start codon of the ORF-A is present in the "region around the vicinity of the stop codon" of the ORF-B. Since the start codon of the ORF-A is far from the stop codon of the ORF-B, in order to start translating at high efficiency, it is desirable that a ribosome binding sequence is present upstream of the start codon of the ORF-A. In other words, in order to determine the candidate for the translation start codon of the ORF-A as the translation start codon, it is desirable that a ribosome binding sequence which enables a ribosome and mRNA to bind is present in the sequence upstream of said candidate.

A method of investigating a ribosome binding sequence includes a method which comprises obtaining a score which expresses the binding state between a ribosome and mRNA, by expressing the paired state between a mRNA sequence present in the region of 1 to 30 bases upstream of said candidate and a mRNA binding sequence within the 16S rRNA 3' terminal sequence as a numerical value (S306), and determining said candidate for the start codon as the start codon of ORF-A (S307) if the score exceed the threshold value, or the like.

As the mRNA binding sequence within the 16S rRNA 3' terminal sequence, many bacteria and Archaea have the sequence 3'-UUCCUCC-5' or 3'-UCCUCC-5'. Accordingly, when determining the start codon of a coding region of a prokaryotic cell whose 16S rRNA 3' terminal sequence is not known, it is also desirable to utilize 3'-UUCCUCC-5' or 3'-UCCUCC-5' as an mRNA binding sequence within the 16S rRNA 3' terminal sequence. It is also possible to utilize a sequence of 6 to 13 bases which includes a sequence (normally 1 to 3 bases) of a region around this sequence as an mRNA binding sequence. Furthermore, when determining the start codon of a coding region of a prokaryotic cell for which the 16S rRNA 3' terminal sequence is known, it is also possible to utilize said 16S rRNA 3' terminal sequence.

For starting translation at high efficiency, it is necessary for a sequence termed the SD sequence which participates in the binding with the mRNA binding sequence to be present in an appropriate position for the start of translation upstream of the start codon. So that, it is investigated whether a sequence which pairs with the 16S rRNA 3' terminal sequence is present in a region of 1 to 30 bases upstream of the start codon.

Specifically, this investigation is performed by a method of calculating a score which shows the binding state between the ribosome and the mRNA by expressing the paired state between a 3'-UUCCUCC-5' mRNA binding sequence within the 16S rRNA 3' terminal sequence and an mRNA sequence of 4 to 17 bases upstream of said start codon, or the paired state between a 3'-UCCUCC-5' mRNA binding sequence within the 16S rRNA 3' terminal sequence and an mRNA sequence of 4 to 16 bases upstream of said start codon, as a numerical value. As a means of calculating the score which shows the paired state between the ribosome and the mRNA, any method can be used, providing that it was a method of expressing as a numerical value a base pairing between an mRNA sequence which is in a region of 1 to 30 bases upstream of the start codon of the coding region, and an mRNA binding sequence within the 16S rRNA 3' terminal sequence. Said method includes, for example, a method using the rule of dissociation temperature of a nucleotide hybrid, or a method using the value of free energy which shows the binding state of a nucleotide hybrid [Schurr, T. et al.: Nucleic Acids Research Vol. 21, 4019-4023 (1993)], or a method of searching for a ribosome binding site utilizing a weight matrix [Frishman, D. et al.: Nucleic Acids Research Vol. 26, 2941-2947 (1998)], or the like.

When a score which shows the binding state between the ribosome and the mRNA is obtained by using the above described method, it is possible to determine that a sequence which has a score which exceeds a fixed threshold value which was set by various methods is the ribosome binding sequence.

In the following, a method of calculating the score which shows the binding state between the ribosome and the mRNA using the rule of the dissociation temperature of base-paired nucleotides will be explained.

The dissociation temperature of a hybrid of a DNA and an oligonucleotide often calculated by defining pairing of G and C as 4°C, pairing of A and T as 2°C, and pairing of G and T as 1°C, and it is possible to apply the same calculation method to a method using the rule of dissociation temperatures of a nucleotide hybrid. The score (hereinafter the score is referred to as the "ribosome binding score") is obtained based upon a pairing between the mRNA binding sequence within the 3' terminal sequence of the 16S rRNA and the nucleotide sequence upstream of the start codon, for example, by defining pairing of G and C as +4, pairing of A and U as +2, and pairing of G and U as +1. Furthermore, it is also desirable to allocate a penalty when no pairing occurs between the bases next to the bases between which the pairing was observed. As a penalty score, for example, -1 may be utilized. Accordingly, as a method for calculation of the score utilizing the rule of dissociation temperature of a hybrid substance of nucleic acids, the method is utilized of converting into a numerical value according to the four rules:

(1) A pairing of G and C yields +4;
(2) A pairing of A and U yields +2;
(3) A pairing of G and U yields +1;
(4) When no pairing is recognized at a base pair which is adjacent to a base pair for which a pairing has been recognized, then this yields -1.

Normally, the "ribosome binding score" is calculated for the 8 distances between the SD sequence and the start codon, each of which is 5 to 12 base pairs, and, after obtaining the maximum value, it is also desirable to utilize the value in the decision of the truth or falsity of the CDS.

Since it is known that, the better is the paired state between the mRNA sequence and the mRNA binding sequence within the 16S rRNA 3' terminal sequence, the higher is the starting efficiency of translation, accordingly it is possible to predict that, the greater is the value of the ribosome binding score, the higher is the starting efficiency of translation. Accordingly, it is possible to determine the position of the start codon from the magnitude of the value of the ribosome binding score. As a method of determining the start codon, it is possible to utilize a method which comprises obtaining the ribosome binding score for each start codon and selecting a start codon whose score exceeds some threshold value $V_1$ as the start codon. It is known that translation starting can be commenced at high efficiency when the SD sequence is AGGA or AGG, and the ribosome binding scores of these sequences are respectively 11 and 9. From this knowledge, when the rule of dissociation temperature of nucleotide hybrid is used in the calculation of the ribosome binding score, the threshold value $V_1$ is set to an integer from 7 to 14. It is known that many start codons of CDSs are present in the 5' terminal region of the longest ORF. Accordingly, the ribosome binding score is calculated for N candidates for the start codon (wherein N is an integer from 5 to 10) which are found from the 5' terminal of the longest ORF. Moreover, since the closer the candidate is to the 5' terminal, the higher is the possibility that it is the true start codon, therefore the ribosome binding score is compared with the threshold value $V_1$ in order from the candidate for the start codon which is closest to the 5' terminal, and it is possible to determined the candidate for start codon for which said score first exceeds the threshold value $V_1$ as the start codon. Since the translation starting is commenced even if the SD sequence is AAG or GG, therefore it is also desirable, if it is not possible to determine the start codon when the threshold value $V_1$ is used, to determine the start codon again by using a plurality of threshold values having values smaller than $V_1$, in a stepwise manner.

However, in the previously described process (d), if a candidate for the translation start codon is present in a region around the "vicinity of the translation stop codon" of ORF-B, then, since the ribosome which terminated the translation of ORF-B performs translation starting for ORF-A, the above described threshold value which is used for selecting the start codon may be a value $V_3$ which is smaller than $V_1$. As a value for $V_3$, an integer between 4 and 12 is desirable.

Generally, as the start codon, it is considered that the efficiency of translation starting is highest in the order ATG, GTG, and TTG. Accordingly, it is desirable to correct the ribosome binding score so as to reflect the differences between the start codon bases. As the means for this correction, for example, when calculating the ribosome binding score, it is possible to deduct a numerical value $P_G$ when the start codon is GTG, and to deduct a numerical value $P_T$ when the translation start codon is TTG. When using a method which use the rule of dissociation temperature of a nucleotide hybrid in a calculation for obtaining a ribosome binding sequence, it is possible to utilize an integer between 1 and 4 as $P_G$, and to utilize an integer between 2 and 6 as $P_T$, but more preferably, it is possible to utilize 2 as $P_G$, and to utilize 4 as $P_T$.

In FIG. 5, an example of a method of calculation of the ribosome binding score is shown as a flow chart.

First, one from among the start codon candidates which have already been found (ATG, GTG, TTG) is selected (S501), and, then, the distance d between the SD and the start codon is set to 12 (S502). This means that the score for upstream of the start codon candidate whose paired state is being investigated is set to be calculated for the 8 bases of the region from the 17th base to the 10th base upstream.

In S503, first the score for d=12 is calculated according to the setting in S502. In other words, the score for the 8 bases of the region from the 17th base to the 10th base upstream of the start codon for which the paired state is investigated is calculated by a predetermined method; and then, when in S504 the start codon is GUG or UUG, a penalty score is allocated in S505.

And thus, the steps from S503 to S505 is repeated by reducing the setting of d by one at a time, so as to obtain the score for the region (d=11) from the 16th base to the 9th base upstream of the start codon candidate, the score for the region (d=10) from the 15th base to the 8th base upstream of the start codon candidate, the score for the region (d=9) from the 14th base to the 7th base upstream of the start codon candidate, the score for the region (d=8) from the 13th base to the 6th base upstream of the start codon candidate, the score for the region (d=7) from the 12th base to the 5th base upstream of the start codon candidate, the score for the region (d=6) from the 11th base to the 4th base upstream of the start codon candidate, and the score for the region (d=5) from the 10th base to the 3rd base upstream of the start codon candidate, and the maximum value among these is defined as the ribosome binding score of the above described start codon candidate.

Thus, the steps of S501 to S506 are repeated for all the start codon candidates, and a ribosome binding score is obtained for all the start codon candidates.

When a specific example of the calculation of the ribosome binding score is presented, as shown in FIG. 6, the ribosome binding score of trpL gene which is the first gene in the Escherichia coli tryptophan operon is calculated as 12.

The ribosome binding score which is obtained in this manner, as described above, can be used for selecting the start codon whose score exceeds a threshold value $V_1$ as the start codon.

The ORF-A whose translation start codon is the codon which is selected in this manner is determined as a true coding region, and it is determined that said ORF-A and the ORF-B is form a single transcription unit, and the results of determination are written into the memory.

For an ORF-A which is not decided to have a possibility to form a polycistronic transcription unit in process (b), or for an ORF-A whose start codon can not to be determined in the process (e), the truth or falsity as a start codon is decided from the candidate for start codon, including the provisional start codon which yields the longest ORF, which is present in the 5' terminal region of the longest ORF by the process (f). The reason is that, if probability that the next start codon and stop codon appear at the upstream of the correct start codon is predicted, the probability that a plurality of ATG, GTG, or TTG codons appear without a stop codon appearing is low. Generally, ribosome binding scores of the number N of candidates for start codon from the 5' terminal (where N is an integer from 5 to 20) are obtained by the above described method or the like (S309) and compared the score with the threshold value $V_1$ (the threshold value $V_1$ is a value which is greater than the threshold value $V_3$), and it is possible to determine the candidate for start codon for which said score exceeded the threshold value $V_1$ first as the start codon (S310). However, since translation starting is initiated even when the SD sequence is AAG or GG, it is also desirable, when it is not possible to determine the start codon when the threshold value $V_1$ has been utilized, to determine the start codon again (S311) by using a plurality of threshold values which have values less than $V_1$, in a stepwise manner. More desirably, in addition to the threshold value $V_1$, it is possible to use a threshold value $V_2$ which is less than $V_1$, and a threshold value $V_3$ which is less than $V_2$. In this case, it is desirable to utilize an integer from 5 to 13 as the value of $V_2$, and to utilize an integer from 4 to 12 as the value of $V_3$.

The ORF-A whose translation start codon is the selected candidate which corresponds to said ribosome binding sequence is determined as a true coding region, and the results of determination are written into the memory (S312).

Here, it is desirable to investigate the presence of a ribosome binding site at a plurality of threshold values which has values smaller than $V_1$, and, when it is not possible to determine a translation start codon, not to take ORF-A as including a true coding region (S313).

By process (g), from the results of determination of the process (c), (e), or (f), the positions of the start codon and the stop codon, the coding region, and the transcription unit are confirmed, and the genetic structure is determined (S312).

It is possible to output the positions of the start codon and the stop codon, and the information related to the CDS which is included in the various transcription units, by displaying them upon the display, by printing them by outputting them to a printer, or by recording them upon a recording medium. In the case of a start codon which is upon the plus strand, the position of the start codon can be given by the position of first base of 3 bases of the codon, while, in the case of a start codon which is upon the minus strand, it can be given by the position of first base or by the position of the last base of 3 bases of the codon. In the case of a stop codon which is upon the plus strand, the position of the stop codon can be given by the position of first base or by the position of last base of 3 bases of the codon, while, in the case of a stop codon which is upon the minus strand, it can be given by the position of the first base of 3 bases of the codon.

In relation to the structure of the transcription unit, it is necessary to specify whether the transcription unit which has been determined is a monocistron, or whether it is a polycistron. Furthermore, in the case of a polycistron, it is also desirable to output information which distinguishes between the first CDS, the last CDS, and the CDSs which are present internally between the first CDS and the last CDS. As an ideal example of a method of output in relation to the information about the structure of a transcription unit, it is possible to allocate the label "1" to a CDS of a monocistron, and to allocate the labels "2", "4", and "3" respectively to the first CDS, to the last CDS, and to the CDSs which are present internally between the first CDS and the last CDS.

Apart from the above described "method of determining coding regions aimed at transcription unit structure", it is also possible to determine the genetic structure by utilizing a variant of this method. This variant of this method is a method of determining a genetic structure for a prokaryote which includes the processes (a1) through (g3) described below.

(a1): From the nucleotide sequence information of the prokaryote, a translation stop codon is set, and a provisional translation start codon which yields the longest ORF is set based upon said translation start codon.

(a2): After selection, from the ORFs which have been obtained by the process (a1), one for which the length of said ORF (the positional difference between the position of the first translation stop codon and the position of the first translation start codon) is greater than or equal to $L_O$ bases, the names of the first ORF, the second ORF, ... the K-th ORF are appended to the K ORFS which have been chosen, in order from the 5' terminal side at the position of their translation stop codons [here $L_O$ is an integer from 30 to 900, and K is an integer greater than or equal to 2].

(b1): When the provisional translation start codon of the I-th ORF which has been obtained by the process (a2) either is upstream of the translation stop codon of the J-th ORF which includes a coding region for which it has been decided that it is "a true coding region" by the process (g3) among the ORFs from the first ORF to the (I-1)-th ORF, or is within $D_S$ bases downstream of said translation stop codon, then it is decided that there is a possibility that the I-th ORF and the J-th ORF may create a polycistronic transcription unit [here, I is an integer from 1 to K, J is a positive integer less than I, and $D_S$ is an integer from 20 to 100].

(c1): Within the J-th ORF for which it has been decided in the process (b1) that there is a possibility of creating a polycistronic transcription unit with the I-th ORF, after having chosen the ORF for which the value of J is the minimum, when there is a candidate for the translation start codon in the "vicinity of the translation stop codon" of the J-th ORF, in other words, in "the region within $D_B$ bases in the downstream direction from the first T residue of said stop codon and within $U_B$ bases in the upstream direction from said T residue", then this candidate is determined as being the codon start codon of the I-th ORF [here, $D_B$ is an integer from 10 to 20, and $U_B$ is an integer from 3 to 15]. Here, if there are a plurality of candidates for the translation start codon, then a priority ranking is determined from the shorter ones, with the distance between each candidate and the translation stop codon of the J-th ORF taken as an indicator, and thereby the translation start codon of the I-th ORF is determined.

(d1): If it has not been possible to determined the translation start codon of the I-th ORF in the process (c1), then it is investigated as to whether or not a candidate for the translation start codon of the I-th ORF is present in a position in which it is possible to restart translation within the "coding region around the vicinity of the stop codon" of the J-th ORF.

(e1): When in the process (d1) a candidate for the translation start codon of the I-th ORF is present in said region, then, when the paired state between the mRNA sequence of 4 to 17 bases upstream of said candidate and the sequence (3'-UUCCUCC-5') within the 16S rRNA 3' terminal sequence which is involved in the binding with the mRNA, or between the mRNA sequence of 4 to 16 bases upstream of said translation start codon and the sequence (3'-UCCUCC-5') within the 16S rRNA 3' terminal sequence which is involved in the binding with the mRNA, is expressed as a numerical value according to the four rules described below:

(1) A pairing of G and C yields +4;
(2) A pairing of A and U yields +2;
(3) A pairing of G and U yields +1;
(4) When no pairing is recognized at a base pair which is adjacent to a base pair for which a pairing has been recognized, then this yields -1.

this numerical value is taken as a "score which shows the state of binding between the mRNA and the ribosome (the ribosome binding score)", and, when said score exceeds a threshold value V3, said candidate is determined as being the translation start codon of the I-th ORF. Here, if there is a plurality of candidates for the translation start codon, the "coding region around the vicinity of the stop codon" of the J-th ORF is divided into the two portions "the region downstream of said vicinity" and "the region upstream of said vicinity", and the preference for translation starting is determined in the order "the region downstream of said vicinity" and "the region upstream of said vicinity", and furthermore, if there is a plurality of translation start codons within each of these regions, then the candidates are selected stepwise by determining a priority ranking from the shorter ones, with the distance from the translation stop codon of the J-th ORF being taken as an indicator, and the one which exceeds the threshold value $V_3$ is determined as being the translation start codon of the I-th ORF.

(e2): If it has not been possible to determine the translation start codon of the I-th ORF in the process (d1) or in the process (e1), then return to the process (c1), and, among the J-th ORFs for which in the process (b1) it has been decided that there is a possibility of creating a polycistronic transcription unit with the I-th ORF, after having chosen the ORF for which the value of J is the next least, the work of determination of the translation start codon of the I-th ORF by the processes from the process (c1) through the process (e1) is repeated until the length of the I-th ORF becomes less than that of the $L_O$ amino acids [the $L_O$ here is the same value as the $L_O$ which was shown by the process (a2)].

(f1): For the I-th ORF for which it has not been decided in the process (b1) that there is a possibility of forming a polycistronic transcription unit, or for the i-th ORF for which it has not been possible to determine the translation start codon during the processes (c1) through (e2), after having searched for candidates for the translation start codon from the 5' terminal through at most N ones, including the provisional start codon which yields the longest ORF, a "ribosome binding score" is obtained for each of the candidates by doing the same as in the process (e1), and the one for which said score exceeds the threshold value $V_1$ is determined as being the translation start codon of ORF-A. Furthermore, if there is no candidate for which the threshold value V1 is exceeded, then one or more threshold values are set for which said score is smaller than $V_1$ and which include a threshold value $V_3$, and the translation start codon of ORF-A when this threshold value has been exceeded is determined in a stepwise manner [here, $V_1$ is an integer from 7 to 14 which is greater than the $V_3$ of the process (e2), and N is an integer from 5 to 20].

(g1): The I-th ORF for which it has not been possible to determine the translation start codon in the processes from the process (c1) through the process (f1) is decided to be a "false ORF".

(g2): The I-th ORF for which the process (c1), the process (d1), the process (e1), or the process (f1) has determined the translation start codon of its coding region is decided to include a "true ORF", and the positions of its translation start codon and its translation stop codon, its coding region, and its transcription units are confirmed, thus determining its genetic structure.

(g3): For all of the K ORFs from the first ORF to the K-th ORF, "true" or "false" of the coding regions is decided by the methods of the process (g1) and the process (g2), and, for all of the coding regions for which "true" has been decided, the positions of their translation start codons and their translation stop codons, their coding regions, and their transcription units are confirmed, thus determining their genetic structures [here, K is an integer greater than or equal to 2].

In the following, a variant of the above described "method of determining coding regions aimed at transcription unit structure", which is to be performed with a computer, will be explained in detail with reference to the flow charts of FIG. 7 and FIG. 8.

First, in the process (a1), from the nucleotide sequence information of a prokaryote which has been inputted by the input device, the CPU sets a translation stop codon, and sets the provisional translation start codon which yields the longest ORF based upon said translation stop codon (S701). The definitions of the translation stop codon, of the translation start codon, and of the longest ORF are the same as those described above.

In the process (a2), since the possibility is high, among the plurality of ORFs which have been obtained by the process (a1), that the shortest ORF is not a true coding region, the CPU calculates the length of each of the ORFs, and those ORFs which are greater than or equal to a fixed length are selected. Although it is possible to use from 30 to 900 bases as the length which is utilized for this selection, from 30 to 600 bases is desirable. For the ORFs which have been selected in this manner, it is desirable, in order to make the work in the subsequent processes convenient, to assign names to the first ORF, to the second ORF, ... . to the K-th ORF in order from the 5' terminal side, based upon the positions of the stop codons of each ORF [here, K is an integer greater than or equal to 2]. This data is stored in the memory (S702).

In the process (b1), when the provisional translation start codon of the i-th ORF which has been obtained by the process (a2) is upstream of the translation start codon of the j-th ORF among the ORFs from the first ORF to the (I-1)-th ORF (S704), or is within $D_S$ bases downstream of said translation stop codon (S705), the CPU decides (S706) that there is a possibility of the i-th ORF and the j-th ORF creating a polycistronic transcription unit [here I is an integer from 1 to K, and J is a positive integer smaller than I, while $D_S$ is an integer from 20 to 100]. Since it also may happen than the j-th ORF is an ORF for which it has been decided that it is a "false ORF" by the process (g3), in this case, the CPU decides that the j-th ORF dose not create a polycistron with the i-th ORF (S703). If a plurality of ORFs has been obtained upstream of the i-th ORF for which there is a possibility of creating a polycistron with the i-th ORF, then it is also desirable for this plurality of ORFs to be chosen out, for them to be ordered by rearranging them in the order by which their numbers are small, and for them to be stored in the memory.

The process (c1) is the same as the previously described process (c) (S708).

The process (d1) is the same as the previously described process (d) (S709).

The process (e1) is the same as the previously described process (e) (S801, S802). Furthermore, in this process, if the length of the coding region which is computed from the position of the start codon which has been determined by this process is extremely short, then it is desirable to decide that it is a "false coding region" and to discard it. For the length which is utilized for this decision, it is desirable to utilize the length which was utilized in the previously described process (a2).

In the process (e2), if it has not been possible to determine the translation start codon of the I-th ORF in the process (d1) or in the process (e1), return to the process (c1), and the CPU searches for another ORF for which there is a possibility of creating a polycistronic transcription unit with the I-th ORF. In other words, after having chosen the one, among the J-th ORFs for which it has been decided in the process (c1) that there is a possibility of creating a polycistronic transcription unit with the I-th ORF, for which the value of J is the next smallest (S803, S804), the CPU performs the job of determining the translation start codon of the i-th ORF by the processes from the process (c1) through the process (e1). It is also desirable to stop the repetition from the process (c1) through the process (e1) at the time point that the length of the I-th ORF has become small. For this length, it is desirable to utilize the length which was utilized in the previously described process (a2).

In the process (f1), for the I-th ORF for which in the process (b1) it was not decided that there is a possibility of creating a polycistronic transcription unit in the process (b1), or for the I-th ORF for which it was not possible to determine the translation start codon by the processes from the process (c1) through the process (e2), although a translation start codon is found by the CPU obtaining a "ribosome binding score", this method is the same as the previously described process (f) (S805-S808).

In the process (g1), the I-th ORF for which the CPU has not been able to determine the translation start codon by the processes of the process (c1) through the process (f1) is decided to be a "false ORF" (S809).

In the process (g2), although the I-th ORF for which the translation start codon of its coding region has been determined by the process (c1), the process (d1), the process (e1), or the process (f1) is decided as including a "true" coding region (S810), along with confirming the position of the translation stop codon and the coding region by confirmation of the position of the translation start codon, when in the process (b1) through the processes (e2) there appears a possibility that the I-th ORF may create a polycistron with an ORF upstream thereof, it is possible to confirm that the i-th ORF is a gene which participates in polycistron creation.

In the process (g3), the CPU decides upon the "truth" or the "falsity" of the coding regions for all the K ORFs from the first ORF to the K-th ORF [here K is an integer greater than or equal to 2] by the methods of the process (g1) and the process (g2), and accordingly is able to determine the genetic structure by confirming the positions of the translation start codons and the translation stop codons of all the coding regions for which it has been decided that they are "true coding regions", and also the coding regions and transcription units. Finally, the determination results which have been obtained are outputted via the output device (S811).

Using the above described method of determining a genetic structure, after having predicted the structure of the transcription units along with selecting the candidates for CDSs from the plus strand and the minus strand of the DNA of a prokaryotic cell, it is possible to enhance the accuracy of determination of the CDSs by deciding upon the truth or the falsity of CDSs or transcription units which mutually overlap one another, by the following method. That is, after having investigated the positional relationship of a plurality of CDSs and transcription units which have been selected, if there is a transcription unit P or a CDS-A with another transcription unit Q or another CDS-B which is present upon the same strand being included in that transcription unit P or that CDS-A, it is possible to decide whether it is a "false transcription unit" or a "false CDS". Furthermore, if there is a transcription unit P or a CDS-A with another transcription unit Q or another CDS-B which is present upon the complementary strand being included in that transcription unit P or that CDS-A, it is possible to decide whether it is a "false transcription unit" or a "false CDS". When the transcription unit P or the CDS-A overlaps with the other transcription unit Q or the other CDS-B which is present upon the complementary strand, it is possible to decide whether the one of the transcription units or CDSs whose length is the shorter is a "false transcription unit" or a "false CDS".

Furthermore, although the CDS or transcription units of plus strands, or the CDS or the transcription units of minus strands, , may be overlapped, if they have no relationship of mutual involvement, if the length of both of them is compared, it is possible to enhance the accuracy of CDS or transcription unit determination even by supposing that the shorter of them is "false CDS" or a "false transcription unit".

Since it is known that, with two coding regions which are adjacent upon the same strand and which meet one another, it sometimes happens that a portion of the 3' terminal side of the upstream coding region and a portion of the 5' terminal side of the downstream coding region mutually overlap, accordingly the mutual overlapping of the coding regions is investigated, and, when such an overlapping has been observed, it will be acceptable to take both of the coding regions as true coding regions. It should be understood that it is normally desirable for the length of this overlap to be 10% or less of the one of the coding regions whose length is the shorter. Furthermore, since it is also known that, for a coding region upon a plus strand and a coding region upon a minus strand, it sometimes happens that portions of their respective 3' terminal sides mutually overlap, therefore the mutual overlapping between the coding regions is investigated, and, when this type of overlap has been observed, it will be acceptable to take both of the coding regions as true coding regions. It should be understood that it is normally desirable for the length of this overlap to be 10% or less of the one of the coding regions whose length is the shorter.

FIG. 9 shows as a flow chart an example of the above described method for deciding upon the truth or the falsity of CDSs or transcription units which mutually overlap one another.

Referring to FIG. 9, in S901, the nucleotide sequence and structure data are inputted via the input device. When determining a large number of CDSs from DNA of large size (a nucleotide sequence of DNA of 1000 base pairs or more), the CDSs are selected by repetitively utilizing the above described "method of determining a genetic structure aimed at transcription unit structure" and so on for each of the ORFs.

In S902, for each of the plus strand and the minus strand, the transcription units are numbered and are arranged in order from the 5' terminal to the 3' terminal, and are stored in the memory.

In S903 through S908, first, each single transcription unit of the plus strand and the minus strand is called out from the memory, and when, for a transcription unit Q which is present upon the complementary strand of a transcription unit P is included in the transcription unit P, then it is decided that it is a "false transcription unit", and this operation is repeated until the processing is concluded for all the combinations.

By the way, in S909, from each of the plus strand and the minus strand, the transcription units which have been determined are numbered and arranged in order from the 5' terminal to the 3' terminal, and are stored in the memory.

In S910 through S916, first, each single CDS of the plus strand and the minus strand is called out from the memory, and when a CDS-A overlaps with another CDS-B which is present upon the complementary strand, then it is decided that the one of the transcription units or the CDSs whose length is the shorter is a "false transcription unit" or a "false CDS", and this operation is repeated until the processing is concluded for all the combinations.

Finally, the results of determination of transcription units which were obtained in S909 and the results of determination of CDSs which were obtained in S916 are outputted via the output device.

As shown in FIG. 9, first, along with performing the determination of the start codon for each ORF from the plus strand, the truth or the falsity as a CDS is decided upon, and, if a CDS appears for which it has been decided that it is a "true CDS", then it is possible to enhance the accuracy of CDS determination by investigating, by the above described method, whether or not said CDS includes another CDS which is present upon the same strand. It should be understood that, if the above described "method of determining a genetic structure aimed at transcription unit structure" is utilized, it is also possible to determined the structure of the transcription units, as well as that of the CDSs. In the same manner, after having determined the structure of the CDSs and the transcription units from the minus strand as well, by investigating, by the above described method, whether or not each transcription unit includes another transcription unit which is present upon the complementary strand, it is possible to enhance the accuracy of the determination of the transcription units by deciding that this transcription unit which has been included in another transcription unit is a "false transcription unit".

In recent years, the entire genome sequences of a large number of microbes have been determined, and the number is increasing from year to year. In this manner, with regard to the determination of the CDSs from the nucleotide sequence of a prokaryote, the necessity of determining the CDSs from the entire genome sequence is increasing. Accordingly, when determining a large number of CDSs from the nucleotide sequence of DNA of large size (DNA of 1000 base pairs or more), for each of the ORFs, it is desirable to select the CDSs, or to decide upon the truth or the falsity as CDSs, by repeatedly utilizing the above described method of determining a genetic structure. As a method of repeatedly utilizing the method of determining a genetic structure, there is offered a method of, after having arranged the ORFs which have been directed upon the same strand by the positions of their stop codons, examining whether their start codons are present in order from the 5' terminal side ORF, and deciding upon the truth or the falsity as CDSs. An example thereof is shown in FIG. 10.

Referring to FIG. 10, in S1001, the nucleotide sequence which is to be analyzed is inputted by the input device.

In S1002 and S1003, the same processing is performed by the CPU as in the above described S701 and S702.

In S1004 and S1005, the CPU performs the same processing as in the above described S703 through S709 and S801 through S811, and, along with storing the results of determination in the memory, for the CDSs for which it has decided that they are true CDSs, proceeds to the following decision processing in order to enhance the accuracy of their determination.

In S1006 through 1011, the CPU investigates the presence or absence of an overlap with the upstream CDS and its length and its inclusion, and, if the amount of overlapping is great, decides the shorter of the CDSs as being false, while if it include the upstream CDS, it decides that this upstream CDS is false.

This processing is repeated until the processing in S1002 has been completed for all the ORFs, and the decision results are stored in the memory, and finally the results of decision are outputted via the output device (S1013).

According to the method shown in FIG. 10, first, along with performing the determination of the start codon for each of the ORFs from the plus strand, its truth or its falsity as a CDS is decided upon, and, if a CDS appears for which it has been decided that it is a true CDS, then it is possible to enhance the accuracy of CDS determination by investigating by the above described method whether or not said CDS includes another CDS which is present upon the same strand. In the same manner, after having determined the CDSs from the minus strand as well, by investigating

by the above described method whether or not each CDS or transcription unit includes another CDS or transcription unit which is present upon the complementary strand, it is possible to enhance the accuracy of CDS determination by deciding that the CDS or the transcription unit which has been included in the other CDS or transcription unit is a "false CDS" or a "false transcription unit".

2. A method of determining a genetic structure utilizing a shadow discrimination function.

Normally, it often happens that a "false CDS" is present which overlaps upon the complementary strand with a CDS of a prokaryotic cell, and the existence of these "false CDSs" causes a difficulty with enhancement of CDS determination accuracy. This fact that "false CDSs" appear upon the complementary strand is often termed "gene shadow". With determination methods for CDSs which have been developed up till now, since they are aimed at the frequency of use of combinations of characteristic nucleotide sequences or codons in each "true" CDS of the prokaryote, and they discriminate these "gene shadows", accordingly it is necessary to find out the true CDSs by a different method.

However, as described below, with the present invention, based upon the ORF information which has been determined by the use of the above described "method of determining a genetic structure aimed at transcription unit structure" or the like, by investigating the truth or the falsity of the CDSs by utilizing a method of calculating for discriminating these "gene shadows", it is envisaged that it is possible to enhance the accuracy of CDS determination, even though no information as to the correct CDSs is available in advance. In other words, it is possible to enhance the accuracy of CDS determination by first, based upon the information for a plurality of CDS which has been determined by utilizing the above described "method of determining a genetic structure aimed at transcription unit structure" or the like, after having selected k combinations of codons for which the frequency of appearance of these codons within CDSs which have been decided to be "true CDSs" is high, and the frequency of appearance of the codons which have the complementary sequence to the 3-base sequence of said codons in said CDSs is low, deciding upon the truth or the falsity of a CDS-A by utilizing a method of calculation (hereinafter termed a "shadow discrimination function") which makes it possible to compare the "number of times the k types of codon whose frequency of appearance is high appear in the CDS-A" and the "number of times the k types of codon whose frequency of appearance is low appear in the CDS-A" [here, k is an integer greater than or equal to 5 and less than or equal to 20].

In the following this method will be abbreviated as "a method of determining a genetic structure using a shadow discrimination function".

The method of determining a genetic structure using a shadow discrimination function of the present invention is not limited to CDSs which have been determined by the above described "method of determining a genetic structure aimed at transcription unit structure"; it can also be applied to CDSs which have been determined by any known method. And although, by applying the "method of determining a genetic structure using a shadow discrimination function" of the present invention, it is possible to enhance the accuracy of determination for CDSs which have been determined by a method of determining coding regions which has been known from the past - such as, for example, GenMark [Borodovsky, M & Mcininch, J.: Computers Chem. Vol. 17, 123-133 (1993)], GenMark.hmm [Lukashin, A.V. & Borodovsky, M.: Nucleic Acids Research Vol. 26, 1107-1115 (1998), Besemer, J. & Borodovsky, M.: Nucleic Acids Research Vol. 27, 3911-3920 (1999)] Glimmer [Salzberg, S. et al.: Nucleic Acids Research Vol. 26, p. 544-548 (1998), Delcher, A.L.: Nucleic Acids Research Vol. 27, p. 4636-4641 (1999)], CRITICA [Badger, J.H. & Losen, G.J. et al.: Mol. Biol. Evol. Vol. 16, p. 512-524 (1999)], ORPHELUS [Frishman, M. et al.: Nucleic Acids Research Vol. 26, p. 2941-2947 (1998)], or GenMarkS [Besemer, J., Lomsadze, A. & Borodovsky, M.: Nucleic Acids Research Vol. 29, 2607-2618 (2001)] and the like - nevertheless it is not limited thereto.

This "method of determining a genetic structure using a shadow discrimination function" can be implemented by causing a computer to perform processing for:

(k) among a plurality of T coding regions for the prokaryote which have already been determined and have been inputted via an input means, investigating the type of the codons which are utilized and the number thereof, and, from among them, selecting k types of combination of codons for which "the appearance frequency of some codon is high, and the appearance frequency of a codon which has the complementary sequence of the 3-base sequence of said codon is low", and storing them in the memory;

(l) from the data of a coding region A, which is assumed to be the coding region, and which has been inputted via an input means, measuring the frequency of appearance of the above described selected codons in said coding region A, and, by comparing together the above described "number of appearances in a coding region A which is assumed to be a coding region of the k types of codons whose frequency of appearance is high" and the "number of appearances in said coding region A of the k types of codons whose frequency of appearance is low", deciding upon the truth or falsity of said coding region A [Here k is an integer greater than or equal to 5 and less than or equal to 20];

and displaying the results of the above described decision upon an output device.

It is also possible to utilize any calculation method as the "shadow discrimination function", which is the method of calculation which makes it possible to compare the above described "number of times the k types of codon whose frequency of appearance is high appear in the CDS-A" and the "number of times the k types of codon whose frequency of appearance is low appear in the CDS-A", provided that it includes a comparison between the number of time the former codon appears and the number of times the latter codon appears. As an example of a suitable method for calculating this "shadow discrimination function", if the number of times the former codon appears is supposed to be "H" and the number of times the latter codon appears is supposed to be "L", there are offered: H/L, L/H, (H/L+1), (L/H+1), 1/(H/L+1), 1/(L/H+1), 2H/(H+L), and the like. Among these methods of calculation, it is possible to enhance the accuracy of CDS determination by deciding that said CDS-A is a "false CDS" by using "the reciprocal of the sum obtained by adding 1 to the ratio of the number of the latter to the number of the former", and furthermore when the value of said reciprocal is less than a fixed value [here k is an integer which is greater than or equal to 5 and is less than or equal to 20].

In the following, a concrete example will be explained of this "shadow discrimination function" method.

First, when the 64 types of codons:

TTA, CTA, TCA, TTT, TTC, TTG, TCT, TCC, TCG, TAT, TAC, TGT, TGC, TGG, CTT, CTC, CTG, CCT, CCC, CCG, CAT, CAC, CGT, CGC, ATT, ATC, ACT, ACC, AAC, AGC, GTC, GCC, TAA, TAG, TGA, AAA, GAA, CAA, AGA, GGA, CGA, ATA, GTA, ACA, GCA, CCA, AAG, GAG, CAG, AGG, GGG, CGG, ATG, GTG, ACG, GCG, AAT, GAT, AGT, GGT, GTT, GCT, GAC, and GGC

have been lined up in order, the 3 base of the i-th codon (where i is less than or equal to 32) has the complementary sequence to the 3 base of the (i+32)-th codon. When the frequency at which the 3 base of the i-th codon appears in true CDSs is high, and the frequency at which the 3 base which corresponds to the (i+32)-th codon appears in true CDSs is low, the frequency with which the 3 base of the i-th codon appears in the nucleotide sequence of the opposite strand becomes low, and the frequency at which the 3 base which corresponds to the (i+32)-th codon appears in the nucleotide sequence of the opposite strand becomes high.

Due to this fact, when the codons which appear in a true CDS have been analyzed, the difference between the frequency of appearance of the 3 base of the i-th codon and the frequency of appearance of the 3 base of the (i+32)-th codon is obtained, and, the greater this difference is, the likelier does it become that the i-th codon appears in a true CDS, and furthermore the likelier does it become that the (i+32)-th codon appears in the nucleotide sequence of the opposite strand of a true CDS.

In other words, T CDSs are selected as true CDSs, and, when the number of times that the i-th codon appears in the t-th CDS which has been selected is termed

$C_j^t$

then the above described difference $y_i$ and $y_{i+32}$ is given by the following equation:

$$y_i = \left(\sum_{t=1}^{T} C_i^t - \sum_{t=1}^{T} C_{i+32}^t\right) \Big/ \sum_{t=1}^{T} \sum_{j=1}^{64} C_j^t \qquad (2)$$

$$y_{i+32} = \left(\sum_{t=1}^{T} C_{i+32}^t - \sum_{t=1}^{T} C_i^t\right) \Big/ \sum_{t=1}^{T} \sum_{j=1}^{64} C_j^t \qquad (3)$$

Next, the values of $y_i$ and $y_{i+32}$ are calculated (where i is an integer less than or equal to 32), and the above described 64 types of codons are arranged in the order of magnitude of this value. When the leading k codons in order of magnitude of the value of $y_i$ or $y_{i+32}$ are chosen, the value of the shadow discrimination function of the n-th CDS (hereinafter abbreviated as Sd) is given by the following equation:

$$Sd_n = 2 \times \sum_{i=1}^{k} C_i^n \Big/ \left(\sum_{i=1}^{k} C_i^n + \sum_{i=65-k}^{64} C_i^n\right) \qquad (8)$$

(k is an integer from 5 to 20)
Here, when

$$\left(\sum_{i=1}^{k} C_i^n + \sum_{i=65-k}^{64} C_i^n\right)$$

is zero, the value of Sdn is taken as being 1.

By doing the following, based upon the value of this shadow discrimination function Sd, it is possible to decide, when the value of Sd for some ORF exceeds a threshold value (for example 1.0) which is specified in advance, that it is a "true" CDS. Furthermore, when two CDSs overlap, or are in an inclusion relationship, it is possible to decide upon the truth or the falsity of the CDSs by calculating the Sd value of each CDS, and by comparing their values. When deciding upon the truth or the falsity, not of a CDS, but of a transcription unit, after having computed the Sd value based upon all the codons of the CDSs which make up the transcription unit, it is possible to decide upon the truth or the falsity of the transcription unit from this Sd value.

In the above described method, as shown in FIG. 14:

(m) constructing a codon table by arranging the 64 types of codons so that the 3-base sequence of the i-th codon has the complementary sequence to the nucleotide sequence of the (i+32)-th codon, and storing it in the memory (S1401)

(n) inputting the nucleotide sequence of T coding regions of a prokaryote which have already been determined, and, when the number of times that the i-th codon appears in the t-th coding region is taken as

$C_i^t$

obtaining $y_i$ from the equation (2) below and $y_{i+32}$ from the equation (3) below following it:

$$y_i = \left(\sum_{t=1}^{T} C_i^t - \sum_{t=1}^{T} C_{i+32}^t\right) \Big/ \sum_{t=1}^{T} \sum_{j=1}^{64} C_j^t \qquad (2)$$

$$y_{i+32} = \left(\sum_{t=1}^{T} C_{i+32}^t - \sum_{t=1}^{T} C_i^t\right) \Big/ \sum_{t=1}^{T} \sum_{j=1}^{64} C_j^t \qquad (3)$$

(S1402)

(o) calling out the codon table which was obtained in the step (m) from the memory, setting up a correspondence between the $y_i$ and $y_{i+32}$ for the codons in the table, and, after having rearranged the sequence of the codons in the table in the order of magnitude of the $y_i$ and the $y_{i+32}$ (S1403), choosing k leading codons for which the value of $y_i$ or of $y_{i+32}$ is large, and obtaining the value of $Sd_A$ for a coding region A by the following equation (4):

$$Sd_A = 2 \times \sum_{i=1}^{k} C_i^A \Big/ \left(\sum_{i=1}^{k} C_i^A + \sum_{i=65-k}^{64} C_i^A\right) \qquad (4)$$

[Here, when

$$\left(\sum_{i=1}^{k} C_i^A + \sum_{i=65-k}^{64} C_i^A\right)$$

is zero, the value of $Sd_A$ is taken as being 1](S1404);

(p) When the value of $Sd_A$ for the coding region A which has been obtained by the above described processing is greater than a threshold value $S_1$, then said coding region is take as a true coding region, while, when said value of $Sd_A$ is less than the threshold value S1, it is taken as a false coding region (S1405) [here T is an integer greater than or equal to 2, i is a positive integer less than or equal to 32, j is a positive integer less than or equal to 64, t is a positive integer less than or equal to T, k is an integer from 5 to 20, and $S_1$ is a value from 0.8 to 1.8].

An implementation is possible in which a computer is caused to execute processing to output the above described decision results via an output device.

As an example of taking advantage of this shadow discrimination function Sd for deciding upon the truth or the falsity of two CDSs or transcription units, after having determined long CDSs (the length of the polypeptide for which the CDSs code is greater than or equal to L amino acids, where L is a positive integer greater than or equal to 100) by the above described "method of determining a genetic structure aimed at transcription unit structure", the value of the above described shadow discrimination function Sd is obtained from the sequence information of these CDSs, and it is possible to enhance the accuracy of CDS determination by deciding upon the truth or the falsity of the CDSs by using said Sd value as a threshold value. In this method, as a method of determining the CDSs, it will be acceptable to utilize some method other than the above described "method of determining a genetic structure aimed at transcription unit structure".

When a CDS or a transcription unit upon the plus strand and a CDS or a transcription unit upon the minus strand overlap, or are in an inclusion relationship, if the lengths of them both are greatly different, it will be acceptable to decide that the one of these CDSs or transcription units whose length is the shorter is a "false CDS" or a "false transcription unit". However, if their lengths do not differ greatly, then it is possible to decide upon the truth or the falsity of the CDSs or transcription units by the above described method, and in particular by comparing the values of the shadow discrimination function for the individual CDSs or transcription units. More desirably, it is possible to compare together the length $L_A$ (in base pairs) of the CDS-A or of the transcription unit A and the length $L_B$ of the CDS-B or of the transcription unit, and to decide that the CDS-B or the transcription unit B is a "false CDS" or a "false transcription unit" when $L_B$ is less than or equal to $T_P$% of $L_A$ [here $T_P$ is a positive integer from 30 to 95]. Furthermore, when $L_B$ exceeds $T_P$% of $L_A$, it is possible to decide upon the truth or the falsity of the CDS-A or of the transcription unit A; or the CDS-B or of the transcription unit B by the above described method which uses the "shadow discrimination function" [here $T_P$ is a positive integer from 30 to 95].

This method of deciding upon the truth or the falsity of a CDS-A or a transcription unit A and a CDS-B or a transcription unit B is not limited to CDSs which have been determined by the above described "method of determining a genetic structure aimed at transcription unit structure"; it can also be applied to CDSs or transcription units which have been determined by any known method. And although, by applying the above described method of deciding upon the truth or the falsity of a CDS-A or a transcription unit A and a CDS-B or a transcription unit B to CDSs or transcription units which have been determined by a method which has been known from the past - such as, for example, GenMark [Borodovsky, M & Mcininch, J.: Computers Chem. Vol. 17, 123-133 (1993)], GenMark.hmm [Lukashin, A.V. & Borodovsky, M.: Nucleic Acids Research Vol. 26, 1107-1115 (1998), Besemer, J. & Borodovsky, M.: Nucleic Acids Research Vol. 27, 3911-3920 (1999)], Glimmer [Salzberg, S. et al.: Nucleic Acids Research Vol. 26, p. 544-548 (1998), Delcher, A.L.: Nucleic Acids Research Vol. 27, p. 4636-4641 (1999)], CRITICA [Badger, J.H. & Losen, G.J. et al.: Mol. Biol. Evol. Vol. 16, p. 512-524 (1999)], ORPHELUS [Frishman, M. et al.: Nucleic Acids Research Vol. 26, p. 2941-2947 (1998)], or GenMarkS [Besemer, J., Lomsadze, A. & Borodovsky, M.: Nucleic Acids Research Vol. 29, 2607-2618 (2001)] and the like - it is possible to determine their truth or the falsity, nevertheless it is not limited thereto.

In the method shown in FIG. 10, in the processing for deciding upon the truth or the falsity of CDSs or transcription units which mutually overlap with one another, it is possible to perform the decision by utilizing the above described "shadow discrimination function".

As described above, in recent years, the requirement has increased for determining the CDSs from the entire genome sequence of a microbe at high speed and moreover at high accuracy. In the following, an example of a method for enhancing the accuracy of CDS determination by taking advantage of the value of the above described shadow discrimination function Sd when determining the CDSs from the entire genome sequence of a prokaryote will be described in detail.

When determining a large number of CDSs from the nucleotide sequence of DNA of large size (DNA of 1000 base pairs or more), for each of the ORFs, the CDSs are selected by repeatedly utilizing the above described "method of determining a genetic structure aimed at transcription unit structure" or the like.

As shown in FIG. 9, first, along with performing determination of the start codon for each ORF from the plus strand, its truth or its falsity as a CDS are decided upon, and, if a CDS has appeared for which it has been decided that it is a "true CDS", then it is possible to enhance the accuracy of CDS determination by investigating, with the above described method, whether or not said CDS includes another CDS upon the same strand. It should be understood that, if the above described "method of determining a genetic structure aimed at transcription unit structure" is employed, it is possible to determine, not only the structure of the CDSs, but also the structure of the transcription units. After having determined the structure of the CDSs and of the transcription units from the minus strand in the same manner, it is possible to enhance the accuracy of transcription unit and CDS determination by investigating by the above described method whether or not each transcription unit includes another transcription unit upon the complementary strand, and by deciding that a transcription unit which has been included in another transcription unit is a "false transcription unit". Based upon the information for the CDSs which has been determined in this manner, k combinations (where k is an integer from 5 to 20) of the two types of codons are selected: ones for which the frequency of the codons within CDSs for which it has been decided that they are "true CDSs" is high, and ones for which the frequency of appearance of codons which have the complementary sequence to the 3-base sequence of said codons in said CDSs is low. Based upon the "k types of codons of which the frequency of appearance is high" and the "k types of codons of which the frequency of appearance is low" which have been selected in this manner, it is possible to obtain the value of the shadow discrimination function Sd for each of the CDSs by the above described method.

Next, the structure of the CDSs and the transcription units is determined for a second time from the plus strand and the minus strand, using the above described "method of determining a genetic structure aimed at transcription unit structure".

As shown in FIG. 11, by taking advantage of the value of the above described shadow discrimination function Sd for the $U_{P1}$ transcription units which have been selected from the plus strand and the $U_{M1}$ transcription units which have been selected from the minus strand, it is possible to decide upon the truth or the falsity of the transcription units, and to enhance the accuracy of determination of the transcription units and the CDSs

If the CDSs have been determined by a method other than the above described "method of determining a genetic structure aimed at transcription unit structure", when the distance between two adjacent CDSs, in other words, the distance between the stop codon of the upstream CDS and the start codon of the downstream CDS, is $D_C$ base pairs (where $D_C$ is an integer from 30 to 120), it is possible to create a transcription unit by deciding upon the creation of a polycistron.

For a transcription unit which has been created in this manner, it is possible to decide upon the truth or the falsity of transcription units by taking advantage of the value Sd of the above described shadow discrimination function, thus making it possible to enhance the accuracy of CDS and transcription unit determination.

Referring to FIG. 11, in S1101, the data for the nucleotide sequence which is to be analyzed is inputted via the input device.

In S1102, the transcription units upon the plus strand and the minus strand are selected, and based upon the positions of their stop codons, it is lined up from the 5' terminal to the 3' terminal and are stored in the memory. The selecting and lining up of the transcription methods may be performed by any of the above described methods.

Next, the CPU calls out the transcription units of the plus strand and the minus strand which have been stored in S1102 one at a time in order from the 5' terminal, and investigates whether or not they are mutually included (S1103, S1106), and, if they are thus included, compares the lengths of the two transcription units (S1104, 1107), and, if one of them is less than $P_1$% of the other, calculates the shadow discrimination function for each of them, compares them (S1105, S1108), and takes the one whose shadow discrimination function is the smaller as being false.

This operation is performed for the transcription units which have been stored in the memory in S1102, and as a result, the transcription units of the plus strand and the minus strand which have not been taken as false are selected, based upon the positions of their stop codons, it is lined up as standard from the 5' terminal to the 3' terminal, and are stored in the memory (S1109).

Next, the CPU calls out the transcription units of the plus strand and the minus strand which have been stored in the memory in S1109 one at a time in order from the 5' terminal, and investigates whether or not their 5" terminal sides and 3" terminal sides are overlapped (S1110, S1113), and, if they do thus overlap, compares the lengths of the two transcription units (S1111, 1114), and, if one of them is less than $P_1$% of the other, calculates the shadow discrimination function for each of them, compares them (S1112, S1115), and takes the one whose shadow discrimination function is the smaller as being false.

This operation is performed for the transcription units which have been stored in the memory in S1109, and as a result, the transcription units of the plus strand and the minus strand which have not been taken as false are selected, and the results thereof are outputted via the output device (S1116).

3. A method of determining a genetic structure aimed at the GC content of the bases in the codons

When determining CDSs from a nucleotide sequence of a prokaryote in which the GC content is high, it is difficult to determine the CDSs at high accuracy, since a large number of long ORFs are present. With the present invention it is apparent that, as a characteristic of the CDSs of a prokaryote whose GC content exceeds 50%, the GC content of the first and the third bases of the codons within said CDSs is high, and a "method of determining a genetic structure aimed at the GC content of the bases in the codons" has been conceived of which takes advantage of this characteristic for enhancing the accuracy of CDS determination. In other words, this is a method which, after having calculated said content using a calculation expression which takes into account the contents of the first and the third G residue and C residue of the codons within said CDS, enhances the accuracy of determination of the CDSs of the prokaryote by deciding that said CDS is a "false CDS" when said content is less than a fixed value. Although it is possible to apply this method when the value of the GC content of the nucleotide sequence is greater than or equal to 50%, it is more desirable for it to be greater than or equal to 60%.
It is possible to utilize any type of calculation expression as the calculation expression for this method, provided that it is a calculation expression which yields a content of the first and the third G residue and C residue of the codons within said CDS; and, as examples of suitable such calculation expressions, it is possible to utilize an "expression obtained by dividing the total of the first and the third G residues and C residues of all the codons within said CDS by the number of bases of all the codons", an "expression obtained by dividing said total by the total of G residues and C residues of all the codons", or an "expression obtained by dividing said total by the total of the second G residues and C residues of all the codons". More desirably, a calculation expression is utilized, for which it is difficult for the value of said calculation expression to receive influence from the GC content of the nucleotide sequence. As an "expression obtained by dividing the total of the first and the third G residues and C residues of all the codons within said CDS by the total of bases of G residue and C residue of all the codons", for the i-th CDS, it is possible to utilize the value of GCi (hereinafter abbreviated as the "GC function" or GC) which is computed by the expression (5) below:

[Here, when the r-th base (r=1, 2, 3) of the n-th codon of the i-th CDS is b (b=1, 2, 3, 4), then

$$GC_i = (y^{i(1)} + y^{i(3)}) \Big/ \sum_{r=1}^{3} y^{i(r)} \quad \text{where} \quad y^{i(r)} = \sum_{n=1}^{N_i} \sum_{b=1}^{4} x_{n(b)}^{i(r)} \qquad (5)$$

where

$$\chi_{n|b|}^{i|r|} \text{ is } \chi_{n|b|}^{i|r|} = 1 \text{ (b= 1 or 2)}$$

$$\chi_{n|b|}^{i|r|} = 0 \text{ (b= 3 or 4)}$$

and, at this time, when the r-th base of the n-th codon of the i-th CDS is G, C, A, or T, then b is respectively 1, 2, 3, or 4. It should be understood that i and n are positive integers, while $N_i$ is the total of the codons in the i-th CDS (excluding its stop codon).

When the value of the "GC function" of a CDS whose GC content is 50% is obtained, on average it is 2/3 (0.66666). In other words, the value of the "GC function" is a value scattered around 2/3. Here, it will be understood that the value of the "GC function" of a CDS whose GC content exceeds 60% exceeds 2/3 for almost all CDSs. Accordingly, when discriminating the truth or the falsity of a CDS using the above described "GC function", it is desirable to utilize a numerical value within the range of 0.6 to 0.75 as a fixed value.
The "method of determining a genetic structure aimed at the GC content of the bases in the codons" of the present invention is not limited to CDSs which have been determined by the above described "method of determining a genetic structure aimed at transcription unit structure"; it can also be applied to CDSs or transcription units which have been determined by any known method. And although, by applying the "method of determining a genetic structure aimed at the GC content of the bases in the codons" of the present invention to CDSs which have been determined by a method of determining coding regions which has been known from the past - such as, for example, GenMark [Borodovsky, M & Mcininch, J.: Computers Chem. Vol. 17, 123-133 (1993)], GenMark.hmm [Lukashin, A.V. & Borodovsky, M.: Nucleic Acids Research Vol. 26, 1107-1115 (1998), Besemer, J. & Borodovsky, M.: Nucleic Acids Research Vol. 27, 3911-3920 (1999)], Glimmer [Salzberg, S. et al.: Nucleic Acids Research Vol. 26, p. 544-548 (1998), Delcher, A.L.: Nucleic Acids Research Vol. 27, p. 4636-4641 (1999)], CRITICA [Badger, J.H. & Losen, G.J. et al.: Mol. Biol. Evol. Vol. 16, p. 512-524 (1999)], ORPHELUS [Frishman, M. et al.: Nucleic Acids Research Vol. 26, p. 2941-2947 (1998)], or GenMarkS [Besemer, J., Lomsadze, A. & Borodovsky, M.: Nucleic Acids Research Vol. 29, 2607-2618 (2001)] and the like - it is possible to enhance the accuracy of their determination, nevertheless it is not limited thereto.

The above described "method of determining a genetic structure aimed at the GC content of the bases in the codons", as shown in FIG. 15, can be implemented by causing a computer to perform the processing of: from the data (S1501) of a coding region which has already been selected of a prokaryote for which the GC content exceeds 50% and which has been inputted via an input device, calculating the content of the first and the third G residue and C residue of the codons within the above described coding region by utilizing a predetermined calculation expression (S1502), and, when the content which has been obtained by this calculation is less than a fixed value, deciding that said coding region is a "false coding region" (S1503), and outputting the results of this decision via an output device.

When determining a CDS, it may happen that the accuracy of CDS determination is deteriorated by selecting a false start codon. When a codon which is upstream of the true start codon of a CDS has been selected as its start codon, the false CDS comes to be formed as linked to the 5' terminal of the true CDS. Thus, after having computed the content of the first and the third G residue and C residue of the codon of the 5' terminal side region of said CDS by utilizing a calculation expression which yields said content, when said content is less than a fixed value, the possibility is high that the 5' terminal side region is not a portion of the true CDS. In such a case, it is possible to enhance the determination accuracy of the start codon by again performing the search for the translation start codon in the downstream direction from this start codon. It is also possible to utilize any method as a method for again searching for this start codon. For example, there may be offered a method of determining the start codon by taking the fact that a ribosome binding sequence is present upstream of the start codon as an indicator, or the method of determining the start codon which is used during the "method of determining a genetic structure aimed at transcription unit structure" of the present invention. As a calculation expression which yields the content of the first and the third G residue and C residue of the codons of the 5' terminal side region of said CDS, it is possible to utilize the above described calculation expressions. Furthermore, as the 5' terminal side region, a region of length from 30 to 300 base pairs is desirable.

The above described method can be implemented by causing a computer to perform the processing of: from the data of a coding region which has already been selected of a prokaryote for which the GC content exceeds 50% and which has been inputted via an input device, calculating the content of the first and the third G residue and C residue of the codons of the region of the 5' terminal side of the above described coding region by utilizing a predetermined calculation expression; and, when the content which has been calculated is less than a fixed value, deciding that the translation start codon of said coding region is a "false translation start codon", and, along with outputting the results of this decision via an output device, also calling out the nucleotide sequence data of the above described coding region which has been inputted via the input device, and again searching for a translation start codon which is present downstream of said false translation start codon.

The above described "method of determining a genetic structure aimed at the GC content of the bases in the codons" can be utilized in combination with a different method of CDS determination. Desirably, it is possible to enhance the accuracy of CDS determination for a prokaryote by utilizing the "method of determining a genetic structure aimed at transcription unit structure" and the "method of determining a genetic structure using a shadow discrimination function" of the present invention.

Although it also may happen that, when determining the CDS of a prokaryotic cell, the accuracy of CDS determination is not enhanced by combining two per se known method of determining a genetic structures, in many cases, it is possible further to enhance the accuracy of CDS determination by combining a "method of deciding upon the truth or the falsity of a CDS by utilizing the coding potential" which has been computed by using the appearance frequency and the codon use frequency of the nucleotide sequence of the true CDS and a possibility process model such as a Markov model or a hidden Markov model or the like with any of the "method of determining a genetic structure aimed at transcription unit structure", the "method of determining a genetic structure using a shadow discrimination function", or the "method of determining a genetic structure aimed at the GC content of the bases in the codons" of the present invention.

Although, as an example of said "method of deciding upon the truth or the falsity of a CDS by utilizing the coding potential", it is possible to suggest a method of, based upon the nucleotide sequence of T CDSs of the prokaryote which have already been determined, deciding upon the truth or the falsity of said CDS-A by utilizing a calculation expression which is capable of comparing the "number of times m types of codons whose frequency of appearance is high appear in the CDS-A" and the "number of times m types of codons whose frequency of appearance is low appear in the CDS-A" in the T CDSs, it would also be possible to utilize any calculation expression, provided that it were a calculation expression which gives a coding potential [here, T is a integer greater than or equal to 2, and m is an integer greater than or equal to 5 and less than or equal to 20].

As an example of said calculation expression, although, as a calculation expression which is capable of comparing the "number of times m types of codons whose frequency of appearance is high appear in the CDS-A" and the "number of times m types of codons whose frequency of appearance is low appear in the CDS-A", it is possible to suggest "the reciprocal of adding 1 to the ratio of the latter number of times to the former number of times", a "Cd value" which is an example of this reciprocal is shown below:

$$Cd_A = 2 \times \sum_{i=1}^{m} C_i^A \Big/ \left(\sum_{i=1}^{m} C_i^A + \sum_{i=62-m}^{61} C_i^A\right) \qquad (7)$$

Here, when

$$\left(\sum_{i=1}^{m} C_i^A + \sum_{i=62-m}^{61} C_i^A\right)$$

is zero, the value of $Cd_A$ is taken as being 1.

An example of the above described "method of deciding upon the truth or the falsity of a CDS by utilizing the coding potential" is:

(m) A codon table is created in which the 64 types of codons are arranged so that the sequence of the 3 base of the i-th codon has the complementary sequence to the nucleotide sequence of the (i+32)-th codon, and it is stored in the memory;

(s) When the number of times the i-th codon appears in the t-th coding region is taken as $C_j^t$ then $y_i$ is obtained by the following equation (6):

$$y_i = \sum_{t=1}^{T} C_i^t \Big/ \sum_{t=1}^{T} \sum_{j=1}^{64} C_j^t \qquad (6)$$

(t) The codon table is called out from the memory, and, after having rearranged the 64 types of codon in the order of magnitude of $y_i$, "m leading codons for which the value of $y_i$ is large" and "m trailing codons for which the value of $y_i$ is large, excluding the translation stop codon" are chosen, and the value of $Cd_A$ for the coding region A is obtained from the following equation (7):

$$Cd_A = 2 \times \sum_{i=1}^{m} C_i^A \Big/ \left(\sum_{i=1}^{m} C_i^A + \sum_{i=62-m}^{61} C_i^A\right) \qquad (7)$$

Here, when

$$\left(\sum_{i=1}^{m} C_i^A + \sum_{i=62-m}^{61} C_i^A\right)$$

is zero, the value of $Cd_A$ is taken as being 1;

(u) When the value of $Cd_A$ for the coding region A which has been computed in the step (t) is greater than or equal to a threshold value CV, then said coding region is taken as being a true coding region, while when said value of $Cd_A$ is less than the threshold value CV then it is taken as a false coding region, and the results of this decision are outputted via the output device [here, T is an integer greater than or equal to 2, i is a positive integer less than or equal to 64, j is a positive integer less than or equal to 64, t is a positive integer less than or equal to T, m is an integer from 5 through 20, and CV is a value from 0.8 to 1.8].

An implementation is possible in which this processing is caused to be performed upon a computer.

If a CDS-A of the prokaryote overlaps with a CDS-B on the complementary strand, and moreover the CDS-B is included in the CDS-A, then, when determining another CDS of the same prokaryote by utilizing the above described method, it will also be acceptable to decide upon "the truth or the falsity" of the CDS by utilizing a method which includes the processes (q) and (r) described below:

(q) The length $L_A$ of the CDS-A (in base pairs) and the length of the CDS-B (in base pairs) are compared together, and, when $L_B$ is less than or equal to $T_P$% of $L_A$, it is decided that the CDS-B is a "false CDS"; and

(r) When $L_B$ is greater than $T_P$% of $L_A$, the truth or falsity of the CDS-A and the CDS-B are decided by the above described method utilizing "as a calculation expression which is capable of comparing the number of times m types of codons whose frequency of appearance is high appear in the CDS-A and the number of times m types of codons whose frequency of appearance is low appear in the CDS-A, the reciprocal of the result obtained by adding 1 to the ratio of the latter number of times to the former number of times", or "the above described Cd value" [here, Tp is a positive integer from 30 through 95].

When deciding upon the truth or the falsity of a transcription unit by utilizing said method, it is possible to apply said method after having linked the CDSs which make up several transcription units, excluding their stop codons, and having made them into a single CDS.

It is possible to execute the "method of determining a genetic structure aimed at transcription unit structure", the "method of determining a genetic structure using a shadow discrimination function", or the "method of determining a genetic structure aimed at the GC content of the bases in the codons" which have been disclosed for the present invention at higher speed by utilizing a computer. For this, it is necessary to create a program which commands the computer to perform each of the processes of the method of the present invention. This program can be made using a programming language such as C, C++, Perl, Fortran, BASIC, JAVA, or the like. And such a program can be executed upon an operating system such as UNIX, LINUX, Windows, MacOS, or the like.

Although, provided that it is endowed with a function of operating as a computer, it is possible to utilize any computer as the computer which is utilized for causing the above described program to be executed, it is desirable for this computer to be one whose speed of calculation is high. As concrete examples, it is possible to offer the personal computer PCG-XR9F/K made by Sony Corporation, the personal computer Let's Note CF-A77J81 of Matsushita Electronics Manufacturing Co. Ltd., and the SUN Ultra 80 workstation made by the company Sun Microsystems, or the like. There is no requirement to perform each method or each process which has been disclosed for the present invention by utilizing the same computer. In other words, it would also be acceptable to output the results which have been obtained by some process or method which has been described for the present invention to another computer, and to perform the processing for the next such process or for another method upon said computer.

By taking advantage of a recording medium which can be read in by a computer, and upon which can be recorded a program for causing a computer to execute the process of the "method of determining a genetic structure aimed at transcription unit structure", the "method of determining a genetic structure using a shadow discrimination function", or the "method of determining a genetic structure aimed at the GC content of the bases in the codons" disclosed by the present invention, it is possible to increase the level of automatic operation of these methods. Said recording medium is the recording medium of the present invention.

By "recording medium which can be read in by a computer" there is meant any recording medium which can be directly read in and accessed by a computer. Although, as this type of recording medium, it is possible to offer a floppy disk, a hard disk, a magnetic storage medium such as a magnetic tape or the like, an optical storage medium such as a CD-ROM, a CD-R, a CD-RW, a DVD-ROM, a DVD-RAM, a DVD-RW or the like, a magnetic storage medium such as RAM or ROM or the like, or a hybrid of these categories (for example, a magneto-optical storage medium such as MO or the like), it is not limited to being one of these.

A genetic structure determination system based upon a computer which takes advantage of the above described recording medium which can be read in by a computer according to the present invention is a system of the present invention.

By a "genetic structure determination system based upon a computer" is meant a system which is made up of a hardware means, a software means, and a data storage means, and which is used for analyzing the information which has been recorded upon the recording medium of the present invention which can be read in by a computer of the present invention.

In the following, examples of the present invention is disclosed.

Programs for commanding a computer to execute the processes which are shown in the examples described below were made using the programming language Perl version 5.005, and were executed upon the operating system Windows 2000. A "VAIO PCG-KR9F/K" manufactured by Sony Corporation was used as the computer.

Example 1: The determination of the genetic structure from the sequence of a DNA fragment including a threonine operon of Escherichia coli K-12 strain

According to the process shown in the flow charts of FIG. 7 and FIG. 8, the CDSs (sometimes comprising a true ORF) were determined from the sequences of the plus strand and the minus strand of a DNA fragment including the threonine operon of Escherichia coli K-12 strain. The details thereof are described below.

(1) Inputting of a nucleotide sequence to the computer

The sequence of a DNA fragment including a threonine operon of Escherichia coli K-12 strain (the accession number was AE000111 and the length of the sequence was 10,596 base pairs) was obtained via the internet from GenBank of the National Center for Biotechnology Information (hereinafter abbreviated as NCBI), which is the U.S. bio-information management organization, and was stored on the hard disk.

(2) Determination of the provisional start codon which yields the longest ORF

The stop codons TAA, TAG, and TGA which were present upon the plus strand of the DNA fragment represented by AE000111 were searched for. The provisional start codon (any one of ATG, GTG, and TTG) which yields the longest ORF for each found stop codon was determined, and the ORFs which encode polypeptides of 200 amino acids or more in length were selected. 4 ORFs were detected by said selecting process. Based upon the position of the stop codon, each ORF was numbered as ORF-D1 to ORF-D4 in the order from the 5' terminal.

In the same manner for the minus strand, stop codons were searched for and the provisional start codons were determined to select 3 ORFs. Those which encode polypeptides of 200 amino acids or more in length were selected, and, based upon the position of the stop codons, each ORF was numbered as ORF-C1 to ORF-C3 in the order from the 3' terminal of the minus strand.

(3) The method of calculating the ribosome binding score and the method of searching for the start codon

Since no ORFs existed upstream of ORF-D1, the start codon was determined based upon a ribosome binding score which was calculated by the following method.

From data analysis of the genome sequence of Escherichia coli K-12 strain in GenBank of the NCBI, it was confirmed that the 16S RNA 3' terminal sequence of Escherichia coli K-12 strain was 3'-AUUCCUCCA-5'. Next, according to the process shown in FIG. 5, the paired states between the sequence 3'-UUCCUCCA-5' in the above sequence and the sequence of 8 bases upstream of the start codon were investigated, the following values were assigned thereto:

A pairing of G and C: +4
A pairing of A and U: +2.
A pairing of G and U: +1.

No pairing is seen at a base adjacent to a base where a pairing is present: -1.

If the start codon is GTG: -2.
If the start codon is TTG: -4.

and the sum of these numerical values were termed the ribosome binding score (S). However, no value was assigned to the pairing in which the final base A of the 3'-UUCCUCCA-5' participated.

As the 8 bases upstream of the start codon for which the paired state was investigated, the 8 sequences of the region of the 17th base to the 10th base upstream to the region of the 10th base to the 3rd base upstream, were utilized. The ribosome binding scores were obtained for these 8 sequences (hereinafter termed U-1 to U-8 in order of the position from the upstream), and the one which yields the maximum value was selected.

If two or more sequences which yields the same maximum value existed, U-4, U-5, and U-6 had a priority, and the next U-7 and U-8 had a priority.

Using the method of the calculating the ribosome binding score, the ribosome binding scores (S) were calculated for up to 10 candidates for the start codon (ATG, GTG, or TTG) in the order from the 5' terminal of each ORF.

The ribosome binding scores (S) for the five candidates, the first to the fifth in the order from the 5' terminal of each ORF, for the start codon were compared with a threshold value $V_1$ ($V_1$ = 9.0), and if the ribosome binding score for the candidate exceeded the threshold value ($S>V_1$), then this candidate for the start codon was selected as a candidate for the true start codon. If no ribosome binding score did not exceed $V_1$ and a candidate for the start codon for which the score exceeds a threshold value $V_2$ ($V_2$=7.0) ($S>V_2$), then this candidate for the start codon was selected as a candidate for the true start codon.

If there were no candidate for the start codon for which the score exceeded either of the threshold values, the ribosome binding scores for five candidates, the sixth to the tenth from the 5' terminal, for the start codon were obtained, the ribosome binding score for each start codon was checked from the first start codon, and, if the score was greater than or equal to a threshold value $V_3$ ($V_3$=6.0), then the candidate for the start codon which yields this score was selected as the start codon.

If the ribosome binding scores of all the candidates for the start codon were less than the threshold value $V_3$ ($S<V_3$) and any candidate for the start codon which must be selected did not exist, then that ORF was decided as a "false CDS".

As the results, the candidate for the true start codon of ORF-D1 was the 337th base.

(4) search for ORFs which have a possibility to form a polycistronic transcription unit

Using the ORF based upon the candidate for the true start codon of ORF-D1 which was selected in the above described process (3), It was examined whether the ORFs which were present downstream of said ORF to form a polycistron.

Since the provisional start codon of ORF-D2 was present within 60 bases downstream of the stop codon of the ORF which was decided and selected as the candidate for a true ORF of ORF-D1 in the above described process (3), it was decided that ORF-D2 and ORF-D1 had a possibility to form a polycistronic transcription unit.

In the same manner, it was decided that ORF-D3 and ORF-D2 had a possibility to form a polycistronic transcription unit. However, ORF-D3 and ORF-D4 did not have a possibility to form a polycistronic transcription unit.

(5) Determination of the start codons of the ORFs which have a possibility to form a polycistronic transcription unit

Since it was decided that ORFs -D1 to -D3 had a possibility to form a polycistronic transcription unit in the above described process (4), their true start codons were determined according to a "priority ranking rule for the start codon shown in FIG. 4" described below (herein termed a "rank function", having a value which is an integer greater than or equal to 1).

The "vicinity" of the stop codon of the ORF-B is defined as the region for which the value of the rank function in FIG. 4 was from 1 to 8. If a candidate (ATG or GTG) for the start codon of the ORF-A was present in this region, the candidate was determined as the "start codon", whatever was the value of the ribosome binding score obtained by the above described method. The candidates for the start codon were limited to ATG or GTG only in the region of the "vicinity".

If no candidate for the start codon of the ORF-A was present in the vicinity of the stop codon of the ORF-B, it is examined whether a candidate (ATG, GTG, or TTG) for the start codon of the ORF-A was present in the region downstream of the vicinity of the stop codon of the ORF-B. Since, as a condition for the forming of a polycistron, the distance between the stop codon of the ORF-B and the start codon of the ORF-A should be within 60 bases, if a candidate for the start codon of the ORF-A was present in the region for which the value of the rank function was from 9 to 23, the ribosome binding score for each candidate for the start codon was calculated using the method described in the process (3) above in ascending order of the value of the rank function, and, if said score was greater than or equal to a threshold value $V_3$ (6.0), then this candidate was selected as the start codon.

If no candidate for the start codon of the ORF-A was present in the vicinity of the stop codon of the ORF-B, and furthermore in the region downstream of this vicinity, then it was examined whether a candidate (ATG, GTG, or TTG) for the start codon of the ORF-A was present in the region upstream of the vicinity of the stop codon of the ORF-B. If a candidate for the start codon of the ORF-A was present in the region downstream of this vicinity, for which the value of the rank function was from 24 to $R_{DN}$, then the ribosome binding score for each candidate for the start codon was calculated in ascending order of the value of the rank function, using the method described in the process (3) above, and if said score was greater than or equal to a threshold value $V_3$ (6.0), then this candidate was selected as the start codon. The value of $R_{DN}$ was defined as the sum of the integer value which was closest to and less than a value of 10% of the number of amino acids of the ORF-A and the numerical value 23; however, if this value exceeded 53, the value of $R_{DN}$ was defined as 53.

As the results, the position of the candidate for the true start codon of ORF-D2 was the base 2801, and the position of the candidate for the true start codon of the ORF-D3 was the base 3734.

(6) Determination of the start codon of an ORF which can not be determined in the above described processes

The true start codon for the ORF-D4, which was decided to have no possibility to form a polycistronic transcription unit, was determined in the manner described below.

The candidate for the true start codon was determined using the method of (3) described above, and it was decided whether the ORF-A is a true ORF. The position of the candidate for the true start codon which was determined are compared with the positions of the stop codons of all the ORFs which are present upstream of the ORF-A, and it was examined whether the ORF-A overlaps with upstream ORFs. If the ORF-A overlaps with an ORF-B, then it was examined whether the length of their overlapping region was greater than or equal to 90 base pairs or greater than or equal to 10% of the length of the ORF-B.

If the length of the overlapping region was greater than or equal to 90 base pairs, or greater than or equal to 10% of the length of the ORF-B, then this ORF-A was decided as a "false ORF". If the length of the overlapping region was less than or equal to 90 base pairs, or less than or equal to 10% of the length of the ORF-B, then the ORF-A was selected as a " candidate for a true ORF".

As the results, the candidate for the true start codon of ORF-D4 was the 8175-th base.

(7) Deleting an ORF which is included on the same strand

If the ORF-A was selected as a "candidate for a true ORF" in the above described process, then, it was examined whether all the "candidates for a true ORF" which were present upstream of the ORF-A were included between the start codon and the stop codon of the ORF-A, and the ORFs which were included were decided to be "false ORFs".

By the above described process, it was decided that all the four ORFs from ORF-D1 to ORF-D4 were "candidates for a true ORF".

(8) Delectation of the candidates for an ORF upon the minus strand

The "candidates for a true ORF" upon the minus strand were determined by exactly the same process as described above for selecting the "candidates for a true ORF" upon the plus strand. In the result, it was decided that all of the three ORFs from ORF-C1 to ORF-C3 were "candidates for a true ORF".

The candidate for the true start codon of ORF-C1 was the 4162-th base; the candidate for the true start codon of ORF-C2 was the 6459-th base; and the candidate for the true start codon of ORF-C3 was the 7959-th base. From these results, it was decided that ORF-C1 and ORF-C2 had a possibility to form a polycistronic transcription unit.

(9) Comparing the transcription units upon the plus strand with those on the minus strand

If the distance between the start codon of the true ORF (CDS) and the stop codon of a CDS upstream thereof was within 90 base pairs, it was decided that both of the CDSs were present upon the same transcription unit.

The structures of the transcription units upon the plus strand and those upon the minus strand were investigated in this manner, and it was found that the number of transcription units upon the plus strand was two (one of which was a polycistronic transcription unit), and that the number of transcription units upon the minus strand was two (one of which was a polycistronic transcription unit).

The position of the start codon of the first CDS of each of the transcription units and the position of the stop codon of its last CDS were obtained, and it was examined whether there was a region of overlapping between the transcription unit upon the plus strand and the transcription unit upon the minus strand.

Since ORF-C1 which was determined upon the minus strand had a overlapping region with the ORF-D3 which was determined upon the plus strand, the truth or falsity of the transcription units were decided by the method described below. The length of a transcription unit was the difference between the "position of the start codon of the first CDS" and the "position of the stop codon of the last CDS".

If a transcription unit P upon the plus strand included a transcription unit Q upon the minus strand, then the transcription unit Q upon the minus strand was decided as a "false transcription unit". Next, if a transcription unit P upon the plus strand was included in a transcription unit Q upon the minus strand, then the transcription unit P was decided as a "false transcription unit".

Furthermore, if the transcription unit P and the transcription unit Q overlapped, but the transcription unit P did not include the transcription unit Q, and the transcription unit Q did not include the transcription unit P, then the length of the transcription unit P was compared with that of the transcription unit Q, and the one whose length was shorter was decided as a "false transcription unit".

Finally, if the truth or the falsity of the transcription unit P and the transcription unit Q was not able to be decided, then both of the transcription units were defined as true.

Using the above method of deciding on the truth or falsity of the transcription units, the monocistronic transcription unit ORF-C1 (the region from base 4162 to base 3512) upon the minus strand was decided to be a "false transcription unit" (refer to Table 1).

(10) Outputting the information about the determined CDSs

The information about the CDSs which was determined by the process described above was outputted as a file upon the hard disk. The outputted information was shown in Table 1.

TABLE 1

| ORF number | plus strand/ minus strand | position of start codon | position of stop codon | information about a transcription unit structure | truth or falsity of CDS |
|---|---|---|---|---|---|
| ORF-D1 | + | 337 | 2799 | 2 | true |
| ORF-D2 | + | 2801 | 3733 | 3 | true |
| ORF-D3 | + | 3734 | 5020 | 4 | true |
| ORF-D4 | + | 8175 | 9191 | 1 | true |
| ORF-C1 | - | 4162 | 3512 | | false |
| ORF-C2 | - | 6459 | 5683 | 4 | true |
| ORF-C3 | - | 7959 | 6529 | 2 | true |

The information in Table 1 was compared with the annotation information which is appended to the sequence registered in GenBank of the NCBI under accession number AE000111, and it was understood that the number of CDSs of 200 amino acids or more was 6 in both, and that all the 6 CDSs which were determined were identical between both. The positions of the start codons of 5 of the CDSs, with the exception of ORF-D4, were identical with the annotation information registered in GenBank. Furthermore it was shown that information about a transcription unit structure, which was not present in the annotation information registered in GenBank, was also obtained in the present invention.

In the table, for information about the structure of a transcription unit, the label "1" was appended to a monocistronic CDS, and the labels "2", "4", and "3" were respectively appended to the first CDS, to the last CDS, and to a CDS which was present internally between the first CDS and the last CDS.

Example 2: Determination of the CDSs from the sequence of a DNA fragment which includes a tryptophan operon of Escherichia coli K-12 strain

The CDSs were determined from the sequence of a DNA fragment which includes a tryptophan operon of Escherichia coli K-12 strain according to the method described in Example 1.

(1) Inputting of a nucleotide sequence to the computer

The entire genome sequence of Escherichia coli K-12 strain was obtained via the internet from GenBank of the NCBI (the accession number was U00096 and the length of the sequence was 4,639,221 base pairs), and was stored upon the hard disk. Furthermore, the sequence from base 1,314,001 to base 1,321,021, which included a tryptophan operon, was extracted from this sequence.

(2) Determination of the CDSs and the start codons

From the plus strand and the minus strand of the sequence above described in (1), the CDSs which encode polypeptides of 200 amino acids or more in length were determined according to the method described in (2) of Example 1,

Although ORFs were not be determined from the plus strand, a total of 5 ORFs were determined from the minus strand. Each ORF was numbered as ORF-C11 to ORF-C15 in the order from the 3' terminal of the minus strand, based upon the position of its stop codon.

Among the candidates for ORF upon the minus strand, ORFs which had the possibility to form a polycistronic transcription unit were searched for according to the method described in (4) of Example 1, and the truth or falsity of the 5 ORFs (ORF-C11 to ORF-C15) were decided and their start codons were determined according to the method for calculating the ribosome binding score for each ORF and for determining the true start codon which was described in (3) to (6) of Example 1. Furthermore, CDSs which were included in other CDS upon the same strand were deleted.

As the results, 5 CDSs and 1 transcription unit were determined from the minus strand.

(3) Outputting the results about the determined CDSs and the evaluation of these results

The results about the CDSs which were finally determined were outputted as a text file on the hard disk. These results are shown in Table 2.

Table 2

| ORF number | plus strand/ minus strand | position of start codon | position of stop codon | information about a transcription unit structure | truth or falsity of CDS |
|---|---|---|---|---|---|
| ORF-C11 | - | 1246 | 440 | 4 | true |
| ORF-C12 | - | 2439 | 1246 | 3 | true |
| ORF-C13 | - | 3812 | 2451 | 3 | true |
| ORF-C14 | - | 5408 | 3813 | 3 | true |
| ORF-C15 | - | 6970 | 5408 | 2 | true |

The information in Table 2 was compared with the annotation information which is appended to the genome sequence (accession number U00096) of the Escherichia coli K-12 strain which is registered in GenBank of the NCBI, and it was understood that the number of CDSs of 200 amino acids or more of the tryptophan operon region was 5 in both, and that all the 5 CDSs which were determined were identical completely with the CDSs which are registered in GenBank. The positions of the start codons of these 5 CDSs were identical with the annotation information registered in GenBank. Furthermore it was shown that information about the structure of a transcription unit, which was not present in the annotation information registered in GenBank, was also obtained in relation to transcription units in the present invention.

Example 3: Determination of the CDSs from the sequence of a DNA fragment which includes a ribosomal protein operon of Escherichia coli K-12 strain

The CDSs which encode polypeptides of 200 amino acids or more in length were determined from the sequence of a DNA fragment which includes a ribosomal protein operon of Escherichia coli K-12 strain according to the method described in Example 1, .

(1) Inputting of a nucleotide sequence to the computer

The sequence of a DNA fragment including a ribosomal protein operon of Escherichia coli K-12 strain was obtained via the internet from GenBank of the NCBI (the accession number was AE000408 and the length of the sequence was 14,659 base pairs), and was stored upon the hard disk.

(2) Determination of the CDSs and the start codons

The stop codons and the provisional start codons were determined from the sequence above described in (1) according to the method described in (2) of Example 1.

A total of 6 ORFs were determined from the plus strand, and a total of 5 ORFs were determined from the minus strand. Each ORF was numbered as ORF-D21 to ORF-C26 in the order from the 5' terminal of the plus strand, and numbered as ORF-C21 to ORF-C25 in the order from the 3' terminal of the minus strand.

Among the candidates for ORF upon the plus strand and upon the minus strand, ORFs which had a possibility to form a polycistronic transcription unit were searched for according to the method described in (4) of Example 1, and the truth or falsity of the 11 ORFs were decided and their start codons were determined according to the methods for calculating the ribosome binding score for each ORF and for determining the start codon which were described in (3) to (6) of Example 1. Furthermore CDSs which were included in other CDS upon the same strand were deleted according to the method described in (7) and (8) of Example 1.

As the results, 6 CDSs and 5 transcription units were determined from the plus strand, and 5 CDSs and 4 transcription units were determined from the minus strand.

Furthermore, overlapping between selected transcription units upon the plus strand and those upon the minus strand was investigated, and the truth or falsity of the candidate for a true ORF was decided according to the method described in (9) of Example 1,.

AS the result, the 7 CDSs ORF-D21, ORF-D22, ORF-D24, ORF-D25, ORF-D26, ORF-C21, and ORF-C22 were decided to be true, and the 4 CDSs ORF-D23, ORF-C23, ORF-C24, and ORF-C25 were decided to be false.

(3) Outputting the results about the determined CDSs and the evaluation of these results

The results about the 7 true CDSs and the 4 false CDSs which were finally determined were outputted as a text file on the hard disk. These results are shown in Table 3.

Table 3

| ORF number | plus strand / minus strand | position of start codon | position of stop codon | information about a transcription unit structure | truth or falsity of CDS |
|---|---|---|---|---|---|
| ORF-D21 | + | 2148 | 2774 | 2 | true |
| ORF-D22 | + | 2825 | 3446 | 4 | true |
| ORF-D23 | + | 6763 | 7418 | | false |
| ORF-D24 | + | 7910 | 8961 | 1 | true |
| ORF-D25 | + | 9172 | 9809 | | true |
| ORF-D26 | + | 9743 | 10446 | | true |
| ORF-C21 | - | 1624 | 293 | 1 | true |
| ORF-C22 | - | 7410 | 6709 | 1 | true |
| ORF-C23 | - | 8891 | 8070 | | false |
| ORF-C24 | - | 9813 | 9208 | 4 | false |
| ORF-C25 | - | 10453 | 9824 | 2 | false |

The information in Table 3 was compared with the annotation information which is appended to the sequence of accession number AE000408 registered in GenBank of the NCBI, and it was understood that the number of CDSs of 200 amino acids or more in ribosomal protein operon regions which was determined by the method of the present invention were 7, and that the number of the CDSs which were registered in GenBank of the NCBI was 5. Among the determined 7 CDSs, CDSs identical with the annotation information of GenBank were the 2 CDSs ORF-C21 and ORF-C22. The positions of the start codons of these 2 CDSs were identical with the annotation information registered in GenBank. Furthermore it was shown that information about a transcription unit structure, which is not present in the annotation information registered in GenBank, was also obtained in the present invention.

Example 4: Determination of the CDSs which encode polypeptides of 34 amino acids or more from the sequence of a DNA fragment which includes a ribosomal protein operon of Escherichia coli K-12 strain (1)

The CDSs which encode polypeptides of 34 amino acids or more in length were determined from the sequence of a DNA fragment which includes a ribosomal protein operon of Escherichia coli K-12 strain According to the method described in Example 1.

(1) Inputting of a nucleotide sequence to the computer

The sequence of a DNA fragment including a ribosomal protein operon of Escherichia coli K-12 strain was obtained via the internet from GenBank of the NCBI (the accession number was AE000408 and the length of the sequence was 14,659 base pairs), and was stored upon the hard disk.

(2) Determination of the CDSs of 34 amino acids or more and the start codons

From the above described sequence (1), the stop codons and the provisional start codons of the ORFs which encode polypeptides of 34 amino acids or more were determined according to the method described in (2) of Example 1, .

A total of 53 ORFs were determined from the plus strand, and a total of 38 ORFs were determined from the minus strand. Each ORF were numbered as ORF-D101 to ORF-D153 in the order from the 5' terminal of the plus strand, and numbered as ORF-C101 to ORF-C138 in the order from the 3' terminal of the minus strand, based upon the position of its stop codon.

Among the candidates for ORF upon the plus strand and upon the minus strand, ORFs which had a possibility to form a polycistronic transcription unit were searched for according to the method described in (4) of Example 1, and the truth or falsity of the 91 ORFs were decided and their start codons were determined by using the method of calculating the ribosome binding score for each ORF and the method of searching for the start codon which were described in (3) to (6) of Example 1. Furthermore, CDSs which were included in other CDS upon the same strand were deleted according to the method described in (7) and (8) of Example 1. As the results, 22 CDSs and 13 transcription units were determined from the plus strand, and 23 CDSs and 2 transcription units were determined from the minus strand. Furthermore, overlapping between selected transcription units upon the plus strand and those upon the minus strand was investigated, and the truth or falsity of the candidates for a true ORF was decided according to the method described in (9) of Example 1.

As the results, the 23 CDSs on the minus strand were decided to be true, and that the 22 CDSs on the plus strand were decided to be false.

(3) Outputting the results about the determined CDSs and the evaluation of these results

The results about the true ORFs (CDSs) which was finally determined were outputted as a text file on the hard disk. These results are shown in Table 4.

Table 4

| ORF number | plus strand / minus strand | position of start codon | position of stop codon | information about a transcription unit structure | truth or falsity of CDS |
|---|---|---|---|---|---|
| ORF-C101 | - | 261 | 145 | 4 | true |
| ORF-C102 | - | 1624 | 293 | 3 | true |
| ORF-C105 | - | 2066 | 1632 | 3 | true |
| ORF-C107 | - | 2249 | 2070 | 3 | true |
| ORF-C109 | - | 2756 | 2253 | 3 | true |
| ORF-C111 | - | 3124 | 2771 | 3 | true |
| ORF-C113 | - | 3667 | 3134 | 3 | true |
| ORF-C114 | - | 4072 | 3680 | 3 | true |
| ORF-C116 | - | 4411 | 4106 | 3 | true |
| ORF-C118 | - | 4965 | 4426 | 3 | true |
| ORF-C119 | - | 5294 | 4980 | 2 | true |
| ORF-C120 | - | 5676 | 5305 | 4 | true |

Table 4 (continued)

| ORF number | plus strand / minus strand | position of start codon | position of stop codon | information about a transcription unit structure | truth or falsity of CDS |
|---|---|---|---|---|---|
| ORF-C121 | - | 6095 | 5841 | 3 | true |
| ORF-C122 | - | 6286 | 6095 | 3 | true |
| ORF-C123 | - | 6696 | 6286 | 3 | true |
| ORF-C125 | - | 7410 | 6709 | 3 | true |
| ORF-C127 | - | 7760 | 7428 | 3 | true |
| ORF-C129 | - | 8053 | 7775 | 3 | true |
| ORF-C130 | - | 8891 | 8070 | 3 | true |
| ORF-C133 | - | 9211 | 8909 | 3 | true |
| ORF-C135 | - | 9813 | 9208 | 3 | true |
| ORF-C137 | - | 10453 | 9824 | 3 | true |
| ORF-C138 | - | 10797 | 10486 | 2 | true |

The information in Table 4 was compared with the annotation information which is appended to the sequence of accession number AE000408 registered in GenBank of the NCBI, and it was understood that the number of CDSs of 34 amino acids or more in ribosomal protein operon regions was 23 in both, and that all the 23 determined CDSs were identical between both. The positions of the start codons of these 23 CDSs were identical with the annotation information registered in GenBank. Furthermore it was shown that information about a transcription unit structure, which is not present in the annotation information registered in GenBank, was also obtained in the present invention.

Example 5: Determination of the CDSs which encode a polypeptide of 34 amino acids or more from the sequence of a DNA fragment which includes a threonine operon of Escherichia coli K-12 strain (1)

In order to further enhance the accuracy of determination of CDSs which encodes polypeptides of 34 amino acids or more by the method described in Example 4, the process of analysis based upon the determined CDS information which encodes polypeptides of 200 amino acids was added, then the CDS which encodes polypeptides of 34 amino acids or more were determined at high accuracy as described below.

(1) Searching for the ORFs which encode polypeptides of 34 amino acids or more

According to the method of Example 4, from the sequence of a DNA fragment including a ribosomal protein operon of Escherichia coli K-12 strain (the accession number was AE000111 and the length of the sequence was 10,596 base pairs), the ORFs which encode polypeptides of 34 or more amino acids upon the plus strand were searched for.

As the results, 47 ORFs were obtained from the plus strand, and 51 ORFs were obtained from the minus strand. The positions of the start codons of these ORFs were determined and the candidates for a true ORF were selected according to the method described in Example 4.

However, the ORF whose length is less than 180 base pairs, and moreover the start codon is TTG, was not selected as the CDS.

(2) Deciding truth or falsity of the CDSs with a shadow discrimination function

For each of the true ORF candidates which was selected by the above described process (1), the truth or the falsity of each ORF was decided from the value of a shadow discrimination function as described below.

The frequency of appearance of codons of the 6 CDSs which was determined in Example 1 and encodes polypeptides of 200 amino acids or more and were present in the sequence of the DNA fragment including a threonine operon of Escherichia coli K-12 strain were obtained.

Based on the frequency of appearance of codons of the 6 CDSs which was determined by the process (1) and encodes polypeptides of 200 amino acids or more, 13 combinations of codons were selected, wherein "the frequency of times of a codon appearing in CDSs which were decided to be "true CDSs" was high, and the frequency of times of a codon which has the complementary sequence to the 3-base sequence of said codon appearing in said CDSs was low", and the values of the shadow discrimination function were obtained according to the method described below,.

That is, when the 64 types of codons:

TTA, CTA, TCA, TTT, TTC, TTG, TCT, TCC, TCG, TAT, TAC, TGT, TGC, TGG, CTT, CTC, CTG, CCT, CCC, CCG, CAT, CAC, CGT, CGC, ATT, ATC, ACT, ACC, AAC, AGC, GTC, GCC, TAA, TAG, TGA, AAA, GAA, CAA, AGA, GGA, CGA, ATA, GTA, ACA, GCA, CCA, AAG, GAG, CAG, AGG, GGG, CGG, ATG, GTG, ACG, GCG, AAT, GAT, AGT, GGT, GTT, GCT, GAC, GGC

was arranged in the above order, a number was appended to each codon so that the first codon was TTA, and the second codon was CTA. According to the following formula, the values of $y_i$ and $y_{i+32}$ (i is a positive integer less than or equal to 32) were calculated, the above described 64 types of codon were rearranged in descending order of these values, and a number was appended to each codon in order.

$$y_i = \left(\sum_{t=1}^{6} C_i^t - \sum_{t=1}^{6} C_{i+32}^t\right) \Big/ \sum_{t=1}^{6}\sum_{j=1}^{64} C_j^t \qquad (2-1)$$

(herein i is a positive integer less than or equal to 32) or

$$y_{i+32} = \left(\sum_{t=1}^{6} C_{i+32}^t - \sum_{t=1}^{6} C_i^t\right) \Big/ \sum_{t=1}^{6}\sum_{j=1}^{64} C_j^t \qquad (3-1)$$

(herein i is a positive integer less than or equal to 32)

wherein the number of times of the i-th codon appearing in the t-th CDS is expressed as: $C_j^t$

Next, the top 13 codons for which the value of $y_i$ or $y_{i+32}$ is large and the bottom 13 codons for which the value of $y_i$ or $y_{i+32}$ is small were selected, and the value of the shadow discrimination function $Sd_A$ for the ORF-A which was the candidate for true ORF was obtained by the following formula:

$$Sd_A = 2 \times \sum_{i=1}^{13} C_i^A \Big/ \left(\sum_{i=1}^{13} C_i^A + \sum_{i=52}^{64} C_i^A\right) \qquad (4-1)$$

herein the value of $Sd_A$ is defined as 1 if

$$\left(\sum_{i=1}^{13} C_i^A + \sum_{i=52}^{64} C_i^A\right)$$

was zero.

Based on the $Sd_A$ which was obtained in this manner, the ORF-A was decided to be a true CDS when the following condition was satisfied. That is to say, the ORF is decided to be a true ORF (CDS): if "$Sd_A$ is greater than or equal to 0.9", in the case of an ORF which encodes a polypeptide of 100 amino acids or more; if "$Sd_A$ is greater than or equal to 1.0", in the case of an ORF which encodes a polypeptide of 60 amino acids or more and 99 amino acids or less; and if "$Sd_A$ is greater than or equal to 1.0", in the case of an ORF which encodes a polypeptide of 34 amino acids or more and 59 amino acids or less.

(3) Selection of the CDSs when two ORFs overlap upon the same strand

When two ORFs which were selected by the above described process overlapped upon the same strand, the CDSs were selected out in the following manner.

When the 5' terminal side of an ORF-A which is the candidate for a true ORF and the 3' terminal side of an ORF-B which is present upstream of said ORF-A overlapped, the truth or falsity of these ORFs was decided by the following method.

If the length of the overlapping region of the ORF-A and the ORF-B was less than or equal to 90 base pairs, was less than or equal to 10% of $L_A$, the length of the ORF-A, and was less than or equal to $L_B$, the length of the ORF-B, then both of the ORFs were selected as true CDSs.

If the length of the overlapping region did not satisfy the above described condition, the truth or falsity of the ORFs was decided by the following method.

If $L_A$ was greater than $L_B$, and the following formula (9) was satisfied, then the ORF-A was selected as a CDS:

$$L_B < L_A \times (L_A + 12000)/20000 \qquad (9)$$

If $L_A$ was smaller than $L_B$, and moreover the following formula (10) is satisfied, then the ORF-B was selected as a CDS:

$$L_A < L_B \times (L_B + 12000)/20000 \qquad (10)$$

When the relationship between the length of the ORF-A and the length of the ORF-B did not satisfy the formula (9) or the formula (10), then the truth or falsity of the CDS is decided according to the magnitude of the values of the shadow discrimination function of the ORF-A and the ORF-B.

If an ORF was selected as a "candidate for a true ORF" by the above described process, then, it was examined whether all the "candidates for a true ORF" which are upstream of said ORF were included between the start codon and the stop codon of said ORF, and the ORFs which were included were all decided as "false ORFs".

As the results of decision by the above method, 15 among the 47 ORFs which were present upon the plus strand, and 16 among the 51 ORFs which were present upon the minus strand, were selected as CDSs.

(4) Comparison of transcription units upon the plus strand and the minus strand

When, in the process described above, the distance between a start codon of a CDS which was selected as a true ORF and the stop codon of a CDS which was present upstream thereof was within 90 base pairs, then it was decided that both the CDSs were present in the same transcription unit to investigate the structure of the transcription unit.

As the result, it was understood that the number of transcription units upon the plus strand was 6, and the number of transcription units upon the minus strand was 9.

Next, the position of the start codon of the first CDS and the position of the stop codon of the last CDS of each of the transcription units were obtained, and it was examined whether there was an overlapping region between the transcription unit of the plus strand and the transcription unit of the minus strand. If a transcription unit P of the plus strand included a transcription unit Q of the minus strand, or if a transcription unit P of the plus strand was included in a transcription unit Q of the minus strand, the following process is performed for deciding on the truth or falsity of the transcription units. The length of a transcription unit was the difference between the "position of the start codon of the first CDS" and the "position of the stop codon of the last CDS", and the lengths of the transcription unit P and of the transcription unit Q are termed $L_P$ and $L_Q$ respectively.

If $L_P$ was greater than $L_Q$, and the following formula (11) was satisfied, then the transcription unit P was decided as a true transcription unit, and the transcription unit Q was decided as a false transcription unit.

$$L_Q < L_P \times (L_P + 14000)/20000 \qquad (11)$$

When $L_P$ was smaller than $L_Q$, and the following formula (12) was satisfied, then the transcription unit P was decided as a false transcription unit, and the transcription unit Q was decided as a true transcription unit.

$$L_P < L_Q \times (L_Q + 14000)/20000 \qquad (12)$$

When the relationship between the lengths of the transcription unit P and the transcription unit Q did not satisfy the formula (11) or the formula (12), then, all the CDSs which form the transcription unit P and the transcription unit Q were linked up respectively, the values of the shadow discrimination function for these linked coding regions were calculated according to the method described in (2) of Example 5, and the one for which this value is the greater is decided as a true transcription unit.

(5) The comparison of the CDSs upon the plus strand and the minus strand, and the processing when the CDSs upon the plus strand and the minus strand are overlapped at the 5' terminal side

For the CDSs which form transcription units and were selected in the above described manner, the truth or the falsity of two CDSs which are in an inclusion relationship is decided by the method described in (4) above.

After the decision, for the CDSs which were selected as true CDSs, it is examined whether a CDS upon the plus strand (termed CDS-A) and a CDS upon the minus strand (termed CDS-B) were overlapped at the 5' terminal side.

The truth or the falsity of two CDSs (CDS-A and CDS-B) for which overlapping was prudent is decided according to the process described below.

Determination of a new start codon was carried out in the downstream from the next codon to the start codon of the CDS-A, utilizing the method described in (3) of Example 1. In the same manner, Determination of a new start codon was carried out in the downstream from the next codon to the start codon of the CDS-B, utilizing the method described in (3) of Example 1. If a new start codon was determined, it was examined whether a combination of start codons, also including the previous start codon, existed wherein the CDS-A and the CDS-B do not overlap. The positions of the original start codon and the newly determined start codon were compared together, and, when it was possible to avoid overlapping of the CDS-A and the CDS-B, both of the CDS-A and the CDS-B were decided as true CDSs. When it was not possible to avoid overlapping of the CDS-A and the CDS-B, then the truth or falsity of the CDSs was decided by the following method.

If $L_A$ was greater than $L_B$, wherein lengths of the CDS-A and the CDS-B were termed $L_A$ and $L_B$ respectively, and if the following formula (9) was satisfied, then the CDS-B was decided as a "false CDS".

$$L_B < L_A \times (L_A + 12000)/20000 \qquad (9)$$

If $L_A$ was smaller than $L_B$, and the following formula (10) was satisfied, then the CDS-A was decided as a "false CDS".

$$L_A < L_B \times (L_B + 12000)/20000 \qquad (10)$$

If it was not possible to decide on the truth or the falsity of the CDS-A and the CDS-B even by either of the above described methods, then the truth or the falsity of the CDSs was decided on by comparing the values of the shadow discrimination function for each of the CDSs according to a method described above.

(6) The processing when the CDSs of the plus strand and the minus strand overlap at the 3' terminal side

If the CDS-A of the plus strand and the CDS-B of the minus strand overlapped at their respective 3' terminal sides, the CDS-A did not include the CDS-B, and the CDS-B did not include the CDS-A, then the truth or the falsity of the CDS-A and of the CDS-B were decided by using a method above described in process (5).

First, if the length of the overlapping region of the CDS-A and the CDS-B was less than 20% of each of the CDSs, then both of the CDSs were decided to be true CDSs. If the length of the overlapping region was greater than or equal to 20% of one of the CDSs, and less than 20% of the length of the other one of the CDSs, then it was decided that the former CDS was a false CDS. If it was not possible to decide on the truth or the falsity of the CDSs by these conditions, then the truth or the falsity of the CDSs was decided according to the method using the formula (9) and the formula (10) described above.

By the above described processing, 7 true ORFs (CDSs) were determined from the plus strand, and 5 CDSs were determined from the minus strand. Each ORF was numbered as ORF-D201 to ORF-D207 in order from the 5' terminal of the plus strand, and numbered as ORF-C201 to ORF-C205 in order from the 3' terminal of the minus strand, by the position of its stop codon.

(7) Outputting of the results about the determined CDSs and the evaluation of these results

By adding the above described processing, 7 ORFs were determined from the plus strand and 5 ORFs were determined from the minus strand. Each ORF was numbered as ORF-D201 to ORF-D207 in order from the 5' terminal of the plus strand, and numbered as ORF-C201 to ORF-C205 in order from the 3' terminal of the minus strand, by the position of its stop codon. The results about the 12 CDSs which was finally determined were outputted as a text file upon the hard disk. These results are shown in Table 5.

Table 5

| ORF number | plus strand / minus strand | position of start codon | position of stop codon | information about a transcription unit structure | truth or falsity of CDS |
|---|---|---|---|---|---|
| ORF-D201 | + | 337 | 2799 | 2 | true |
| ORF-D202 | + | 2801 | 3733 | 3 | true |
| ORF-D203 | + | 3734 | 5020 | 3 | true |
| ORF-D204 | + | 5088 | 5235 | 4 | true |
| ORF-D205 | + | 7986 | 8141 | 2 | true |
| ORF-D206 | + | 8175 | 9191 | 4 | true |
| ORF-D207 | + | 9306 | 9893 | 1 | true |
| ORF-C201 | - | 5657 | 5310 | 4 | true |
| ORF-C202 | - | 6459 | 5683 | 3 | true |
| ORF-C203 | - | 7959 | 6529 | 2 | true |
| ORF-C204 | - | 10452 | 9928 | 4 | true |
| ORF-C205 | - | 10571 | 10452 | 2 | true |

The information in Table 5 was compared with the annotation information which is appended to the sequence of accession number AE000111 registered in GenBank of the NCBI, and it was understood that the number of CDSs of 34 amino acids or more was determined by the method of the present invention was 12, while the number of the CDSs which were registered in GenBank of the NCBI was 9. The CDSs, among the determined 12 CDSs, CDSs identical with the annotation information of GenBank were the 8 CDSs ORF-D201, ORF-D202, ORF-D203, ORF-D206, ORF-D207, ORF-C202, ORF-C203, and ORF-C205. The positions of the start codons of 6 of these 8 CDSs were identical with the annotation information registered in GenBank. Furthermore it was shown that information about a transcription unit structure, which is not present in the annotation information registered in GenBank, was also obtained in the present invention,.

From the results of this example, it was shown that the accuracy of CDS determination was enhanced by combining the "method of determining a genetic structure using a shadow discrimination function" of the present invention with the "method of determining a genetic structure from the viewpoint of a transcription unit structure" of the present invention.

Example 6: Determination of the CDSs which encode a polypeptide of 34 amino acids or more from the sequence of a DNA fragment which includes a ribosomal protein operon of Escherichia coli K-12 strain (2)

According to the method described in Example 5, the CDSs which encode polypeptides of 200 amino acids or more were determined at high accuracy from the sequence of a DNA fragment including a ribosomal protein operon of Escherichia coli K-12 strain (the accession number was AE000408, and the length of the sequence was 14,659 base pairs), and the CDSs which encode polypeptides of 34 amino acids or more were determined based upon the determined CDS information by carrying out the process of the "method of determining a genetic structure using a shadow discrimination function".

As the results, the same results as given in Example 4 were obtained.

Example 7: Determination of the CDSs which encode a polypeptide of 34 amino acids or more from the sequence of a DNA fragment which includes a threonine operon of Escherichia coli K-12 strain (2)

In this Example, it was shown that the accuracy of CDS determination is further enhanced by combining the "method of deciding upon the truth or falsity of CDSs by using a coding potential" with the method described in Example 5 which was combined the "method of determining a genetic structure from the viewpoint of a transcription unit structure" and the "method of determining a genetic structure using a shadow discrimination function". As an indicator of coding potential, as shown below, there is utilized a value which was obtained by calculation formula (hereinafter referred to as the "code function" or Cd) which is based upon the number of appearances of codons which appear often in true CDSs, and of codons which appear rarely therein.

(1) Deciding upon the truth or the falsity of the ORFs according to a method of calculating the value of a "code function"

the value of $y_i$ was obtained from the formula (6) below for the number T of coding regions which was determined:

$$y_i = \sum_{t=1}^{T} C_i^t \Big/ \sum_{t=1}^{T} \sum_{j=1}^{64} C_j^t \qquad (6)$$

wherein the number of times of the i-th codon appearing in the t-th coding region was expressed as $C_j^t$

next, the 64 types of codons were rearranged in descending order of yi, "top m codons for which the value of yi is large" and "bottom m codons for which the value of yi is large, excluding the translation stop codon" were selected, and the value of $Cd_A$ of a specified ORF (termed ORF-A) was obtained from the formula (7) below:

$$Cd_A = 2 \times \sum_{i=1}^{m} C_i^A \Big/ \left(\sum_{i=1}^{m} C_i^A + \sum_{i=62-m}^{61} C_i^A\right) \qquad (7)$$

wherein, the value of $Cd_n$ was defined as 1 if

$$\left(\sum_{i=1}^{m} C_i^A + \sum_{i=62-m}^{61} C_i^A\right)$$

is zero,

[herein T is an integer greater than or equal to 2, i is a positive integer less than or equal to 64, t is a positive integer less than or equal to T, and m is an integer from 5 to 20].

In this Example, m was 15.

Based on the $Cd_A$ which was obtained in this manner, the ORF-A was decided to be a true CDS when the following condition was satisfied.

That is, said ORF was decided as a true CDS: if "$Cd_A$ was greater than or equal to 1.0", in the case of an ORF of 100 amino acids or more; if "$Cd_A$ was greater than or equal to 1.1", in the case of an ORF of 60 amino acids or more and of 99 amino acids or less; and if "$Cd_A$ is greater than or equal to 1.4", in the case of an ORF of 34 amino acids or more and of 59 amino acids or less.

When the value of the "shadow discrimination function" was obtained, the truth or the falsity of the CDSs was decided based upon a value ($Sd_A$ X $Cd_A$) which is the product of the value $Sd_A$ of the "shadow discrimination function" and the value $Cd_A$ of the "code function" of the ORF-A.

In other words, in the case of an ORF of 100 amino acids or more, it was decided that said ORF included a true CDS if "$Sd_A$ X $Cd_A$ is greater than or equal to 1.1". In the case of an ORF of 60 amino acids or more and of 99 amino acids or less, it was decided that said ORF included a true CDS if "$Sd_A$ X $Cd_A$ is greater than or equal to 1.4". In the case of an ORF of 34 amino acids or more and of 59 amino acids or less, it was decided that said ORF included a true CDS if "$Sd_A$ X $Cd_A$ is greater than or equal to 1.6".

(2) Determination of the CDSs which encode polypeptides of 34 amino acids or more from the sequence of a DNA fragment which includes a threonine operon

According to the method described in Example 5, except for the process which utilizes the value of the "shadow discrimination function", determination of the CDSs which encode polypeptides of 34 amino acids or more from the plus strand and the minus strand of the sequence of a DNA fragment which included a threonine operon of Escherichia coli K-12 strain (the accession number was AE000111, and the length of the sequence was 10,596 base pairs) was carried out.

In this Example, in the process of Example 5 using the "shadow discrimination function", the truth or falsity of the CDSs was decided by a decision method which was based upon the value of the "code function" and a decision method which was based upon the value which was the product of the "shadow discrimination function" and the "code function" which were disclosed in (1) above, in addition to the decision method based upon the value of the "shadow discrimination function".

The CDSs were selected according to the process described above. As the results, 6 CDSs (ORF-D301 to ORF-D306) were determined from the plus strand, and 3 CDSs (ORF-C301 to ORF-C303) were determined from the minus strand.

(3) Outputting the results about determined CDS and the evaluation of these results

The results about the 9 CDSs which were finally determined were outputted as a text file upon the hard disk. These results were shown in Table 6.

Table 6

| ORF number | plus strand / minus | position of start codon | position of stop codon | information about a transcription unit structure | truth or falsity of CDS |
|---|---|---|---|---|---|
| ORF-D301 | + | 337 | 2799 | 2 | true |
| ORF-D302 | + | 2801 | 3733 | 3 | true |
| ORF-D303 | + | 3734 | 5020 | 4 | true |
| ORF-D304 | + | 5234 | 5528 | 4 | true |
| ORF-D305 | + | 8238 | 9191 | 1 | true |
| ORF-D306 | + | 9306 | 9893 | 1 | true |
| ORF-C301 | - | 6459 | 5683 | 1 | true |

Table 6 (continued)

| ORF number | plus strand / minus | position of start codon | position of stop codon | information about a transcription unit structure | truth or falsity of CDS |
|---|---|---|---|---|---|
| ORF-C302 | - | 7959 | 6529 | 4 | true |
| ORF-C303 | - | 10494 | 9928 | 2 | true |

The information in Table 6 was compared with the annotation information which is appended to the sequence of accession number AE000111 registered in GenBank of the NCBI, and it was understood that the number of CDSs of 34 amino acids or more which was determined by the method of the present invention was 9 in both, and that these 9 CDSs were all identical between both. The positions of the start codons of these 9 CDSs were identical with the annotation information registered in GenBank. Furthermore it was shown that, information about a transcription unit structure, which is not present in the annotation information registered in GenBank, also obtained in the present invention,.

From comparison of the results of Example 5 with the results of this Example, it was apparent that the accuracy of CDS determination was further enhanced by combining a "method of deciding on the truth or the falsity of a CDS by utilizing the coding potential" with the method which is the combination of the "method of determining a genetic structure from the viewpoint of a transcription unit structure" and "method of determining a genetic structure using a shadow discrimination function" of the present invention.

Example 8: Determination of the CDSs which encode a polypeptide of 34 amino acids or more from the entire genome sequence of Escherichia coli K-12 strain (1)

According to the method of Examples 1 and 5, in other words, utilizing the "method of determining a genetic structure from the viewpoint of a transcription unit structure", the CDSs which encode polypeptides of 34 or more amino acids were determined from the entire genome sequence of Escherichia coli K-12 strain MG1655.

(1) Determination of the CDSs which encode a polypeptide of 34 amino acids or more from the entire genome sequence of Escherichia coli

According to the method described in Example 1, 2679 and 2661 CDSs which encode polypeptides of 34 amino acids or more were determined from the plus strand and from the minus strand, respectively, of the entire genome sequence of Escherichia coli K-12 strain (the accession number is U00096, and the length of the sequence is 4,639,221 base pairs). Furthermore, the positions of the stop codons of a total of 5340 of the determined CDSs were compared with the annotation information which was appended to the genome sequence of Escherichia coli K-12 strain and registered in GenBank of the NCBI, and it was understood that, among the 5340 CDSs, the stop codons of 3391 of the CDSs were identical with the those of CDSs registered in GenBank. The number of CDSs which encode polypeptides of 34 amino acids or more of Escherichia coli K-12 strain which is registered in GenBank of NCBI was 4274.

From the above results, when the annotation information of Escherichia coli K-12 strain which is registered in GenBank of NCBI was correct and the number (4289) of all the CDSs of Escherichia coli K-12 strain was used as the denominator, the accuracy of determination was 0.790 in the sensitivity, and was 0.635 in the specificity.

Example 9: Determination of the CDSs which encode polypeptides of 34 amino acids or more from the entire genome sequence of Escherichia coli K-12 strain (2)

According to the method described in Example 6, in other words, by combining the "method of determining a genetic structure from the viewpoint of a transcription unit structure" and the "method of determining a genetic structure using a shadow discrimination function", the CDSs which encode polypeptides of 34 amino acids or more were determined from the entire genome sequence of Escherichia coli K-12 strain MG1655.

According to the method described in Example 1, 2607 and 2648 CDSs which encode polypeptides of 34 amino acids or more were determined from the plus strand and from the minus strand respectively of the entire genome sequence of Escherichia coli K-12 strain (the accession number is U00096, and the length of the sequence is 4,639,221 base pairs).

The positions of the stop codons of a total of 5255 of the determined CDSs were compared with the annotation information which was appended to the genome sequence of Escherichia coli K-12 strain and registered in GenBank of the NCBI, and it was understood that, among the 5255 CDSs, the stop codons of 4149 of the CDSs was identical with the those of CDSs registered in GenBank.

From the above results, when annotation information of Escherichia coli K-12 strain which is registered in GenBank of NCBI was correct and the number (4289) of all the CDSs of Escherichia coli K-12 strain was used as the denominator, the accuracy of determination was 0.967 in the sensitivity, and was 0.790 in the specificity.

The results of Example 8 was compared with the results of this Example, it was apparent that the accuracy of CDS determination was enhanced by combining the "method of determining a genetic structure using a shadow discrimination function" of the present invention with the "method of determining a genetic structure from the viewpoint of a transcription unit structure" of the present invention.

Example 10: Determination of the CDSs which encode a polypeptide of 34 amino acids or more from the entire genome sequence of Escherichia coli K-12 strain (3)

According to the method of Example 7, which is the combination of the "method of determining a genetic structure from the viewpoint of a transcription unit structure", the "method of determining a genetic structure using a shadow discrimination function", and the "method for enhancing the determination accuracy of CDSs by using a code function" which is one of the "methods of deciding on the truth or falsity of a CDS by utilizing the coding potential", the CDSs which encode polypeptides of 34 amino acids or more were determined from the entire genome sequence of Escherichia coli K-12 strain MG1655.

As the result, 2185 CDSs and 2281 CDSs which encode polypeptides of 34 amino acids or more were determined from the plus strand and from the minus strand respectively of the entire genome sequence of Escherichia coli K-12 strain (the accession number is U00096, and the length of the sequence is 4,639,221 base pairs).

The positions of the stop codons of a total of 4466 of the determined CDSs were compared with the annotation information which was appended to the genome sequence of Escherichia coli K-12 strain and registered in GenBank of the NCBI, and it was understood that, among the 4466 CDSs, the stop codons of 4163 of the CDSs was identical with those of the CDSs registered in GenBank.

From the above results, when the annotation information of Escherichia coli K-12 strain which is registered in GenBank of NCBI was correct and the number (4289) of all the CDSs of Escherichia coli K-12 strain was used as the denominator, the accuracy of determination was 0.971 in the sensitivity, and was 0.932 in the specificity.

By comparing the results of Example 9 with the results of this Example, it is apparent that the accuracy of CDS determination is further enhanced by adding the "method of deciding upon the truth or the falsity of a CDS by utilizing the coding potential" to the combination of the "method of determining a genetic structure from the viewpoint of a transcription unit structure" and the "method of determining a genetic structure using a shadow discrimination function" of the present invention.

Among the CDSs of 34 amino acids or more of Escherichia coli K-12 strain which is registered in GenBank of the NCBI, the 112 CDSs which were not selected by this Example were:

b0005, htgA, b0024, b0302, b0395, ybbD, b0501, ybbV, b0538, ybcM, ninE, b0609, b0667, b0701, b0816, rmf, sfa, ycdF, b1030, b1146, ymgA, b1354, ydaS, b1369, b1371, ydcA, b1455, b1459, b1565, dicB, ydiE, b1936, b2191, b2331, b2390, b2504, b2596, b2635, yfjX, yfjZ, b2651, b2654, yqgC, b3004, ygiA, tdcR, b3122, insA_6, yrhB, tag, rfaL, yidP, rpmH, ilvM, b3808, b3837, yigW_2, b3913, ytfH, b4250, yjhE, insA_7, yjjY, yi82_1, insA_1, yacG, b0105, b0165, rnhA, yafW, ykfG, ykfF, ykfB, b0263, insA_2, insA_3, ykgJ, b0309, b0362, b0502, ybiI, ymfH, ycgW, yciG, lar, b1420, b1425, b1437, b1506, b1551, relF, b1567, malI, b1811, cspC, b1824, insA_5, b2083, b2084, b2641, b2653, b2856, b2859, b2862, yqgB, b3007, yqhC, ilvY, b3776, b3975, yjfA, and pyrL.

(2) Evaluation for the known genes of Escherichia coli K-12 strain

Escherichia coli is a living organism which has been well analyzed by experimental science. The CDSs described below which was considered to be analyzed already was compared with the results of analysis by the method of the present invention.

From the annotation information which is appended to the genome sequence of Escherichia coli K-12 strain which is registered in GenBank of the NCBI, genes which have "rpl", "rps", and "rpm" as the 3 initial letters of the gene name were searched for as ribosomal proteins, and a total of 55 CDSs were selected.

Genes which have "dna" as the 3 initial letters of the gene name were searched for as genes related to DNA synthesis and replication, and a total of 11 CDSs were selected.

Genes which have "rec" as the 3 initial letters of the gene name were searched for, as genes related to DNA recombination and repair, and a total of 13 CDSs were selected.

Genes which have "pyr" or "pur" as the 3 initial letters of the gene name were searched for, as genes related to th synthesis of bases of pyrimidine and purine, and a total of 23 CDSs were selected.

Furthermore, the 8 CDSs thrA, thrB, thrC, trpA, trpB, trpC, trpD, and trpE were selected as genes which constitute operons of well known biosynthesis pathway of amino acids.

As the results of comparison of the total of 110 of these known genes of Escherichia coli with the 4466 ORFs which encode polypeptides of 34 amino acids or more and determined by the method of the present invention, the CDSs which could not be determined by the method of the present invention were only rpmH and pyrL among the known 110 genes.

The positions of the start codons of the 108 known genes which was able to be determined by the method of the present invention were compared with the annotation information which is appended to the genome sequence of Escherichia coli K-12 strain and registered with GenBank of NCBI.

As the result, among the 108 genes, the positions of the start codons of 101 of the CDSs, excepting rplY, rpsB, dnaK, dnaQ, danB, recQ, and pyrF, were identical with the annotation information of GenBank of NCBI.

It was shown that the CDSs and the start codons can be determined at high accuracy from the genome sequence of a microbe by utilizing the method of the present invention.

Example 11: Determination of the CDSs which encode a polypeptide of 34 amino acids or more from the entire genome sequence of Bacillus subtilis (1)

The CDSs which encode polypeptides of 200 amino acids or more were determined at high accuracy from the entire genome sequence of Bacillus subtilis (strain 168) according to the method described in Example 5 which is the combination of the "method of determining a genetic structure from the viewpoint of a transcription unit structure" and the "method of determining a genetic structure using a shadow discrimination function", and by carrying out an additional process based upon the information of these determined CDSs, the CDSs which encode polypeptides of 34 amino acids or more were determined at high accuracy.

(1) Calculation of the ribosome binding score for each ORF and method of searching for the start codon

The ribosome binding score of each ORF of Bacillus subtilis were calculated and the start codons were searched for according to the method described in (3) of Example 1.

The true start codons and the CDSs were determined according to the method of determination of the CDSs from Escherichia coli K-12 strain, and utilizing the information of the sequence 3'-UUCCUCCA-5' in the sequence 3'-UUUCCUCCA-5' of 3' terminal sequence of 16S ribosomal RNA of Bacillus subtilis which was obtained by analysis of the data registered in GenBank of the NCBI.

(2) Determination of the CDSs which encode polypeptides of 200 amino acids or more

According to the method described in (2) to (9) of Example 1, 1255 CDSs and 1348 CDSs which encode polypeptides of 200 or more amino acids were determined from the plus strand and from the minus strand respectively of the entire genome sequence of Bacillus subtilis (strain 168) (the accession number was AL009126 and the length of the sequence was 4,214,814 base pairs).

(3) Determination of the CDSs of 34 amino acids or more and of the start codons

According to the method described in (2) of Example 5, the ORFs which encode polypeptides of 34 or more amino acids were searched for from the plus strand and from the minus strand of the entire genome sequence of Bacillus subtilis (strain 168) (the accession number was AL009126 and the length of the sequence was 4,214,814 base pairs).

As the result, 5325 ORFs were determined from the plus strand, and 5532 ORFs were determined from the minus strand. According to the method described in Example 5, the truth or falsity of the CDSs was decided and their start codons were determined based upon the information of the determined 2603 CDSs which encode polypeptides of 200 amino acids or more. As the results, 2350 CDSs were determined from the plus strand, and 2602 CDSs were determined from the minus strand.

(4) Outputting of the results about the determined CDSs and the evaluation of these results

The results about the 4952 CDSs which had been finally determined were outputted as a text file upon the hard disk.

As the results of comparing the positions of the stop codons of each of the CDSs which were outputted with the annotation information which is appended to the genome sequence of Bacillus subtilis and registered in GenBank of the NCBI, among the 4952 CDSs, the stop codons of 4011 of the CDSs was identical with those of the CDSs registered in GenBank of the NCBI.

When the annotation information for Bacillus subtilis registered in GenBank of NCBI was correct and the entire number (4100) of CDSs of Bacillus subtilis was used as denominator, the accuracy of determination was 0.978 in the sensitivity, and was 0.810 in the specificity.

Example 12: Determination of the CDS which encode polypeptides of 34 amino acids or more from the entire genome sequence of Bacillus subtilis (2)

The CDSs which encode polypeptides of 34 amino acids or more were determined from the entire genome sequence of Bacillus subtilis (strain 168) according to the method described in Example 7, which is the combination of the "method of determining a genetic structure from the viewpoint of transcription unit structure", the "method of determining a genetic structure using a shadow discrimination function", and the "method for enhancing the determination accuracy of CDSs by using a code function" which is one of the "methods of deciding on the truth or falsity of CDSs by using a coding potential".

(1) Determination of the CDS of 34 amino acids or more and the start codons from the entire genome sequence of Bacillus subtilis

According to the method of Example 7, 2,149 CDSs and 2,395 CDSs which encode polypeptides of 34 or more amino acids were determined from the plus strand and from the minus strand respectively of the entire genome sequence of Bacillus subtilis (strain 168) (the accession number was AL009126 and the length of the sequence was 4,214,814 base pairs).

As the results of comparing the positions of the stop codons of total of the 4544 determined CDSs with the annotation information which is appended to the genome sequence of Bacillus subtilis and registered in GenBank of the NCBI, among the 4544 CDSs, the stop codons of 4007 of the CDSs was identical with those of the CDSs registered in GenBank of the NCBI.

From the above results, when annotation information for Bacillus subtilis registered in GenBank of NCBI was correct, and the entire number (4100) of CDSs of Bacillus subtilis was used as the denominator, the accuracy of determination was 0.977 in the sensitivity, and was 0.881 in the specificity.

From the comparison of the results of Example 11 with the results of this embodiment, it is apparent that the accuracy of CDS determination is further enhanced by combining the "method of deciding upon the truth or the falsity of a CDS by utilizing the coding potential" in addition to the combination of the "method of determining a genetic structure from the viewpoint of transcription unit structure" and the "method of determining a genetic structure using a shadow discrimination function" of the present invention.

Among the CDSs of 34 amino acids or more of Bacillus subtilis which are registered in GenBank of NCBI, the 87 CDSs which were not selected by this Example were: yaaB, dacA, yazB, rpmG, ybdL, yczB, comS, phrC, yczI, ydaQ, phrI, ydfH, ydiN, ydiQ, yezD, yfmN, yfmA, yflD, yflC, yhcD, yjcB, ykoP, rpmF, yoaV, phrK, yoqO, ypzC, ypuA, yqzI, yrkM, yraC, yrvP, yscA, ytoA, yufC, sbo, ywhR, phrF, ywdC, yxjJ, yxeE, yycC, yceK, ycgJ, ydzA, sapB, yhdD, yhdS, yheF, yhaY, yhaK, yhfD, yhfH, yhjQ, yjcE, ykrB, yobM, yojC, yotN, yotD, yorY, yoqK, yonT, sunA, ypcP, cotD, ypuE, yqgO, yqfV, rpsU, yrkS, yrkG, yrkB, yrdK, yrdB, sigZ, yrzK, yshA, comX, yuzF, yuiA, yvzB, usd, ywzB, spsA, yyzE, and rpmH.

(2) Evaluation for the known genes of Bacillus subtilis

Bacillus subtilis is a living organism which has been well analyzed by experimental science. The CDSs described below which was considered to be analyzed already was compared with the results of analysis by the method of the present invention.

From the annotation information which is appended to the genome sequence of Bacillus subtilis which is registered in GenBank of the NCBI, genes which have "rpl", "rps", and "rpm" as the 3 initial letters of the gene name were searched for, as rebosomal proteins, and a total of 52 CDSs were selected.

genes which have "dna" as the 3 initial letters of the gene name were searched for, as genes which are related to DNA synthesis and replication, and a total of 11 CDSs were selected.

genes which have "dna" as the 3 initial letters of the gene name were searched for, as genes which are related to DNA synthesis and replication, and a total of 11 CDSs were selected.

genes which have "rec" as the 3 initial letters of the gene name were searched for, as genes which are related to DNA recombination and repair, and a total of 5 CDSs were selected.

genes which have "pyr" or "pur" as the 3 initial letters of the gene name were searched for, as genes which are related to th synthesis of bases of pyrimidine and purine, and a total of 24 CDSs were selected.

The 10 CDSs thrS, thrB, thrC, thrZ, trpA, trpB, trpC, trpD, trpE, and trpF were selected as genes of well known biosynthesis pathway of amino acids.

Results of the detemination of 4007 ORFs which encode polypeptides of 34 amino acids or more by the method of the present invention were investigated in relation to the total of 102 of these known genes of Bacillus subtilis.

As the result, all the 102 genes was able to be determined among the above described 102 known genes. Next, the positions of the start codons of the 102 known genes which was able to be determined by the method of the present invention were compared with the annotation information which is appended to the genome sequence of Bacillus subtilis and registered with GenBank of NCBI.

As the results, the positions of the start codons of 82 CDSs among the 102 CDSs were identical.

Example 13: Determination of the CDS which encode polypeptides of 34 amino acids or more from the entire genome sequence of Pseudomonas aeruginosa genome (1)

Among the microbes for which the entire genome sequence has been determined, the microbe of which the GC content of the entire genome sequence is 60% or more and of which genetic and biochemical analysis has progressed most is Pseudomonas aeruginosa. Thus, in this Example, the CDSs which encode polypeptides of 34 amino acids or more were determined from the entire genome sequence of Pseudomonas aeruginosa (strain PAO1).

According to the method described in Example 5, the CDSs which encode polypeptides of 200 amino acids or more were determined at high accuracy from the entire genome sequence of Pseudomonas aeruginosa (starin PAO1), and based upon the information for the determined CDSs, the CDSs which encode polypeptides of 34 amino acids or more were determined with high accuracy by carrying out the processes of the "method of determining a genetic structure using a shadow discrimination function".

(1) Calculation of the ribosome binding score for each ORF and search for the start codons

According to the method described in Example 1 (3), the ribosome binding score for each ORF of Pseudomonas aeruginosa was calculated and the start codons were searched for.

The true start codons and the CDSs were determined according to the method of determination of the CDSs from Escherichia coli K-12 strain and utilizing the information of the sequence 3'-UUCCUCCA-5' in the sequence 3'-AUUCCUCCA-5' of 3' terminal sequence of 16S ribosomal RNA of Pseudomonas aeruginosa which was obtained by analysis of the data registered in GenBank of the NCBI.

(2) Determination of the CDSs of 200 amino acids or more

Using the method described in Example 1 (2) - (9) and the method of determination of the start codon described above, 2069 CDSs and 1644 CDSs which encode polypeptides of 200 amino acids or more were determined from the plus strand and from the minus strand respectively of the entire genome sequence of Pseudomonas aeruginosa (strain PAO1) with the GC content of 66.56% (the accession number is AE004091, and the length of the sequence is 6,264,403 base pairs).

(3) Determination of the CDSs of 34 amino acids or more and the start codons.

According to the method described in Example 5 (2), the ORFs which encode polypeptides of 34 amino acids or more were searched for from the plus strand and from the minus strand of the entire genome sequence of Pseudomonas aeruginosa (strain PAO1) (the accession number is AE004091, and the length of the sequence is 6,264,403 base pairs).

The threshold value which was used in deciding the truth or falsity of the CDSs based upon the value $Sd_A$ of the "shadow discrimination function" which was described in Example 5 (2) was changed as described below. The ORF was decided to be a true ORF (CDS): if "$Sd_A$ was greater than or equal to 1.0", in the case of an ORF which encodes a polypeptide of 100 amino acids or more; if "$Sd_A$ was greater than or equal to 1.1", in the case of an ORF which encodes a polypeptide of 60 amino acids or more and of 99 amino acids or less; and if "$Sd_A$ is greater than or equal to 1.1", in the case of an ORF which encodes a polypeptide of 34 amino acids or more and of 59 amino acids or less.

As the results of searching for the ORFs based upon this condition, 8519 ORFs were determined from the plus strand, and 8307 ORFs were determined from the minus strand.

The truth or falsity of the CDSs was decided and the start codons were determined according to the method described in Example 5, based upon the information of 3713 CDSs which encode polypeptides of 200 amino acids or more and was determined as described above.

As the results, 3062 CDSs were determined from the plus strand, and 3166 CDSs were determined from the minus strand.

(4) Outputting the results about the determined CDSs and the evaluation of the results

The results about the 6288 CDSs which were finally determined were outputted as a text file upon a hard disk.

The positions of the stop codons of the CDSs which had been outputted were compared with the annotation information which was appended to the genome sequence of Pseudomonas aeruginosa and registered in GenBank of NCBI, and among the 6228 CDSs, the stop codons of the 5018 CDSs were identical with those of the CDSs registered in GenBank.

When the annotation information of Pseudomonas aeruginosa which is registered in GenBank of NCBI was correct and the total number (5565) of CDSs of the Pseudomonas aeruginosa was used as the denominator, the accuracy of determination was 0.902 in the sensitivity, and was 0.806 in the specificity.

Example 14: Determination of the CDS which encode polypeptides of 34 amino acids or more from the entire genome sequence of Pseudomonas aeruginosa genome (2)

In this Example, it was shown that the accuracy of CDS determination was further enhanced by combining a "method of enhancing CDS determination accuracy from the viewpoint of the GC content of bases in the codons" with the method described in Example 13 which is the combnation of the "method of determining a genetic structure from the viewpoint of a transcription unit structure" and the "method of determining a genetic structure using a shadow discrimination function".

(1) A method of determining a genetic structure from the viewpoint of the GC content of bases in the codons

The content of the first and the third G residues and C residues of the codons within the CDS was defined as the GC content of the bases within the codons, and was calculated as the value of $GC_i$ of the formula (5) below (hereinafter referred to as the "GC function"):

$$GC_i = \left(y^{i(1)} + y^{i(3)}\right) \Big/ \sum_{r=1}^{3} y^{i(r)} \quad \text{wherein} \quad y^{i(r)} = \sum_{n=1}^{N_i} \sum_{b=1}^{4} x_{n(b)}^{i(r)} \qquad (5)$$

[herein when the r-th base (r is 1, 2, 3) of the n-th codon of the i-th CDS is b (b is 1, 2, 3, 4), then

$$\chi_{n|b|}^{i|r|} \text{ is } \chi_{n|b|}^{i|r|} = 1 \text{ (b= 1 or 2)}$$

$$\chi_{n|b|}^{i|r|} = 0 \text{ (b= 3 or 4)}$$

and, b is respectively 1, 2, 3, or 4, when the r-th base of the n-th codon of the i-th CDS is respectively G, C, A, or T, i and n are positive integers, and $N_i$ denotes the total number of codons of the i-th CDS (excluding its stop codon)].

(2) The determination of the CDS of 34 amino acids or more from the entire genome sequence of Pseudomonas aeruginosa genome

The value of the above described "GC function" was utilized in the "re-searching for a start codon" and in the "decision of the truth or falsity of a CDS".

In the "re-searching for a start codon", the value of the "GC function" is utilized when it was decided that there was no possibility of forming a polycistronic transcription unit by the method according to the description of Example 1 (3) or (6)

Specifically, the value of the "GC function" of the region of 60 amino acid from the 5' terminal of the selected candidate for a true ORF which encodes a polypeptide of 150 amino acids or more was obtained, and, if the value was less than or equal to 2/3, the start codon was re-searched for from the next codon to the determined start codon in the direction towards the 3'. "Re-searching for a start codon" was repeated a maximum of twice.

With regard to "deciding the truth or falsity of a CDS", the value of the "GC function" of the selected CDS was obtained, and, if its value was less than or equal to 2/3, it was decided that the CDS is a "false CDS".

According to the method wherein these processes were added to the method as described in Example 13, The CDSs which encode polypeptides of more than 34 amino acids were determined from the plus strand and the minus strand of the entire genome sequence (the accession number is AE004091, and the length of the sequence is 6,264,403 base pairs) of Pseudomonas aeruginosa (strain PAO1).

As the results of selecting CDSs in this manner, 2896 CDSs and 3290 CDSs which encode polypeptides of 34 amino acids or more were determined from the plus strand and from the minus strand respectively.

The positions of the stop codons of a total of the determined 6186 CDSs were compared with the annotation information which was appended to the genome sequences of Pseudomonas aeruginosa and registered in GenBank of NCBI.

As the results, the stop codons of 5300 of the CDSs were identical with those of the CDSs registered in GenBank.

From the above results, when the annotation information for Pseudomonas aeruginosa registered in GenBank of NCBI was correct and the entire number (5565) of CDSs of Pseudomonas aeruginosa was used as the denominator, the accuracy of determination was 0.952 in the sensitivity, and was 0.857 in the specificity.

From comparison of the results of Example 13 with the results of this Example, it was shown that the accuracy of CDS determination was further enhanced by combining a "method of determining a genetic structure from the viewpoint of the GC content of the bases in the codons" with the method which is the combination of the "method of determining a genetic structure from the viewpoint of a transcription unit structure" and the "method of determining a genetic structure using a shadow discrimination function" of the present invention.

Example 15: The determination of the CDSs which encode polypeptides of 34 amino acids or more from the entire genome sequence of Pseudomonas aeruginosa genome (3)

According to the method of Example 14 which is the combination of the "method of determining a genetic structure from the viewpoint of a transcription unit structure", the "method of determining a genetic structure using a shadow discrimination function", the "method of determining a genetic structure from the viewpoint of the GC content of the bases in the codons", and the "method for enhancing the determination accuracy of CDSs by using a code function" which is one of the "methods of deciding upon the truth or the falsity of a CDS by utilizing the coding potential", the CDSs which encode polypeptides of 34 amino acids or more were determined from the entire genome sequence of Pseudomonas aeruginosa (strain PAO1).

(1) Determination of the CDS of 34 amino acids or more and the start codons from the entire genome sequence of Pseudomonas aeruginosa

Except changing the threshold value of the shadow discrimination function for selecting the CDSs, according to the method of Example 7, the CDSs which encode polypeptides of 34 amino acids or more were determined from the entire genome sequence (the accession number was AE004091, and the length of the sequence was 6,264,403 base pairs) of a Pseudomonas aeruginosa (strain PAO1).

The threshold value of the shadow discrimination function for selecting the CDS was set in the following manner.

Based upon the value of the "code function" $Cd_A$ which was obtained according to the above Example 7, the ORF-A was decided as a true CDS if the following condition was satisfied. It was decided that said ORF was a true ORF (CDS): if "$Cd_A$ is greater than or equal to 1.5" in the case of an ORF which encodes a polypeptide of 100 amino acids or more; if "$Cd_A$ is greater than or equal to 1.5" in the case of an ORF which encodes a polypeptide of 60 amino acids or more and 99 amino acids or less; if "$Cd_A$ is greater than or equal to 1.6" in the case of an ORF which encodes a polypeptide of 34 amino acids or more and 59 amino acids or less, when.

Furthermore, when the value of the "shadow discrimination function" was obtained, it was possible to decide on the truth or falsity of the CDS, based upon the value ($Sd_A$ X $Cd_A$) which is the product of the value $Sd_A$ of the "shadow discrimination function" of ORF-A and of the value $Cd_A$ of the "code function". It is decided that the ORF is a true ORF (CDS): if " $Sd_A$ X $Cd_A$ is greater than or equal to 1.8" in the case of an ORF which encodes a polypeptide of 100 amino acids or more; if "$Sd_A$ X $Cd_A$ is greater than or equal to 2.0" in the case of an ORF which encodes a polypeptide of 60 amino acids or more and 99 amino acids or less; and if " $Sd_A$ X $Cd_A$ is greater than or equal to 2.1" in the case of an ORF which encodes a polypeptide of 34 amino acids or more and 59 amino acids or less.

As the results of selecting CDSs in this manner, 2716 CDSs and 2859 CDSs which encode polypeptides of 34 amino acids or more were determined from the plus strand and from the minus strand respectively.
The positions of the stop codons of a total of the 5575 determined CDSs were compared with the annotation information appended to the genome sequence of Pseudomonas aeruginosa registered in GenBank of NCBI.
As the results, among the 5575 CDSs, the stop codons of 5299 of the CDSs were identical with those of the CDSs registered in GenBank.
From the above results, when the annotation information for Pseudomonas aeruginosa registered in GenBank of NCBI was correct and the entire number (5565) of CDSs of the K-12 strain of Pseudomonas aeruginosa was used as the denominator, the accuracy of determination was 0.952 in the sensitivity, and was 0.950 in the specificity.
From comparing the results of Example 14 with the results of this Example, it was shown that the accuracy of CDS determination was further enhanced by combining a "method of deciding upon the truth or the falsity of a CDS by utilizing the coding potential" with the method which is the combination of the "method of determining a genetic structure from the viewpoint of a transcription unit structure", the "method of determining a genetic structure using a shadow discrimination function" and the "method of determining a genetic structure from the viewpoint of the GC content of the bases in the codons" of the present invention.
Among the CDSs of 34 amino acids or more of Pseudomonas aeruginosa which are recorded in GenBank of NCBI, the 262 CDSs which were not selected in this example were: PA0012, PA0047, PA0050, PA0104, PA0127, PA0128, PA0135, PA0160, PA0161, PA0167, PA0279, PA0318, PA0433, PA0462, PA0478, PA0483, PA0529, PA0560, PA0621, PA0632, PA0634, PA0635, PA0642, PA0646, PA0647, PA0648, PA0715, PA0716, PA0719, PA0722, PA0729, PA0756, PA0817, PA0819, PA0820, PA0884, PA0885, csrA, PA0954, PA0960, PA0980, PA0981, PA0983, PA0984, PA0986, PA0991, PA0993, PA1096, PA1112, pys2, imm2, PA1152, PA1329, PA1357, PA1369, PA1370, PA1377, galE, PA1385, PA1386, PA1414, PA1426, PA1427, PA1441, PA1468, PA1469, ccmG, PA1545, gpsA, PA1625, pcrR, pscE, pscF, PA1834, PA1882, PA1889, PA1936, PA1963, PA2036, PA2037, PA2105, PA2139, PA2146, PA2221, PA2245, PA2372, PA2456, PA2480, PA2485, cysK, PA2710, PA2816, oprI, PA2878, PA2880, hisJ, rmf, PA3090, xcpP, PA3218, PA3274, PA3451, pyrC, PA3632, PA3662, PA3717, PA3764, PA3843, PA3888, PA3964, thiD, PA3981, PA3988, PA4028, PA4074, PA4095, PA4131, PA4134, PA4141, PA4146, PA4291, PA4295, fimU, PA4776, PA4789, PA4860, PA4880, vanA, PA5202, PA5395, PA5432, PA5462, PA5480, PA0014, PA0076, PA0141, PA0257, PA0258, PA0264, PA0311, PA0388, PA0442, PA0453, PA0468, nirM, PA0532, PA0656, PA0781, prpB, PA0797, PA0805, PA0814, PA0822, PA0874, PA0941, PA0977, PA0985, PA1026, PA1034, PA1044, PA1170, napE, PA1195, PA1332, PA1333, PA1359, PA1371, PA1372, rsaL, PA1508, PA1509, PA1531, PA1540, PA1653, pscQ, PA1799, PA1935, PA1939, PA2182, PA2222, PA2223, PA2224, PA2226, PA2227, PA2228, PA2459, PA2460, PA2461, PA2544, PA2570, PA2582, PA2621, PA2730, PA2731, PA2772, PA2775, PA2794, rpmF, PA2980, moaB2, PA3034, PA3051, PA3065, PA3143, PA3144, wbpL, wbpK, wbpJ, wbpI, wbpH, wbpG, hisF2, hisH2, wzx, wzy, wbpE, wbpD, PA3157, wzz, PA3169, PA3270, PA3291, PA3292, PA3390, PA3520, PA3577, PA3591, PA3623, PA3696, PA3782, PA3829, PA3866, PA3998, PA4041, pchR, rpmC, rpsJ, secE, birA, PA4326, PA4349, PA4388, pilA, PA4534, rpmA, PA4607, PA4638, PA4674, PA4709, secG, tpiA, selA, PA4840, PA4872, PA5061, PA5086, PA5087, PA5088, PA5104, trxA, PA5388, and PA5528.

Industrial Applicability

According to the method of the present invention, it is possible to determine a genetic structure with enhanced accuracy.
In particular, according to the method of the present invention, the prediction of the structure of polycistronic transcription units is possible, and it is possible to enhance the accuracy of determination of the positions of start codons, information in advance is unnecessary, and it is possible to determine a genetic structure for the nucleotide sequence with the high GC content.

## Claims

**1.** A method of determining a genetic structure of a prokaryote, which comprises the steps (a) to (g) described below:

(a) setting a translation stop codon from information about the nucleotide sequence of a prokaryote (a nucleotide sequence is a sequence of DNA or RNA), and setting a provisional translation start codon which yields the longest open reading frame (hereinafter abbreviated as ORF) based upon said translation stop codon;
(b) deciding that the ORF-A and the ORF-B have a possibility to form a single transcription unit if the provisional start codon of the ORF-A is upstream of the translation stop codon of the ORF-B, or is within $D_S$ bases downstream of said translation stop codon [herein $D_S$ is an integer from 20 to 100], wherein any two neighboring ORFs which are obtained in the step (a) and present on the same strand are termed ORF-A and ORF-B from downstream;

(c) determining that the candidate for the translation start codon is the translation start codon of ORF-A if the ORF-A and the ORF-B are decided to have a possibility to form a single transcription unit in the step (b) and if the candidate for the translation start codon is present within a region (hereinafter termed the "vicinity of the translation stop codon") between $D_B$ bases downstream from the first T (thymidine) residue of the translation stop codon of the ORF-B and $U_B$ bases upstream from said T residue [herein $D_B$ is an integer between 10 and 20, and $U_B$ is an integer between 3 and 15], and determining the translation start codon of the ORF-A from a priority ranking determined by using the distance between each candidate and the translation stop codon of the ORF-B as an indicator if there is a plurality of candidates;

(d) examining whether a candidate for the translation start codon of the ORF-A is present within a region (hereinafter termed the "region around the vicinity of the translation stop codon") between $R_D$ bases downstream from the first T residue of the translation stop codon of the ORF-B and $R_U$ bases upstream from said T residue and excluding said "vicinity of the translation stop codon" [herein $R_D$ is an integer from 30 to 120, and $R_U$ is an integer from 20 to 120] if the translation start codon of the ORF-A can not be determined in the step (c);

(e) examining whether a ribosome binding site is present from 1 to 30 bases upstream of a candidate for the translation start codon of the ORF-A if the candidate is present in the region around the vicinity of the translation stop codon in the step (d), determining its ribosome binding sequence if such a ribosome binding site is present, and determining that the candidate which corresponds to said ribosome binding sequence is the translation start codon of the ORF-A;

(f) searching for up to the number N of candidates for the translation start codon including the provisional start codon which yields the longest ORF from the 5' terminal of an ORF-A which is not decided to have a possibility to form a single transcription unit in the step (b) or whose translation start codon is not determined in the step (e), investigating whether a ribosome binding site is present from 1 to 30 bases upstream of each candidate, determining its ribosome binding sequence if such a ribosome binding site is present, and determining that the candidate which corresponds to said ribosome binding sequence is the translation start codon [herein N is an integer from 5 to 20];

(g) confirming the positions of the translation start codon and the translation stop codon, the coding region, and the transcription units from the results of determination by the step (c), the step (e) or the step (f) to determine a genetic structure.

**2.** The method of determining a genetic structure according to Claim 1, wherein the step (e) is a step of determining the translation start codon of an ORF-A by the following steps:

determining that a mRNA sequence whose ribosome binding score exceeds a threshold value $V_3$, described below, is a ribosome binding sequence [herein $V_3$ is an integer from 4 to 12], wherein the paired state between a mRNA sequence of 4 to 17 bases upstream of a candidate for the translation start codon of the ORF-A and a sequence (3'-UUCCUCC-5') involved in the binding to mRNA in a 16S rRNA 3' terminal sequence, or between a mRNA sequence of 4 to 16 bases upstream of said candidate and a sequence (3'-UCCUCC-5') involved in the binding to mRNA in a 16S rRNA 3' terminal sequence, is expressed as a numerical value, which is termed a "score which shows the binding state between mRNA and a ribosome" (hereinafter termed a ribosome binding score), according to the four rules described below:

(1) A pairing of G and C yields +4;
(2) A pairing of A and U yields +2;
(3) A pairing of G and U yields +1;
(4) When no pairing is present at a base pair which is adjacent to a base pair where a pairing is present, then this yields -1;

determining that the candidate which corresponds to said ribosome binding sequence is the translation start codon; dividing the "region of an ORF-B around the vicinity of the stop codon" into the two of "the region downstream of said vicinity" and "the region upstream of said vicinity" if there is a plurality of said translation start codons, and

determining the one of said translation start codons which has the highest priority is the true translation start codon based on the priority of "the region downstream of said vicinity" and "the region upstream of said vicinity" in that order;

determining the translation stop codon of the ORF-A from a priority ranking defined by using the distance from the translation stop codon of the ORF-B as an indicator if a plurality of translation start codons is present within the respective regions.

**3.** The method of determining a genetic structure according to Claim 1 or Claim 2, wherein the step (f) is a step of determining the translation start codon of an ORF-A by the following steps:

determining that the mRNA sequence whose ribosome binding score exceeds a threshold value V, described below,$_1$ is a ribosome binding sequence, wherein the paired state between a mRNA sequence of from 4 to 17 bases upstream of a candidate for the translation start codon of the ORF-A and a sequence (3'-UUCCUCC-5') involved in the binding to mRNA in a 16S rRNA 3' terminal sequence, or between a mRNA sequence of 4 to 16 bases upstream of said candidate and a sequence (3'-UCCUCC-5') involved in the binding to mRNA in a 16S rRNA 3' terminal sequence, is expressed as a numerical value, termed "ribosome binding score", according to the four rules described below:

(1) A pairing of G and C yields +4;
(2) A pairing of A and U yields +2;
(3) A pairing of G and U yields +1;
(4) When no pairing is present at a base pair which is adjacent to a base pair where a pairing is present, then this yields -1;

determining that the candidate which corresponds to said ribosome binding sequence is the translation start codon; determining that the translation start codon corresponding to the ribosome binding sequence which yields the highest score is the true translation start codon if there is a plurality of said translation start codons; setting one or more threshold value(s) smaller than $V_1$, which include the threshold value $V_3$, if there is no candidate which exceeds the threshold value $V_1$, and
determining the translation start codon of the ORF-A in a stepwise manner if said threshold value is exceeded [herein $V_1$ is an integer which is greater than the $V_3$ of Claim 2, and which is between 7 and 14].

**4.** The method of determining a genetic structure according to Claim 2 or Claim 3, wherein the "ribosome binding score" is calculated by deducting a numerical value $P_G$ if the translation start codon is GTG, or by deducting a numerical value $P_T$ if the translation start codon is TTG [herein $P_G$ is an integer from 1 to 4, and $P_T$ is an integer from 2 to 6].

**5.** A method of determining a genetic structure, wherein a transcription unit P, a coding region A, a transcription unit Q, and a coding region B is determined by utilizing the method according to any one of Claim 1 to Claim 4, which further comprises the steps (h) to (j) described below if the transcription unit P or the coding region A overlaps with the transcription unit Q or the coding region B:

(h) deciding that the transcription unit Q or the coding region B is a "false transcription unit" or a "false coding region" if a transcription unit Q or a coding region B which is present upon the same strand as a transcription unit P or a coding region A is included in the transcription unit P or the coding region A;
(i) deciding that the transcription unit Q or the coding region B is a "false transcription unit" or a "false coding region" if a transcription unit Q or a coding region B which is present upon the complementary strand to a transcription unit P or a coding region A is included in the transcription unit P or the coding region A;
(j) deciding that the transcription unit or coding region whose length is shorter is a "false transcription unit" or a "false coding region" when a transcription unit P or a coding region A overlaps with a transcription unit Q or a coding region B which is present upon the complementary strand.

**6.** A method of determining a genetic structure, wherein the method of determining a genetic structure according to any one of Claim 1 to Claim 5 is utilized repeatedly.

**7.** A method of determining a genetic structure of a prokaryote, which comprises the steps (k) and (1) described below:

(k) selecting k types of combination of codons wherein "the frequency of appearance of one codon is high and the frequency of appearance of a codon which has the complementary sequence to the 3-base sequence of said codon is low" in a plurality (the number T) of determined coding regions of the prokaryote;
(l) comparing the "number of times of the k types of codons whose frequency of appearance is high appearing in a coding region A which is assumed to be a coding region" with the "number of times of the k types of codons whose frequency of appearance is low appearing in said coding region A", and deciding on the truth or falsity of said coding region A [herein k is an integer greater than or equal to 5 and less than or equal to 20].

**8.** The method of determining a genetic structure according to Claim 7, wherein the method for comparing the "number of times of the k types of codons whose frequency of appearance is high appearing in a coding region A which is assumed to be a coding region" with the "number of times of the k types of codons whose frequency of appearance is low appearing in said coding region A" is a method which involves using "the reciprocal of the sum of 1 and the ratio of the number of the latter to the number of the former" as a calculation formula and which involves deciding that said coding region A is a "false coding region" if the value of said reciprocal is less than a fixed value.

**9.** The method of determining a genetic structure according to Claim 7, which is based on the nucleotide sequence of the number T of determined coding regions of the prokaryote and comprises the steps (m) to (p) described below:

(m) arranging the 64 types of codons so that the 3-base sequence of the i-th codon has the complementary sequence to the nucleotide sequence of the (i+32)-th codon;
(n) obtaining $y_i$ from the formula (2) below and $y_{i+32}$ from the formula (3) below:

$$y_i = \left(\sum_{t=1}^{T} C_i^t - \sum_{t=1}^{T} C_{i+32}^t\right) \Big/ \sum_{t=1}^{T}\sum_{j=1}^{64} C_j^t \qquad (2)$$

$$y_{i+32} = \left(\sum_{t=1}^{T} C_{i+32}^t - \sum_{t=1}^{T} C_i^t\right) \Big/ \sum_{t=1}^{T}\sum_{j=1}^{64} C_j^t \qquad (3)$$

wherein the number of appearances of the i-th codon in the t-th coding region is expressed as $C_j^t$

(o) rearranging the 64 types of codon in the step (m) in descending order of the $y_i$ and the $y_{i+32}$, selecting top k types of codons for which the value of $y_i$ or of $y_{i+32}$ is large, and obtaining the value of $Sd_A$ for a coding region A by the following formula (4):

$$Sd_A = 2 \times \sum_{i=1}^{k} C_i^A \Big/ \left(\sum_{i=1}^{k} C_i^A + \sum_{i=65-k}^{64} C_i^A\right) \qquad (4)$$

[herein the value of $Sd_A$ is defined as 1 if

$$\left(\sum_{i=1}^{k} C_i^A + \sum_{i=65-k}^{64} C_i^A\right)$$

is zero].
(p) deciding that a coding region A is a true coding region if the value of $Sd_A$ of said coding region calculated in the process (o) is greater than or equal to a threshold value $S_1$, and that it is a false coding region if said value of $Sd_A$ is less than the threshold value $S_1$ [herein T is an integer greater than or equal to 2, i is a positive integer less than or equal to 32, j is a positive integer less than or equal to 64, t is a positive integer less than or equal to T, k is an integer from 5 to 20, and $S_1$ is a value from 0.8 to 1.8].

**10.** A method of determining a genetic structure of a prokaryote, which comprises the steps (q) and (r) described below, wherein a coding region of the prokaryote or a coding region A which is assumed to be a coding region overlaps with a coding region B which is assumed to be a coding region and present upon the complementary strand, and said coding region B is included in said coding region A:

(q) comparing the length $L_B$ (in base pairs) of said coding region B with the length $L_A$ (in base pairs) of said coding region A, and deciding that said coding region B is a "false coding region" if $L_B$ is less than or equal to $T_P$% of $L_A$;
(r) deciding on the truth or falsity of said coding region A and of said coding region B by the method according to any one of Claim 7 to Claim 9 if $L_B$ exceeds $T_P$% of $L_A$ [herein, $T_P$ is a positive integer from 30 to 95].

**11.** A method of determining a genetic structure, **characterized by** removing the translation stop codons from the coding regions which form a transcription unit, and linking up the resulting coding regions into a single coding region, before utilizing the method according to any one of Claim 7 to Claim 10.

**12.** A method of determining a genetic structure, which comprises:

deciding on the truth or falsity of a coding region or of a transcription unit which is determined by the method of determining a genetic structure according to any one of Claim 1 to Claim 6, by utilizing the method of determining a genetic structure according to any one of Claim 7 to Claim 11.

**13.** A method of determining a genetic structure, which comprises:

deciding on the truth or falsity of a coding region which encodes a polypeptide of $L_M$ amino acids or more in length, by using the method of determining a genetic structure according to any one of Claim 7 to Claim 12, based on the nucleotide sequence of a coding region which is determined by using the method of determining a genetic structure according to any one of Claim 1 to Claim 12 and which encodes a polypeptide of $L_F$ amino acids or more in length [herein $L_F$ is a positive integer greater than or equal to 100, and $L_M$ is a positive integer greater than or equal to 20].

**14.** A method of determining a genetic structure of a prokaryote, **characterized by** deciding that a coding region in the nucleotide sequence of the prokaryote is a "false coding region" if the GC content of said nucleotide sequence is greater than 50% and if a content, calculated by utilizing a calculation formula which yields a content of the first and third G residues and C residues of the codons in said nucleotide sequence, is less than a fixed value.

**15.** The method of determining a genetic structure according to Claim 14, wherein the following formula (5) is used as a calculation formula, the value of $GC_i$ described below is used as a calculated content, and one value which is selected from 0.6 to 0.75 is used as a fixed value:

$$GC_i = \left(y^{i(1)} + y^{i(3)}\right) \Big/ \sum_{r=1}^{3} y^{i(r)} \quad \text{wherein} \quad y^{i(r)} = \sum_{n=1}^{N_i} \sum_{b=1}^{4} x_{n(b)}^{i(r)} \qquad (5)$$

[herein when the r-th base (r is 1, 2, or 3) of the n-th codon of the i-th coding region is b (b is 1, 2, 3, or 4), then

$$\chi_{n|b|}^{i|r|} \text{ is } \chi_{n|b|}^{i|r|} = 1 \text{ (b= 1 or 2)}$$

$$\chi_{n|b|}^{i|r|} = 0 \text{ (b= 3 or 4)}$$

and, as for b, when the r-th base of the n-th codon of the i-th coding region is G, C, A, or T, b is 1, 2, 3, or 4, respectively, i and n are positive integers, and $N_i$ denotes the total number of the codons (excluding the translation stop codon) of the i-th coding region].

**16.** A method of determining a genetic structure of a prokaryote, which comprises:

deciding that a coding region in the nucleotide sequence of the prokaryote is a "false coding region" if the GC content of said nucleotide sequence is greater than 50%, and if a content, calculated by utilizing a calculation formula which yields a content of the first and third G residues and C residues of the codons in said nucleotide sequence, is less than a fixed value; and

re-searching for a translation start codon which is present downstream of said translation start codon which is decided to be false.

**17.** The method of determining a genetic structure according to Claim 16, wherein the following formula (5) is used as a calculation formula, the value of $GC_i$ described below is used as a calculated content, and one value which is selected from 0.6 to 0.75 is used as a fixed value:

$$GC_i = \left(y^{i(1)} + y^{i(3)}\right) \Big/ \sum_{r=1}^{3} y^{i(r)} \quad \text{wherein} \quad y^{i(r)} = \sum_{n=1}^{N_i} \sum_{b=1}^{4} x_{n(b)}^{i(r)} \qquad (5)$$

[herein, when the r-th base (r is 1, 2, or 3) of the n-th codon of the i-th coding region is b (b is 1, 2, 3, or 4), then

$\chi_{n|b|}^{i|r|}$ is $\chi_{n|b|}^{i|r|} = 1$ (b= 1 or 2)

$\chi_{n|b|}^{i|r|} = 0$ (b= 3 or 4)

and, as for b, when the r-th base of the n-th codon of the i-th coding region is G, C, A, or T, b is 1, 2, 3, or 4, respectively, i and n are positive integers, and $N_i$ denotes the total number of the codons (excluding the translation stop codon) of the i-th coding region].

**18.** A method of determining a genetic structure of a prokaryote whose GC content in the nucleotide sequence exceeds 50%, wherein the method of determining a genetic structure according to any one of Claim 1 to Claim 13 and the method of determining a genetic structure according to any one of Claim 14 to Claim 17 are utilized.

**19.** A method of determining a genetic structure of a prokaryote, wherein the method of determining a genetic structure according to any one of Claim 1 to Claim 18 and a "method of deciding on the truth or falsity of a coding region by utilizing a coding potential" are utilized.

**20.** The method of determining a genetic structure according to Claim 19, wherein said "method of deciding on the truth or falsity of a coding region by utilizing a coding potential" is a method of deciding on the truth or falsity of the coding region A described below by, based upon the nucleotide sequences of the number T of the determined coding regions of the prokaryote, comparing the "number of times of m types of codons whose frequency of appearance is high appearing in the coding region A which is assumed to be the coding region" with the "number of times of m types of codons whose frequency of appearance is low appearing in the coding region A" for the number T of coding regions [herein, T is an integer greater than or equal to 2, and m is an integer greater than or equal to 5 and less than or equal to 20].

**21.** The method of determining a genetic structure according to Claim 20, wherein the method of comparing the "number of times of m types of codons whose frequency of appearance is high appearing in the coding region A which is assumed to be the coding region" and the "number of times of m types of codons whose frequency of appearance is low appearing in the coding region A" is a method which involves utilizing the "reciprocal of the sum of 1 and the ratio of the number of the latter to the number of the former" as a calculation formula, and which decides that said coding region A is a "false coding region" if the value of said reciprocal is less than a fixed value [herein m is an integer greater than or equal to 5 and less than or equal to 20].

**22.** The method of determining a genetic structure according to Claim 20, which comprises the steps (s) to (u) described below:

(s) obtaining $y_i$ from the following formula (6):

$$y_i = \sum_{t=1}^{T} C_i^t \Bigg/ \sum_{t=1}^{T} \sum_{j=1}^{64} C_j^t \qquad (6)$$

wherein the number of times of the i-th codon appearing in the t-th coding region is expressed as $C_j^t$

(t) rearranging the 64 types of codon in descending order of $y_i$, selecting "top m codons for which the value of $y_i$ is large" and "bottom m codons for which the value of $y_i$ is large, excluding the translation stop codon", and obtaining the value of $Cd_A$ for the coding region A which is assumed to be the coding region from the following formula (7):

$$Cd_A = 2 \times \sum_{i=1}^{m} C_i^A \Bigg/ \left(\sum_{i=1}^{m} C_i^A + \sum_{i=62-m}^{61} C_i^A\right) \qquad (7)$$

[herein the value of $Cd_A$ is defined as 1 if

$$\left(\sum_{i=1}^{m} C_i^A + \sum_{i=62-m}^{61} C_i^A\right)$$

is zero];

(u) deciding that said coding region A is a true coding region if the value of $Cd_A$ for said coding region A which is calculated in the step (t) is greater than or equal to a threshold value CV, and deciding that it is a false coding region if said value of $Cd_A$ is less than the threshold value CV [herein T is an integer greater than or equal to 2; i is a positive integer less than or equal to 64; j is a positive integer less than or equal to 64; t is a positive integer less than or equal to T, m is an integer from 5 to 20; and CV is a value from 0.8 to 1.8].

**23.** A method of determining a genetic structure, which comprises the steps (v) and (w) described below if a coding region of the prokaryote or a coding region A which is assumed to be a coding region overlaps with a coding region B which is assumed to be a coding region and present upon the complementary strand, and if said coding region B is included in said coding region A:

(v) comparing the length $L_B$ (in base pairs) of said coding region B with the length $L_A$ (in base pairs) of said coding region A, and deciding that said coding region B is a "false coding region" if $L_B$ is less than or equal to $T_P$% of $L_A$;

(w) deciding on the truth or falsity of said coding region A and of said coding region B by the method of determining a genetic structure according to any one of Claim 18 to Claim 22 if $L_B$ exceeds $T_P$% of $L_A$ [herein TP is a positive integer from 30 to 95].

**24.** A method of determining a genetic structure, **characterized by** removing the translation stop codons from the coding regions which form a transcription unit, and linking up the resulting coding regions into a single coding region, before utilizing the method of determining a genetic structure according to any one of Claim 18 to Claim 23.

**25.** A program for executing the following steps on a computer:

(a) finding a translation stop codon in the nucleotide sequence of a prokaryote from the information of said nucleotide sequence inputted via an input device, searching for a provisional translation start codon which yields the longest open reading frame (ORF) for all the obtained translation stop codons to make a candidate for ORF which is the combination of the said translation stop codon and provisional translation start codon, and storing the position of these codons in said nucleotide sequence in a memory;
(b) calling up from the memory two adjacent candidates for ORF which are present upon the same strand, investigating the positions of the provisional translation start codon of the downstream side ORF (termed ORF-A) and of the translation stop codon of the upstream side ORF (termed ORF-B) and the distance between the ORF-A and the ORF-B; and deciding that the two adjacent ORFs have a possibility to form a single transcription unit if the provisional translation start codon of the ORF-A is upstream of the translation stop codon of the ORF-B, or is within $D_S$ bases downstream of said translation stop codon [herein $D_S$ is an integer from 20 to 100], and proceeding to the step (c); or deciding that the two adjacent ORFs do not form a single transcription unit if the distance between the positions of the provisional translation start codon of the ORF-A and of the translation stop codon of the ORF-B does not satisfy the above described condition, and proceeding to the step (f);
(c) calling up the above described nucleotide sequence data for the two ORFs which are decided to have a possibility to form a single transcription unit in the step (b), and searching for a candidate for the translation start codon of the ORF-A from a region (hereinafter termed the "vicinity of the translation stop codon") between $D_B$ bases downstream from the first T (thymidine) residue of the translation stop codon of the ORF-B and $U_B$ bases upstream from said T residue [here $D_B$ is an integer between 10 and 20, and $U_B$ is an integer between 3 and 15]; and
determining that the ORF-A whose translation start codon is said candidate is a true coding region if there is a single candidate for the translation start codon, determining that said ORF-A and ORF-B form a single transcription unit, and writing the results of this determination into the memory; or
selecting the candidate whose priority is the highest if there is a plurality of candidates for the translation start codon, wherein the distance between each candidate and the translation stop codon of the ORF-B is used as an indicator of priority, determining that the ORF-A whose translation start codon is said candidate is a true coding region, and determining that said ORF-A and ORF-B constitute a single transcription unit, and writing the results of the determination into the memory;
(d) calling up the above described nucleotide sequence data if the translation start codon of the ORF-A can not be determined in the step (c), examining whether a candidate for the translation start codon of the ORF-A is present within a region (hereinafter termed the "coding region around the vicinity of the translation stop codon") between $R_D$ bases downstream from the first T residue of the translation stop codon of the ORF-B and $R_U$ bases upstream from said T residue [here $R_D$ is an integer from 30 to 120, and $R_U$ is an integer from 20 to 120] and excluding the "vicinity of the translation stop codon"; and
proceeding to the step (e) if a candidate for the translation start codon of the ORF-A is present in said region, or proceeding to the step (f) if no such candidate is present;
(e) calling up the above described nucleotide sequence data for a candidate for the translation start codon of the ORF-A found in the step (d), examining whether a ribosome binding site is present from 1 to 30 bases upstream of each candidate, and determining its ribosome binding sequence if such a ribosome binding site is present, or determining that the ORF-A, whose translation start codon is the candidate which corresponds to said ribosome binding sequence, is a true coding region, determining that said ORF-A and ORF-B form a single transcription unit, and writing the results of the determination into the memory;
(f) calling up the above described nucleotide sequence data for an ORF-A which is not decided to form a single transcription unit in the step (b) or for an ORF-A whose translation start codon can not be determined in the step (e), searching for up to the number N of candidates [here N is an integer from 5 to 20] for the translation start codon, including the provisional start codon which yields the longest ORF, from the 5' terminal, examining whether a ribosome binding site is present from 1 to 30 bases upstream of each candidate, determining its ribosome binding sequence if such a ribosome binding site is present, determining that the ORF-A whose translation start codon is the candidate corresponding to said ribosome binding sequence is a true coding region, and writing the results of the determination into the memory;
(g) repeating the above steps until all of the ORFs stored in the memory are processed;
outputting, via an output device, the results of determination of transcription units and coding regions in step (c), (e) or (f), which have been stored in the memory.

**26.** The program according to Claim 25, wherein the above described step (e) is:

calling up the above described nucleotide sequence data; calculating a "ribosome binding score" which express the paired state between a mRNA sequence of 4 to 17 bases upstream of a candidate for the translation start codon of the ORF-A and a sequence (3'-UUCCUCC-5') involved in the binding to mRNA in a 16S rRNA 3' terminal sequence, or between a mRNA sequence of 4 to 16 bases upstream of said candidate and a sequence (3'-UCCUCC-5') involved in the binding to mRNA within a 16S rRNA 3' terminal sequence as a numerical value according to the four rules described below:

(1) A pairing of G and C yields +4;
(2) A pairing of A and U yields +2;
(3) A pairing of G and U yields +1;
(4) When no pairing is present at a base pair which is adjacent to a base pair where a pairing is present, then this yields -1;

maintaining a threshold value $V_3$ [herein $V_3$ is an integer from 4 to 12] for said ribosome binding score, determining that the above described mRNA sequence whose ribosome binding score exceeds a threshold value V3 is a ribosome binding sequence, and selecting the translation start codon which corresponds to said ribosome binding sequence as the translation start codon of the ORF-A;
dividing the "region around the vicinity of the translation stop codon of the ORF-B" into the two "the region downstream of said vicinity" and "the region upstream of said vicinity" if there is a plurality of said translation start codons for the ORF-A, and selecting the candidate whose priority is highest, wherein the order of priority is the first "the region downstream of said vicinity" and the second "the region upstream of said vicinity";
selecting the candidate whose priority is highest if a plurality of translation start codons is present within the respective regions, wherein the distance from the translation stop codon of the ORF-B is used as an indicator of priority; and
determining that the ORF-A whose translation start codon is the selected candidate is a true coding region, determining that said ORF-A and ORF-B form a single transcription unit, and writing the results of the determination into the memory.

**27.** The program according to Claim 25 or Claim 26, wherein the above described step (f) is:

calling up the above described nucleotide sequence data;
calculating a "ribosome binding score" which express the paired state between a mRNA sequence of 4 to 17 bases upstream of a candidate for the translation start codon of the ORF-A and a sequence (3'-UUCCUCC-5') involved in the binding to mRNA in a 16S rRNA 3' terminal sequence, or between a mRNA sequence of 4 to 16 bases upstream of said candidate and a sequence (3'-UCCUCC-5') involved in the binding to mRNA in a 16S rRNA 3' terminal sequence as a numerical value, according to the four rules described below:

(1) A pairing of G and C yields +4;
(2) A pairing of A and U yields +2;
(3) A pairing of G and U yields +1;
(4) When no pairing is present at a base pair which is adjacent to a base pair where a pairing is present, then this yields -1;

maintaining a threshold value $V_1$ for said ribosome binding score, determining that the above described mRNA sequence which exceeds the threshold value $V_1$ is the ribosome binding sequence, and selecting a candidate for the translation start codon which corresponds to said ribosome binding sequence as the translation start codon of the ORF-A; selecting the translation start codon corresponding to the ribosome binding sequence which yields the highest score as the translation start codon of ORF-A if there is a plurality of said translation start codons;
setting one or more threshold value(s) which is smaller than $V_1$ and include the threshold value $V_3$ in a stepwise manner if there is no candidate which exceeds the threshold value $V_1$, searching for the above described mRNA sequence whose score exceedes said threshold value in a stepwise manner, determining the ribosome binding sequence, and selecting the translation start codon which corresponds to said ribosome binding sequence as the translation start codon of the ORF-A; and
determining that the ORF-A whose translation start codon is the selected candidate is a true coding region, and writing the results of the determination into the memory [herein $V_1$ is an integer which is greater than the $V_3$ of Claim 2, and which is between 7 and 14].

**28.** The program according to Claim 26 or Claim 27, **characterized in that** the above described "ribosome binding score" is calculated by deducting a numerical value $P_G$ if the translation start codon is GTG, and by deducting a numerical value $P_T$ if the translation start codon is TTG [herein, $P_G$ is an integer from 1 to 4, and $P_T$ is an integer from 2 to 6].

**29.** A program for executing the following steps on a computer: calling up the data for transcription units and coding regions stored in the memory after the above described step (g) in the program according to Claim 25 to Claim 28;

(h) deciding that the transcription unit Q or the coding region B is a "false transcription unit" or a "false coding region" if a transcription unit Q or a coding region B which is present upon the same strand as a transcription unit P or a coding region A is included in the transcription unit P or the coding region A;
(i) deciding that the transcription unit Q or the coding region B is a "false transcription unit" or a "false coding region" if a transcription unit Q or a coding region B which is present upon the complementary strand to a transcription unit P or a coding region A is included in the transcription unit P or the coding region A;
(j) deciding that the transcription unit or coding region whose length is shorter is a "false transcription unit" or a "false coding region" if a transcription unit P or a coding region A overlaps with a transcription unit Q or a coding region B which is present upon the complementary strand; and outputting the results of the above described decision via an output device.

**30.** A program for executing the following steps on a computer:

(k) investigating the type of the codons and the number thereof, which are utilized in a plurality (T) of the coding regions of the prokaryote which regions are determined and inputted via an input means, selecting k types of combination of codons among them wherein "the frequency of appearance of one codon is high, and the frequency of appearance of a codon which has the complementary sequence of the 3-base sequence of said codon is low", and storing the codons in the memory;
(l) measuring the frequency of appearance of the selected codons in a coding region A which is assumed to be the coding region from the data of said coding region A inputted via an input means, comparing the "number of times of the k types of codons whose frequency of appearance is high appearing in a coding region A which is assumed to be a coding region" with the "number of times of the k types of codons whose frequency of appearance is low appearing in said coding region A", and deciding on the truth or falsity of said coding region A [herein k is an integer greater than or equal to 5 and less than or equal to 20]; and

displaying the results of the above described decision via an output device.

**31.** The program according to Claim 30, wherein the step (1) is comparing the "number of times of the k types of codons whose frequency of appearance is high appearing in a coding region A which is assumed to be the coding region" and the "number of times of the k types of codons whose frequency of appearance is low appearing in said coding region A" by using "the reciprocal of the sum of 1 and the ratio of the number of the latter to the number of the former" as a calculation formula, and deciding that said coding region A is a "false coding region" if the value of said reciprocal is less than a fixed value.

**32.** The program for executing the following steps on a computer according to Claim 30:

(m) constructing a codon table by arranging the 64 types of codons so that the 3-base sequence of the i-th codon has the complementary sequence to the nucleotide sequence of the (i+32)-th codon, and storing the codon table in the memory;
(n) inputting the nucleotide sequence of the number T of determined coding regions of a prokaryote, and obtaining yi from the formula (2) below and yi+32 from the formula (3) below:

$$y_i = \left(\sum_{t=1}^{T} C_i^t - \sum_{t=1}^{T} C_{i+32}^t\right) \Big/ \sum_{t=1}^{T}\sum_{j=1}^{64} C_j^t \qquad (2)$$

$$y_{i+32} = \left(\sum_{t=1}^{T} C_{i+32}^t - \sum_{t=1}^{T} C_i^t\right) \Big/ \sum_{t=1}^{T}\sum_{j=1}^{64} C_j^t \qquad (3)$$

wherein the number of times the i-th codon appear in the t-th coding region is expressed as $C_j^t$

(o) calling up the codon table which was obtained in the step (m) from the memory, setting up a correspondence between the $y_i$ and $y_{i+32}$ for the codons in the table, rearranging the sequence of the codons in the table in descending order of the $y_i$ and the $y_{i+32}$, selecting top k codons for which the value of $y_i$ or of $y_{i+32}$ is large, and obtaining the value of $Sd_A$ for a coding region A by the following formula (4):

$$Sd_A = 2 \times \sum_{i=1}^{k} C_i^A \Bigg/ \left(\sum_{i=1}^{k} C_i^A + \sum_{i=65-k}^{64} C_i^A\right) \qquad (4)$$

[herein the value of $Sd_A$ is defined as 1 if

$$\left(\sum_{i=1}^{k} C_i^A + \sum_{i=65-k}^{64} C_i^A\right)$$

is zero];

(p) deciding that said coding region is a true coding region if the value of $Sd_A$ of a coding region A obtained in the above described step is greater than or equal to a threshold value $S_1$, and deciding that it is a false coding region if said value of $Sd_A$ is less than the threshold value $S_1$ [herein T is an integer greater than or equal to 2, i is a positive integer less than or equal to 32, j is a positive integer less than or equal to 64, t is a positive integer less than or equal to T, k is an integer from 5 to 20, and $S_1$ is a value from 0.8 to 1.8].

**33.** A program for executing the following steps on a computer:

examining whether there is mutual overlapping and inclusion between coding regions of a prokaryote which are inputted via an input device:

(q) calling up the above described nucleotide sequence data if a coding region or a coding region A which is assumed to be a coding region overlaps with a coding region B which is assumed to be a coding region and present upon the complementary strand, and if said coding region B is included in said coding region A, comparing the length $L_B$ (in base pairs) of said coding region B with the length $L_A$ (in base pairs) of said coding region A, and deciding that said coding region B is a "false coding region" if $L_B$ is less than or equal to $T_P$% of $L_A$;

(r) deciding on the truth or falsity of said coding region A and of said coding region B by the steps of the program according to any one of Claim 30 to Claim 32 if $L_B$ exceeds $T_P$% of $L_A$ [herein $T_P$ is a positive integer from 30 to 95].

**34.** The program according to Claim 33, **characterized by** rewriting the data for the determined coding regions to a single coding region constructed by removing the translation stop codons from the coding regions which form a transcription unit and by linking up the resulting coding regions from said data, before executing the steps (k) and (l) described above.

**35.** A program for deciding on the truth or falsity of a coding region or of a transcription unit which is determined and stored in the memory in any one of Claim 25 to Claim 35, by the steps of the program according to any one of Claim 30 to Claim 34.

**36.** A program for executing the steps:

calling up the data for coding regions which is determined as true coding regions by the steps of the program according to any one of Claim 25 to Claim 35 from the memory,

calculating the length of the polypeptide encoded by each coding region, and deciding on the truth or falsity of the coding regions which encode the polypeptides of $L_M$ amino acids or more in length, by using the program according to any one of Claim 7 to Claim 12, based upon the nucleotide sequences of the coding regions encoding the polypeptide of $L_F$ amino acids or more in length [herein $L_F$ is a positive integer greater than or equal to 100, and $L_M$ is a positive integer greater than or equal to 20].

**37.** A program for executing the following steps on a computer:

calculating the content of the first and third G residues and C residues of the codons in a coding region of a prokaryote whose GC content exceeds 50% by using a predetermined calculation formula from the data for said coding region inputted via an input device; deciding that said coding region is a "false coding region" if the calculated content is less than a fixed value; and
outputting the results of the decision via an output device.

**38.** The program according to Claim 37, wherein the following formula (5) is used as a calculation formula, the value of $GC_i$ described below is used as a calculated content, and one value which is selected from 0.6 to 0.75 is used as a fixed value:

$$GC_i = (y^{i(1)} + y^{i(3)}) \Big/ \sum_{r=1}^{3} y^{i(r)} \quad \text{wherein} \quad y^{i(r)} = \sum_{n=1}^{N_i} \sum_{b=1}^{4} x_{n(b)}^{i(r)} \qquad (5)$$

[herein when the r-th base (r is 1, 2, or 3) of the n-th codon of the i-th coding region is b (b is 1, 2, 3, or 4), then

$$\chi_{n|b|}^{i|r|} \text{ is } \chi_{n|b|}^{i|r|} = 1 \text{ (b= 1 or 2)}$$

$$\chi_{n|b|}^{i|r|} = 0 \text{ (b= 3 or 4)}$$

and, as for b, when the r-th base of the n-th codon of the i-th coding region is G, C, A, or T, then b is 1, 2, 3, or 4, respectively, i and n are positive integers, and $N_i$ denotes the total number of the codons (excluding the translation stop codon) of the i-th coding region].

**39.** A program for executing the following steps on a computer:

calculating the content of the first and third G residues and C residues of the codons of the 5' terminal region of a coding region of a prokaryote whose GC content exceeds 50% by using a predetermined calculation formula, from the data for said coding region inputted via an input device; deciding that the translation start codon of said coding region is a "false translation start codon" if the calculated content is less than a fixed value, and
outputting the results of this decision via an output device;
calling up the nucleotide sequence data of the above described coding region which is inputted via an input device, and re-searching for a translation start codon which is present downstream of said translation start codon decided to be false.

**40.** The program according to Claim 39, wherein the following formula (5) is used as an calculation formula, the value of $GC_i$ described below is used as a calculated content and one value selected from 0.6 to 0.75 is used as a fixed value:

$$GC_i = (y^{i(1)} + y^{i(3)}) \Big/ \sum_{r=1}^{3} y^{i(r)} \quad \text{wherein} \quad y^{i(r)} = \sum_{n=1}^{N_i} \sum_{b=1}^{4} x_{n(b)}^{i(r)} \qquad (5)$$

[herein, when the r-th base (r is 1, 2, or 3) of the n-th codon of the i-th coding region is b (b is 1, 2, 3, or 4), then

$$\chi_{n|b|}^{i|r|} \text{ is } \chi_{n|b|}^{i|r|} = 1 \text{ (b= 1 or 2)}$$

$$\chi_{n|b|}^{i|r|} = 0 \text{ (b= 3 or 4)}$$

and, as for b, when the r-th base of the n-th codon of the i-th coding region is G, C, A, or T, b is 1, 2, 3, or 4, respectively, i and n are positive integers, and $N_i$ denotes the total number of the codons (excluding the translation stop codon) of the i-th coding region].

**41.** A program for executing the following steps on a computer:

selecting m types of codons whose frequency of appearance is high and m types of codons whose frequency of appearance is low in the number T of the coding regions of a prokaryote which are determined by the steps of the program according to any one of Claims 25 to 40, and storing the codons in the memory;
measuring the "number of times of the m types of codons whose frequency of appearance in the number T of the coding regions is high appearing in the coding region A which is assumed to be the coding region" and the "number of times of the m types of codons whose frequency of appearance in the T coding regions is low appearing in said coding region A" from the data for coding regions which are not determined, different from the number T of the coding regions and inputted; deciding on the truth or the falsity of said coding region A by comparing both numbers; and outputting the results of the decision via an output device. [herein T is an integer greater than or equal to 2, and m is an integer greater than or equal to 5 and less than or equal to 20].

**42.** The program according to Claim 41, wherein the method of comparing the "number of times of the m types of codons whose frequency of appearance is high appearing in the coding region A which is assumed to be the coding region" with the "number of times of the m types of codons whose frequency of appearance is low appearing in said coding region A" is the method which utilizes the "reciprocal of the sum of 1 and the ratio of the number of the latter to the number of the former" as a calculation formula, and which decides that said coding region A is a "false coding region" if the value of said reciprocal is less than a fixed value [herein m is an integer greater than or equal to 5 and less than or equal to 20].

**43.** The program for executing the following steps on a computer according to Claim 41:

(m) constructing a codon table in which the 64 types of codons are arranged so that the 3-base sequence of the i-th codon has a complementary sequence to the nucleotide sequence of the (i+32)-th codon, and storing the codon table in the memory;
(s) obtaining $y_i$ by the following formula (6):

$$y_i = \sum_{t=1}^{T} C_i^t \Big/ \sum_{t=1}^{T} \sum_{j=1}^{64} C_j^t \qquad (6)$$

wherein the number of times of the i-th codon appearing in the t-th coding region is expressed as $C_j^t$
(t) calling up the codon table from the memory, rearranging the 64 types of codon in descending order of $y_i$, selecting "top m codons for which the value of $y_i$ is large" and "bottom m codons for which the value of $y_i$ is large, excluding the translation stop codon", and obtaining the value of $Cd_A$ for the coding region A which is assumed to be the coding region from the following formula (7):

$$Cd_A = 2 \times \sum_{i=1}^{m} C_i^A \Big/ \left(\sum_{i=1}^{m} C_i^A + \sum_{i=62-m}^{61} C_i^A\right) \qquad (7)$$

[herein the value of $Cd_A$ is defined as 1 if

$$(\sum_{i=1}^{m} C_i^A + \sum_{i=62-m}^{61} C_i^A)$$

is zero, ] and

(u) deciding said coding region A to be a true coding region if the value of $Cd_A$ for said coding region A which is calculated by the step (t) is greater than or equal to a threshold value CV, deciding said coding region A to be a false coding region if said value of $Cd_A$ is less than the threshold value CV, and outputting the decision results via an output device [herein T is an integer greater than or equal to 2; i is a positive integer less than or equal to 64; j is a positive integer less than or equal to 64; t is a positive integer less than or equal to T, m is an integer from 5 to 20; and CV is a value from 0.8 to 1.8].

**44.** A program for executing the following steps on a computer, wherein a coding region of the prokaryote or a coding region A which is assumed to be a coding region overlaps with a coding region B which is assumed to be a coding region and present upon the complementary strand, and said coding region B is included in said coding region A:

(v) comparing the length $L_B$ (in base pairs) of the coding region B with the length $L_A$ (in base pairs) of the coding region A, and deciding that the coding region B is a "false coding region" if $L_B$ is less than or equal to $T_P$% of $L_A$;

(w) deciding on the truth or falsity of said coding region A and of said coding region B by the method of determining a genetic structure according to any one of Claim 41 to Claim 43 if $L_B$ exceeds $T_P$% of $L_A$, [herein, $T_P$ is a positive integer from 30 to 95]; and

outputting the results of the decision via an output device.

**45.** A program executing the following steps:

removing translation stop codons from the coding regions which form a transcription unit from the data for determined coding regions; linking up the resulting coding regions into a single coding region; and rewriting the data for determined coding regions to the resulting single coding region; and executing the steps of the program according to any one of Claim 41 to Claim 44.

**46.** A computer-readable recording medium on which the program according to any one of Claim 25 to Claim 45 is recorded.

**47.** A system for determining a genetic structure which comprises:

(i) an input means for inputting nucleotide sequence data;

(ii) a means for executing the program according to any one of Claim 25 to Claim 45, using the inputted data; and

(iii) an output device for outputting the results which is obtained by (ii).

FIG. 1

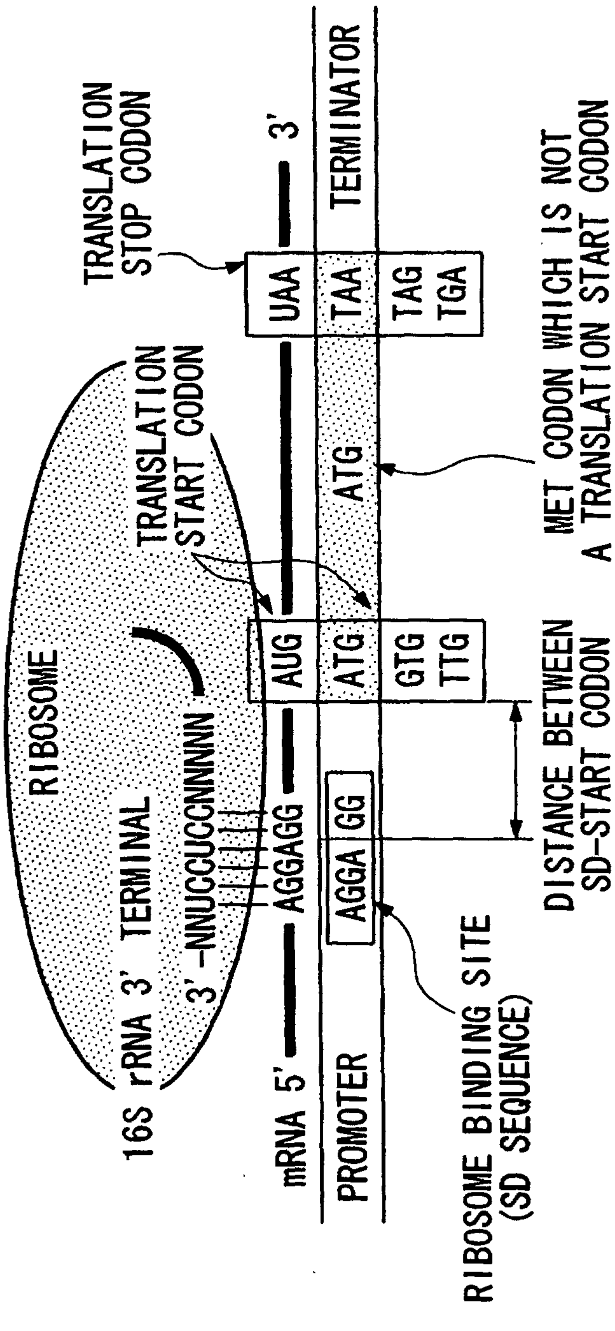
16S rRNA 3' TERMINAL
RIBOSOME
3'-NNUCCUCCNNNNN
mRNA 5'
AGGAGG
AUG
UAA
3'
PROMOTER
AGGA GG
ATG
ATG
TAA
TERMINATOR
GTG
TTG
TAG
TGA
TRANSLATION START CODON
TRANSLATION STOP CODON
RIBOSOME BINDING SITE (SD SEQUENCE)
DISTANCE BETWEEN SD-START CODON
MET CODON WHICH IS NOT A TRANSLATION START CODON

FIG. 2

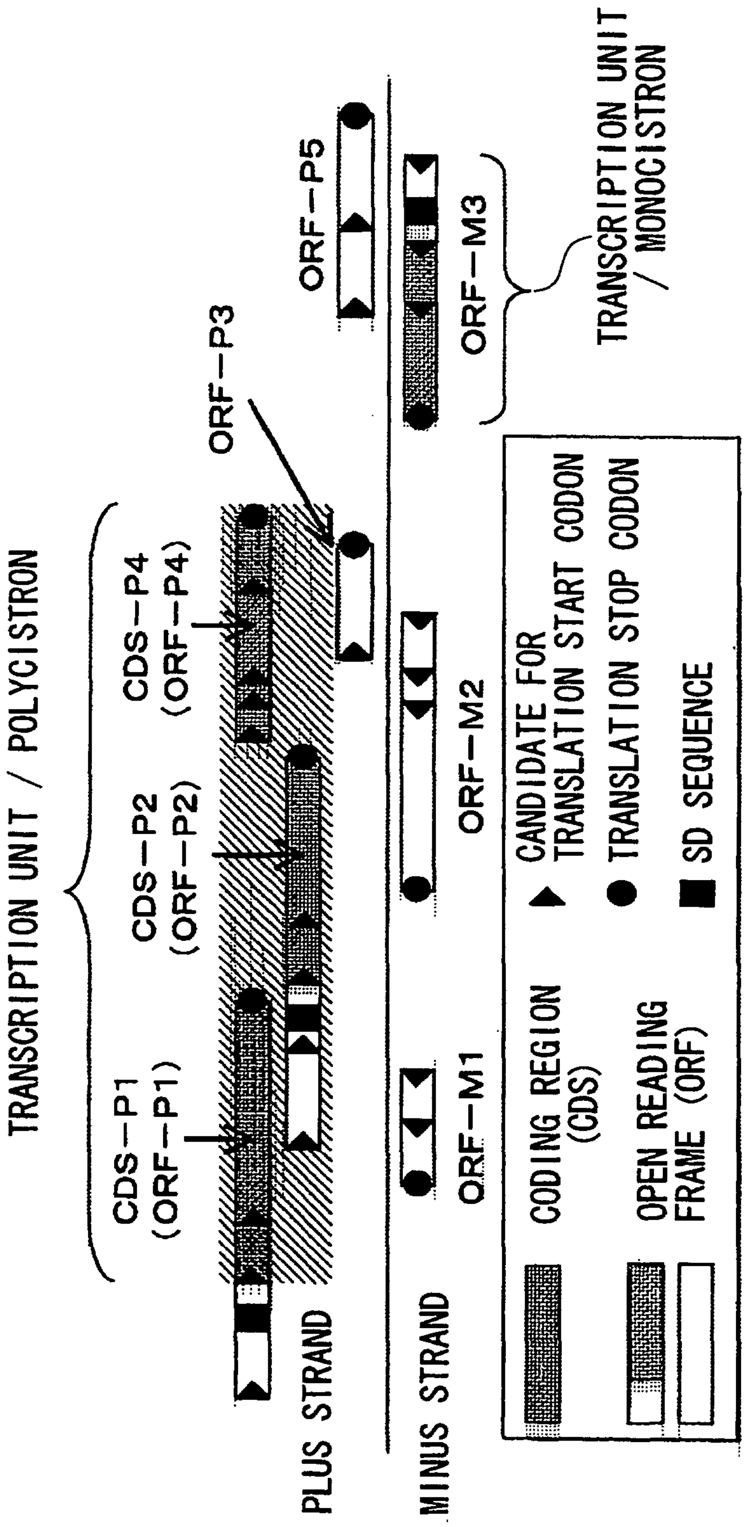

TRANSCRIPTION UNIT / POLYCISTRON
CDS—P1
(ORF—P1)
CDS—P2
(ORF—P2)
CDS—P4
(ORF—P4)
ORF—P3
ORF—P5
PLUS STRAND
MINUS STRAND
ORF—M1
ORF—M2
ORF—M3
TRANSCRIPTION UNIT
/ MONOCISTRON
CODING REGION
(CDS)
OPEN READING
FRAME (ORF)
CANDIDATE FOR
TRANSLATION START CODON
TRANSLATION STOP CODON
SD SEQUENCE

FIG. 3A

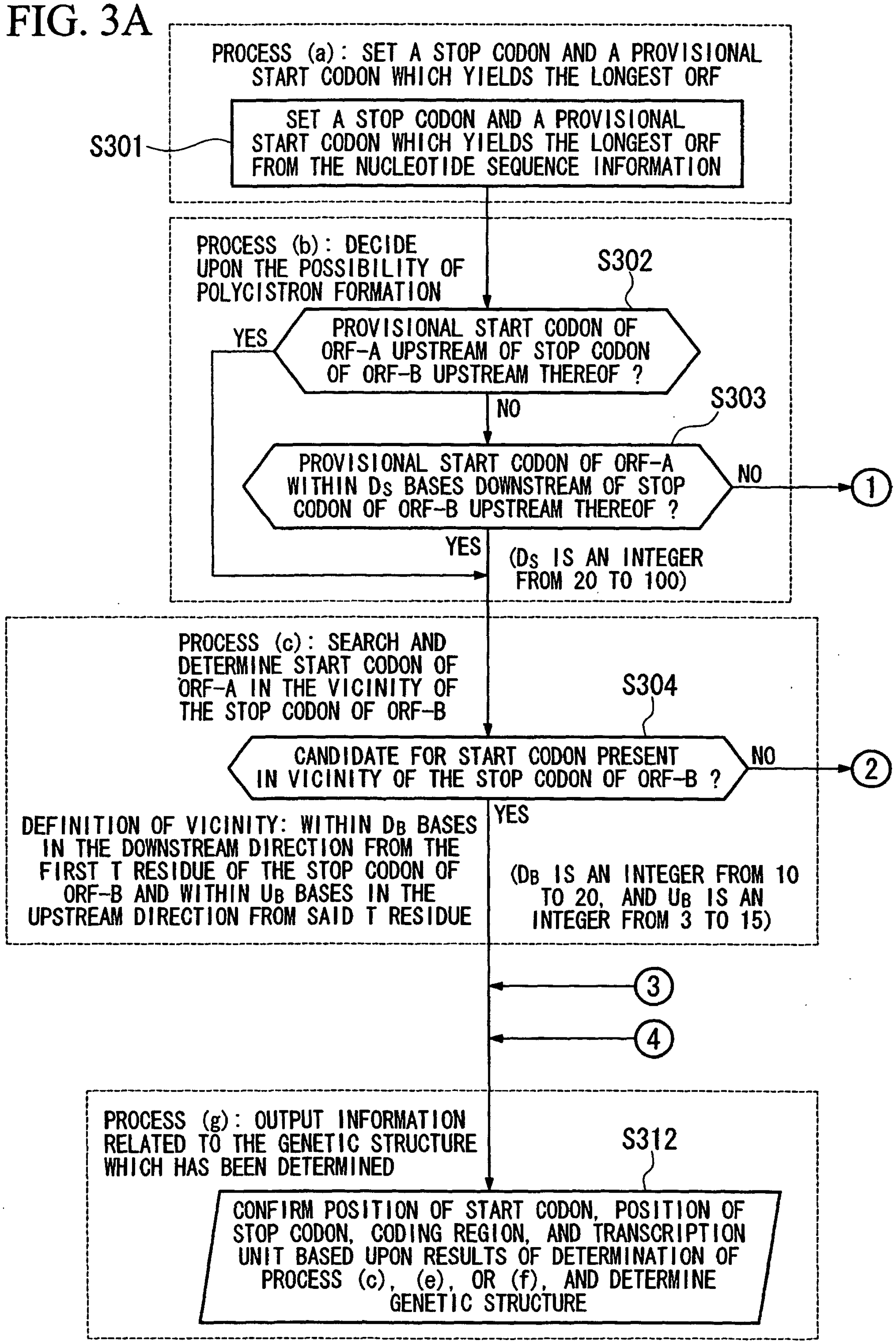
PROCESS (a): SET A STOP CODON AND A PROVISIONAL START CODON WHICH YIELDS THE LONGEST ORF
S301
SET A STOP CODON AND A PROVISIONAL START CODON WHICH YIELDS THE LONGEST ORF FROM THE NUCLEOTIDE SEQUENCE INFORMATION
PROCESS (b): DECIDE UPON THE POSSIBILITY OF POLYCISTRON FORMATION
S302
PROVISIONAL START CODON OF ORF-A UPSTREAM OF STOP CODON OF ORF-B UPSTREAM THEREOF ?
YES
NO
S303
PROVISIONAL START CODON OF ORF-A WITHIN Ds BASES DOWNSTREAM OF STOP CODON OF ORF-B UPSTREAM THEREOF ?
NO
1
YES
(Ds IS AN INTEGER FROM 20 TO 100)
PROCESS (c): SEARCH AND DETERMINE START CODON OF ORF-A IN THE VICINITY OF THE STOP CODON OF ORF-B
S304
CANDIDATE FOR START CODON PRESENT IN VICINITY OF THE STOP CODON OF ORF-B ?
NO
2
YES
DEFINITION OF VICINITY: WITHIN DB BASES IN THE DOWNSTREAM DIRECTION FROM THE FIRST T RESIDUE OF THE STOP CODON OF ORF-B AND WITHIN UB BASES IN THE UPSTREAM DIRECTION FROM SAID T RESIDUE
(DB IS AN INTEGER FROM 10 TO 20, AND UB IS AN INTEGER FROM 3 TO 15)
3
4
PROCESS (g): OUTPUT INFORMATION RELATED TO THE GENETIC STRUCTURE WHICH HAS BEEN DETERMINED
S312
CONFIRM POSITION OF START CODON, POSITION OF STOP CODON, CODING REGION, AND TRANSCRIPTION UNIT BASED UPON RESULTS OF DETERMINATION OF PROCESS (c), (e), OR (f), AND DETERMINE GENETIC STRUCTURE

FIG. 3B

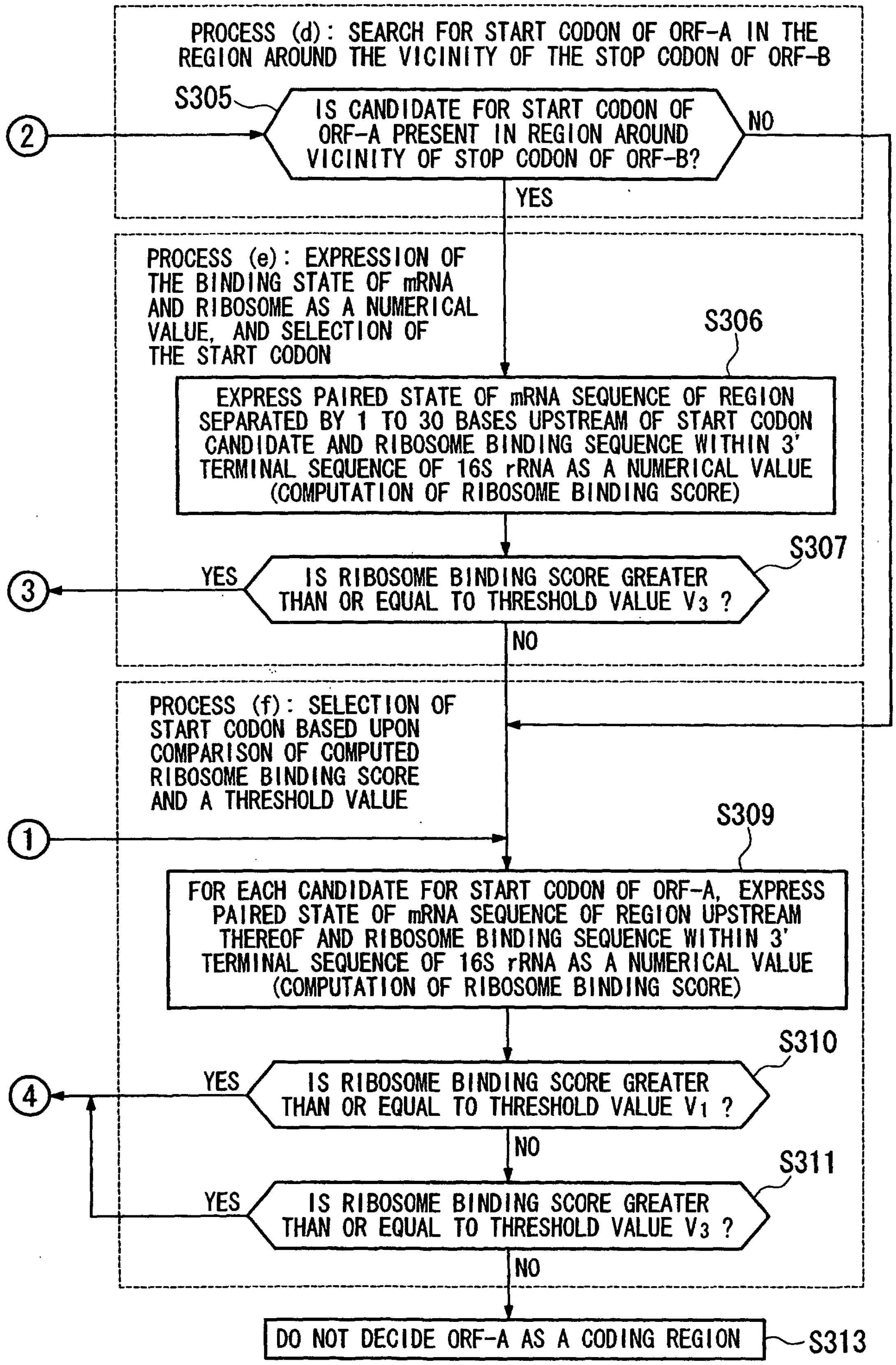
PROCESS (d): SEARCH FOR START CODON OF ORF-A IN THE REGION AROUND THE VICINITY OF THE STOP CODON OF ORF-B
S305
IS CANDIDATE FOR START CODON OF ORF-A PRESENT IN REGION AROUND VICINITY OF STOP CODON OF ORF-B?
2
NO
YES
PROCESS (e): EXPRESSION OF THE BINDING STATE OF mRNA AND RIBOSOME AS A NUMERICAL VALUE, AND SELECTION OF THE START CODON
S306
EXPRESS PAIRED STATE OF mRNA SEQUENCE OF REGION SEPARATED BY 1 TO 30 BASES UPSTREAM OF START CODON CANDIDATE AND RIBOSOME BINDING SEQUENCE WITHIN 3' TERMINAL SEQUENCE OF 16S rRNA AS A NUMERICAL VALUE (COMPUTATION OF RIBOSOME BINDING SCORE)
S307
3
YES
IS RIBOSOME BINDING SCORE GREATER THAN OR EQUAL TO THRESHOLD VALUE V3 ?
NO
PROCESS (f): SELECTION OF START CODON BASED UPON COMPARISON OF COMPUTED RIBOSOME BINDING SCORE AND A THRESHOLD VALUE
1
S309
FOR EACH CANDIDATE FOR START CODON OF ORF-A, EXPRESS PAIRED STATE OF mRNA SEQUENCE OF REGION UPSTREAM THEREOF AND RIBOSOME BINDING SEQUENCE WITHIN 3' TERMINAL SEQUENCE OF 16S rRNA AS A NUMERICAL VALUE (COMPUTATION OF RIBOSOME BINDING SCORE)
S310
4
YES
IS RIBOSOME BINDING SCORE GREATER THAN OR EQUAL TO THRESHOLD VALUE V1 ?
NO
S311
YES
IS RIBOSOME BINDING SCORE GREATER THAN OR EQUAL TO THRESHOLD VALUE V3 ?
NO
DO NOT DECIDE ORF-A AS A CODING REGION
S313

FIG. 4

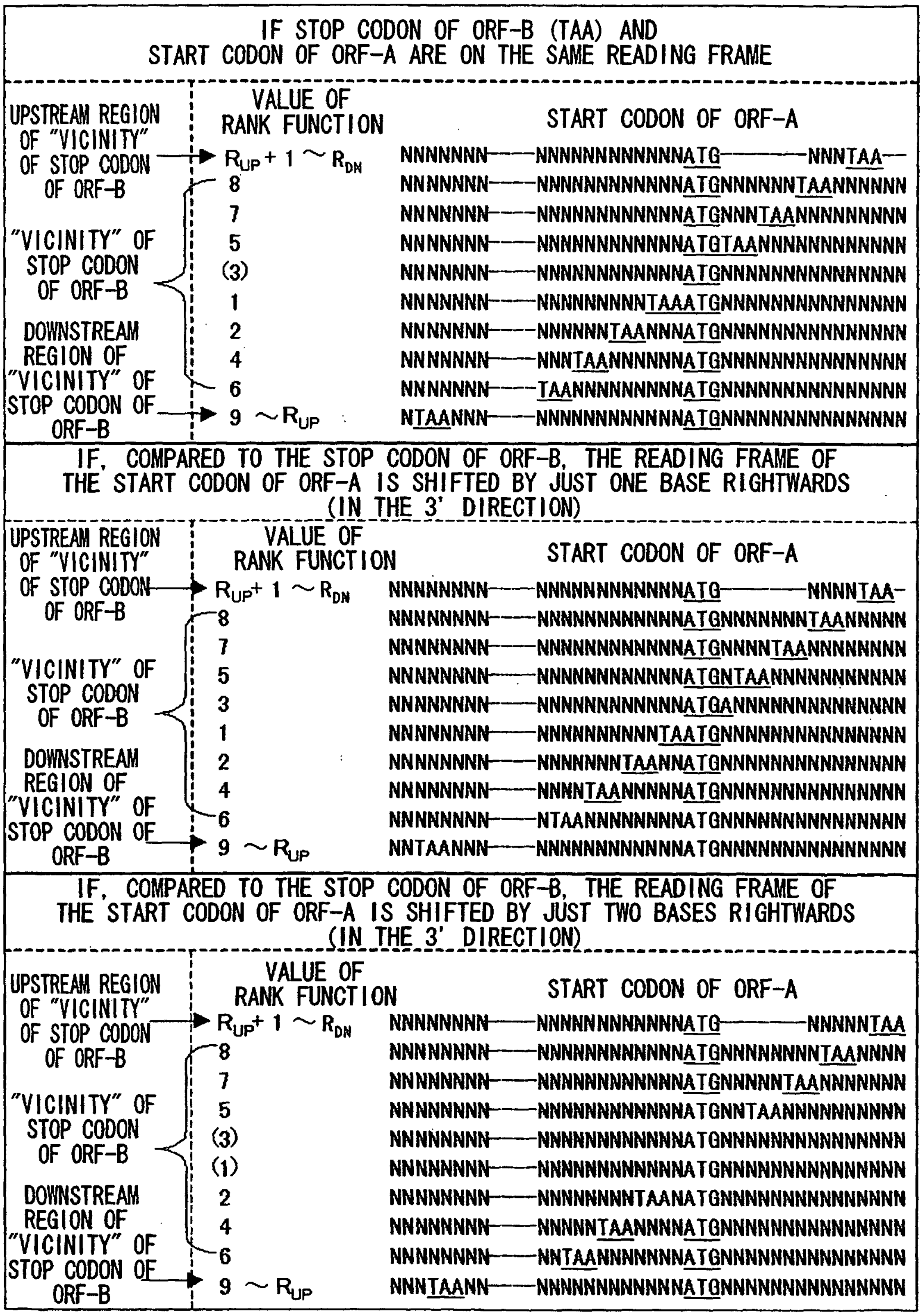
IF STOP CODON OF ORF-B (TAA) AND START CODON OF ORF-A ARE ON THE SAME READING FRAME
UPSTREAM REGION OF "VICINITY" OF STOP CODON OF ORF-B
"VICINITY" OF STOP CODON OF ORF-B
DOWNSTREAM REGION OF "VICINITY" OF STOP CODON OF ORF-B
VALUE OF RANK FUNCTION
START CODON OF ORF-A
RUP + 1 ~ RDN
NNNNNNN—NNNNNNNNNNNNATG—NNNTAA—
8
NNNNNNN—NNNNNNNNNNNNATGNNNNNNTAANNNNNN
7
NNNNNNN—NNNNNNNNNNNNATGNNNTAANNNNNNNNN
5
NNNNNNN—NNNNNNNNNNNNATGTAANNNNNNNNNNNN
(3)
NNNNNNN—NNNNNNNNNNNNATGNNNNNNNNNNNNNNN
1
NNNNNNN—NNNNNNNNNTAAATGNNNNNNNNNNNNNNN
2
NNNNNNN—NNNNNNTAANNNATGNNNNNNNNNNNNNNN
4
NNNNNNN—NNNTAANNNNNNATGNNNNNNNNNNNNNNN
6
NNNNNNN—TAANNNNNNNNNATGNNNNNNNNNNNNNNN
9 ~ RUP
NTAANNN—NNNNNNNNNNNNATGNNNNNNNNNNNNNNN
IF, COMPARED TO THE STOP CODON OF ORF-B, THE READING FRAME OF THE START CODON OF ORF-A IS SHIFTED BY JUST ONE BASE RIGHTWARDS (IN THE 3' DIRECTION)
UPSTREAM REGION OF "VICINITY" OF STOP CODON OF ORF-B
"VICINITY" OF STOP CODON OF ORF-B
DOWNSTREAM REGION OF "VICINITY" OF STOP CODON OF ORF-B
VALUE OF RANK FUNCTION
START CODON OF ORF-A
RUP+ 1 ~ RDN
NNNNNNNN—NNNNNNNNNNNNATG—NNNNTAA-
8
NNNNNNNN—NNNNNNNNNNNNATGNNNNNNNTAANNNNN
7
NNNNNNNN—NNNNNNNNNNNNATGNNNNTAANNNNNNNN
5
NNNNNNNN—NNNNNNNNNNNNATGNTAANNNNNNNNNNN
3
NNNNNNNN—NNNNNNNNNNNNATGANNNNNNNNNNNNNN
1
NNNNNNNN—NNNNNNNNNNTAATGNNNNNNNNNNNNNNN
2
NNNNNNNN—NNNNNNNTAANNATGNNNNNNNNNNNNNNN
4
NNNNNNNN—NNNNTAANNNNNNATGNNNNNNNNNNNNNNN
6
NNNNNNNN—NTAANNNNNNNNATGNNNNNNNNNNNNNNN
9 ~ RUP
NNTAANNN—NNNNNNNNNNNNATGNNNNNNNNNNNNNNN
IF, COMPARED TO THE STOP CODON OF ORF-B, THE READING FRAME OF THE START CODON OF ORF-A IS SHIFTED BY JUST TWO BASES RIGHTWARDS (IN THE 3' DIRECTION)
UPSTREAM REGION OF "VICINITY" OF STOP CODON OF ORF-B
"VICINITY" OF STOP CODON OF ORF-B
DOWNSTREAM REGION OF "VICINITY" OF STOP CODON OF ORF-B
VALUE OF RANK FUNCTION
START CODON OF ORF-A
RUP+ 1 ~ RDN
NNNNNNNN—NNNNNNNNNNNNNATG—NNNNNTAA
8
NNNNNNNN—NNNNNNNNNNNNATGNNNNNNNNTAANNNN
7
NNNNNNNN—NNNNNNNNNNNNATGNNNNNTAANNNNNNN
5
NNNNNNNN—NNNNNNNNNNNNATGNNTAANNNNNNNNNN
(3)
NNNNNNNN—NNNNNNNNNNNNATGNNNNNNNNNNNNNNN
(1)
NNNNNNNN—NNNNNNNNNNNNATGNNNNNNNNNNNNNNN
2
NNNNNNNN—NNNNNNNTAANATGNNNNNNNNNNNNNNN
4
NNNNNNNN—NNNNNTAANNNNATGNNNNNNNNNNNNNNN
6
NNNNNNNN—NNTAANNNNNNNNATGNNNNNNNNNNNNNNN
9 ~ RUP
NNNTAANN—NNNNNNNNNNNNATGNNNNNNNNNNNNNNN

FIG. 5

CALCULATION METHOD FOR RIBOSOME BINDING SCORE $S_A$ OF CDS-A

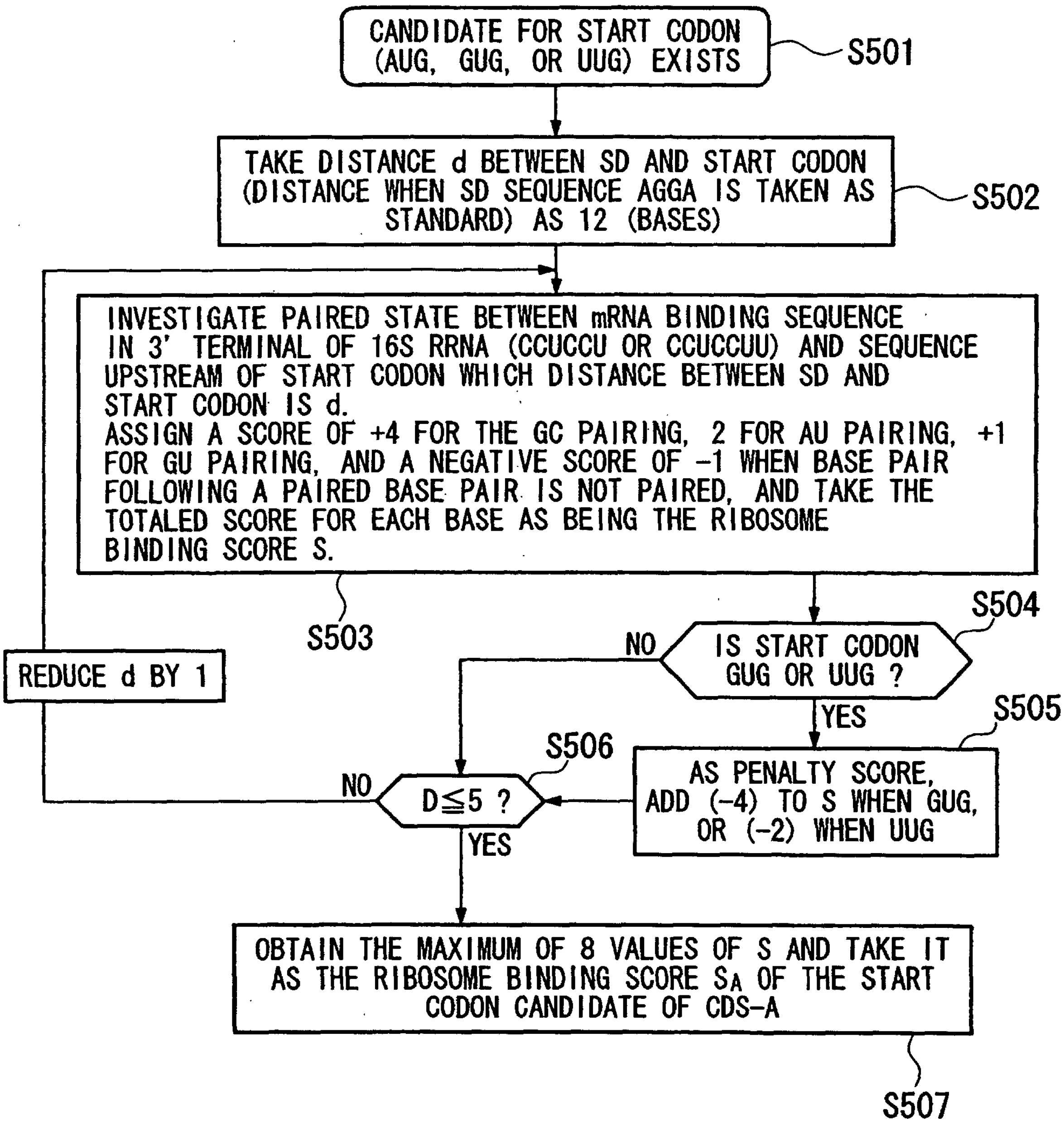

FIG. 6

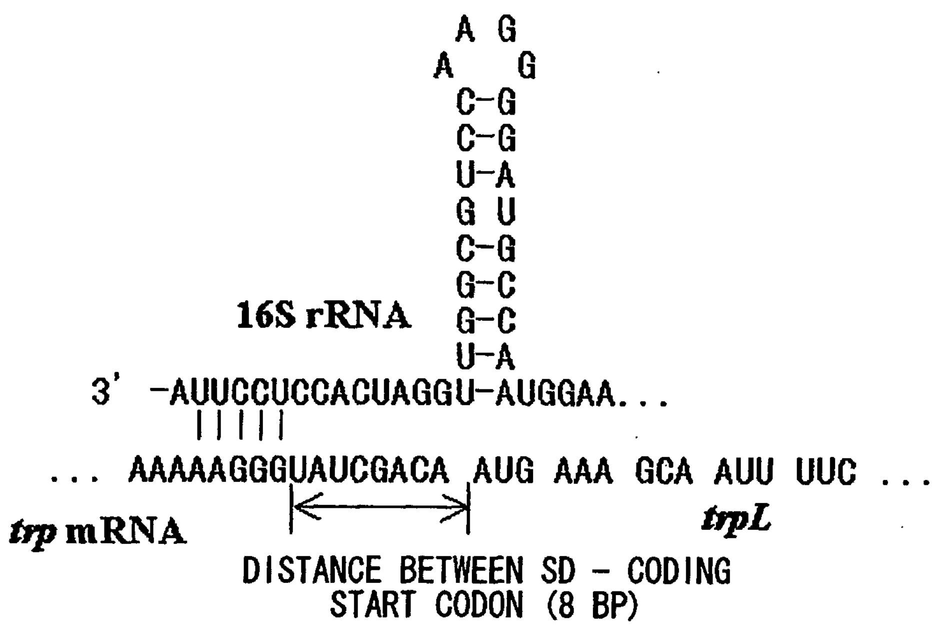
A G
A G
C-G
C-G
U-A
G U
C-G
G-C
G-C
U-A
16S rRNA
3' -AUUCCUCCACUAGGU-AUGGAA...
... AAAAAGGGUAUCGACA AUG AAA GCA AUU UUC ...
trp mRNA
trpL
DISTANCE BETWEEN SD - CODING
START CODON (8 BP)

FIG. 7A

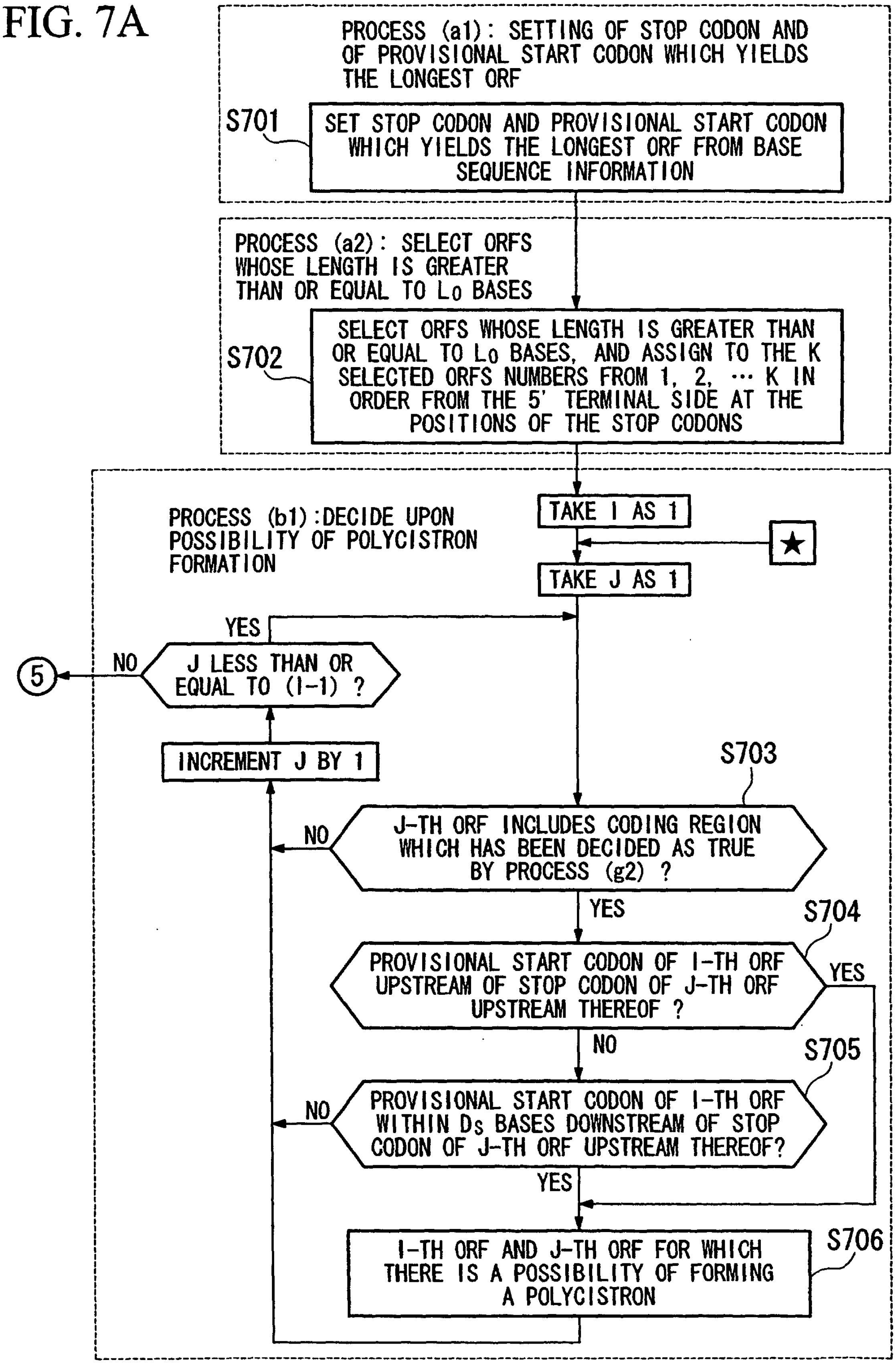
PROCESS (a1): SETTING OF STOP CODON AND OF PROVISIONAL START CODON WHICH YIELDS THE LONGEST ORF
S701
SET STOP CODON AND PROVISIONAL START CODON WHICH YIELDS THE LONGEST ORF FROM BASE SEQUENCE INFORMATION
PROCESS (a2): SELECT ORFS WHOSE LENGTH IS GREATER THAN OR EQUAL TO Lo BASES
S702
SELECT ORFS WHOSE LENGTH IS GREATER THAN OR EQUAL TO Lo BASES, AND ASSIGN TO THE K SELECTED ORFS NUMBERS FROM 1, 2, ··· K IN ORDER FROM THE 5' TERMINAL SIDE AT THE POSITIONS OF THE STOP CODONS
PROCESS (b1):DECIDE UPON POSSIBILITY OF POLYCISTRON FORMATION
TAKE I AS 1
★
TAKE J AS 1
YES
NO
5
J LESS THAN OR EQUAL TO (I-1) ?
INCREMENT J BY 1
S703
NO
J-TH ORF INCLUDES CODING REGION WHICH HAS BEEN DECIDED AS TRUE BY PROCESS (g2) ?
YES
S704
PROVISIONAL START CODON OF I-TH ORF UPSTREAM OF STOP CODON OF J-TH ORF UPSTREAM THEREOF ?
YES
NO
S705
NO
PROVISIONAL START CODON OF I-TH ORF WITHIN Ds BASES DOWNSTREAM OF STOP CODON OF J-TH ORF UPSTREAM THEREOF?
YES
S706
I-TH ORF AND J-TH ORF FOR WHICH THERE IS A POSSIBILITY OF FORMING A POLYCISTRON

FIG. 7B

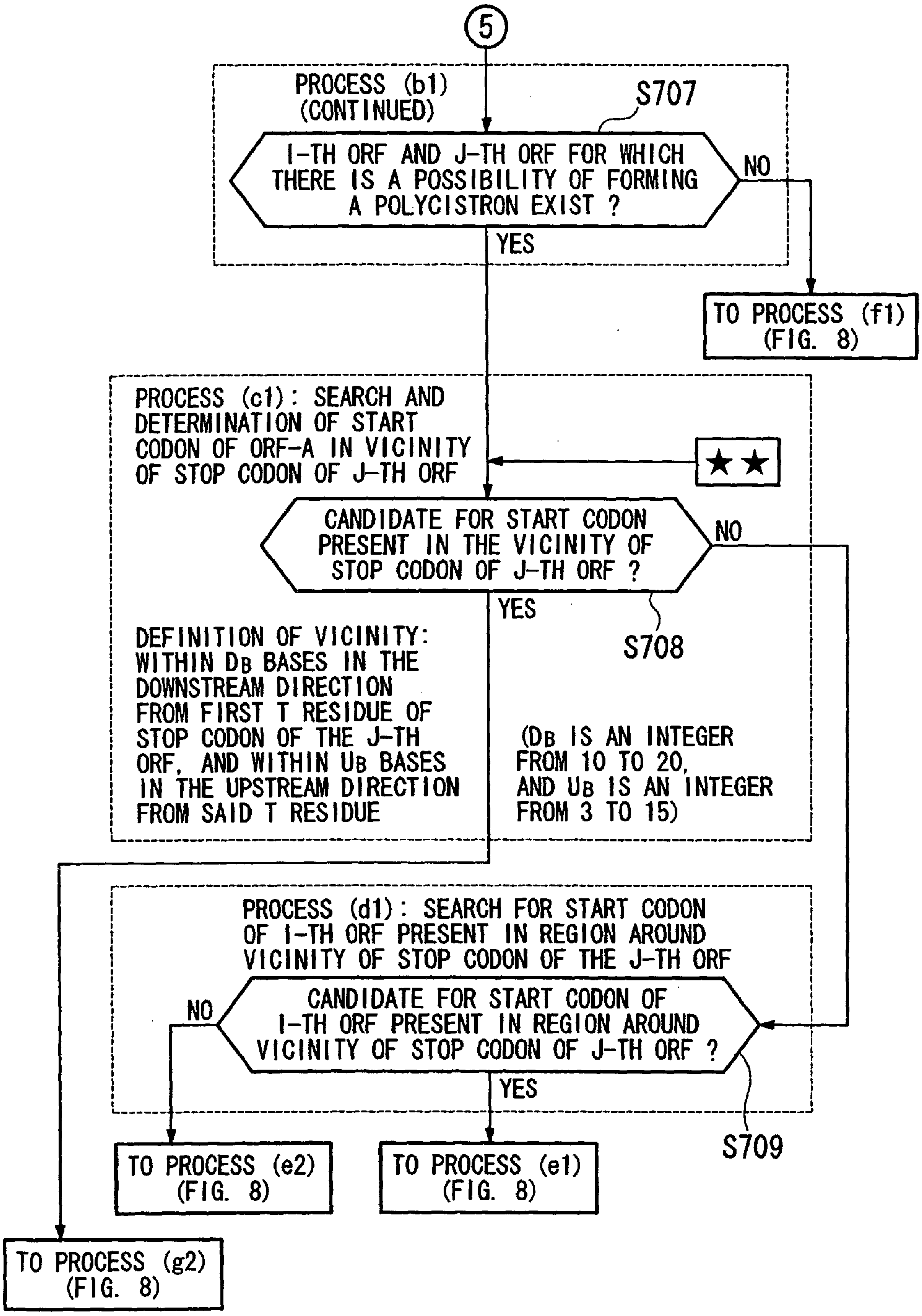
5
PROCESS (b1)
(CONTINUED)
S707
I-TH ORF AND J-TH ORF FOR WHICH THERE IS A POSSIBILITY OF FORMING A POLYCISTRON EXIST ?
NO
YES
TO PROCESS (f1)
(FIG. 8)
PROCESS (c1): SEARCH AND DETERMINATION OF START CODON OF ORF-A IN VICINITY OF STOP CODON OF J-TH ORF
★★
CANDIDATE FOR START CODON PRESENT IN THE VICINITY OF STOP CODON OF J-TH ORF ?
NO
YES
S708
DEFINITION OF VICINITY: WITHIN DB BASES IN THE DOWNSTREAM DIRECTION FROM FIRST T RESIDUE OF STOP CODON OF THE J-TH ORF, AND WITHIN UB BASES IN THE UPSTREAM DIRECTION FROM SAID T RESIDUE
(DB IS AN INTEGER FROM 10 TO 20, AND UB IS AN INTEGER FROM 3 TO 15)
PROCESS (d1): SEARCH FOR START CODON OF I-TH ORF PRESENT IN REGION AROUND VICINITY OF STOP CODON OF THE J-TH ORF
NO
CANDIDATE FOR START CODON OF I-TH ORF PRESENT IN REGION AROUND VICINITY OF STOP CODON OF J-TH ORF ?
YES
S709
TO PROCESS (e2)
(FIG. 8)
TO PROCESS (e1)
(FIG. 8)
TO PROCESS (g2)
(FIG. 8)

FIG. 8A

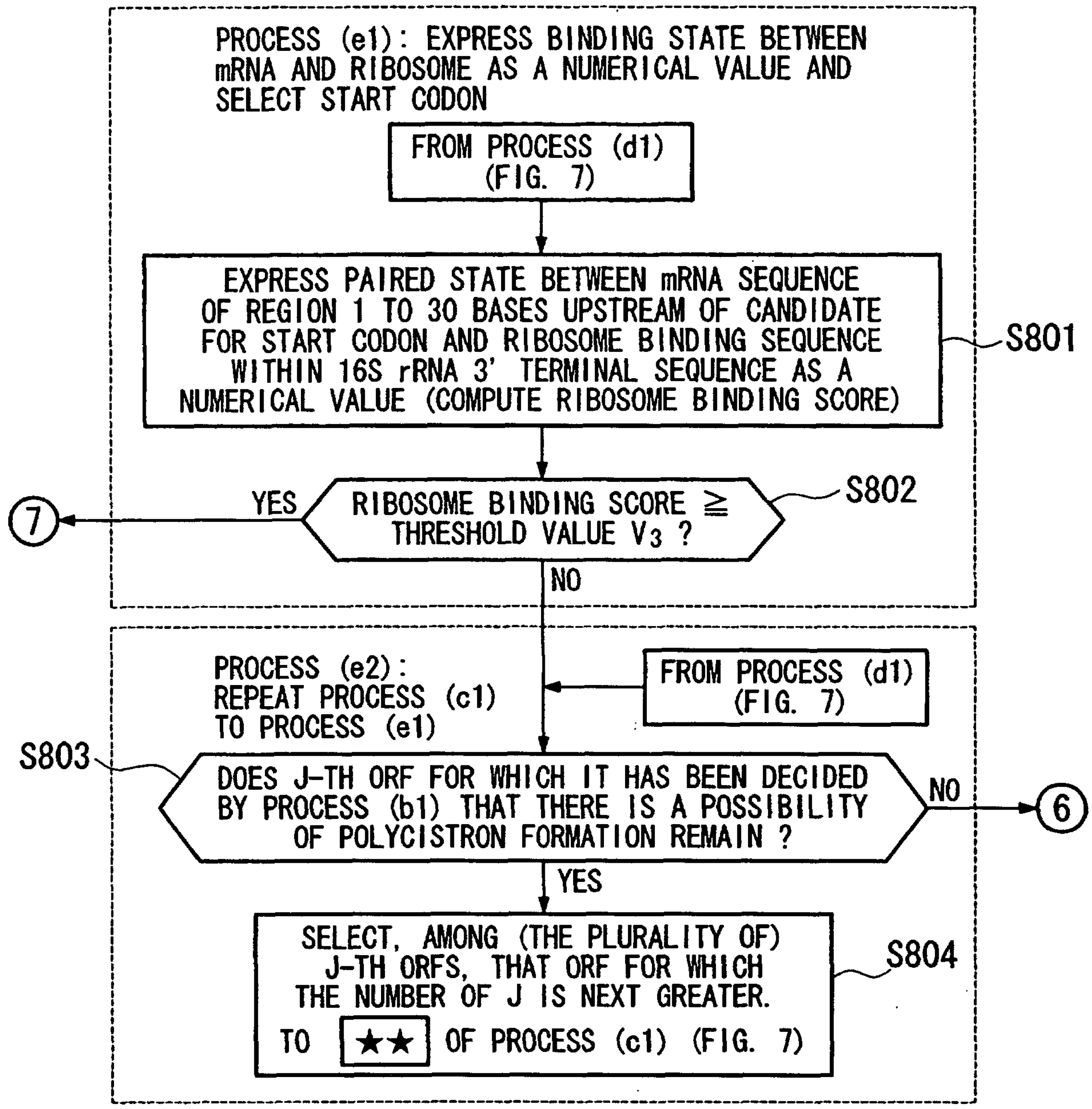
PROCESS (e1): EXPRESS BINDING STATE BETWEEN mRNA AND RIBOSOME AS A NUMERICAL VALUE AND SELECT START CODON
FROM PROCESS (d1) (FIG. 7)
EXPRESS PAIRED STATE BETWEEN mRNA SEQUENCE OF REGION 1 TO 30 BASES UPSTREAM OF CANDIDATE FOR START CODON AND RIBOSOME BINDING SEQUENCE WITHIN 16S rRNA 3' TERMINAL SEQUENCE AS A NUMERICAL VALUE (COMPUTE RIBOSOME BINDING SCORE)
S801
RIBOSOME BINDING SCORE ≧ THRESHOLD VALUE V3 ?
S802
YES
7
NO
PROCESS (e2): REPEAT PROCESS (c1) TO PROCESS (e1)
FROM PROCESS (d1) (FIG. 7)
S803
DOES J-TH ORF FOR WHICH IT HAS BEEN DECIDED BY PROCESS (b1) THAT THERE IS A POSSIBILITY OF POLYCISTRON FORMATION REMAIN ?
NO
6
YES
SELECT, AMONG (THE PLURALITY OF) J-TH ORFS, THAT ORF FOR WHICH THE NUMBER OF J IS NEXT GREATER.
TO ★★ OF PROCESS (c1) (FIG. 7)
S804

FIG. 8B

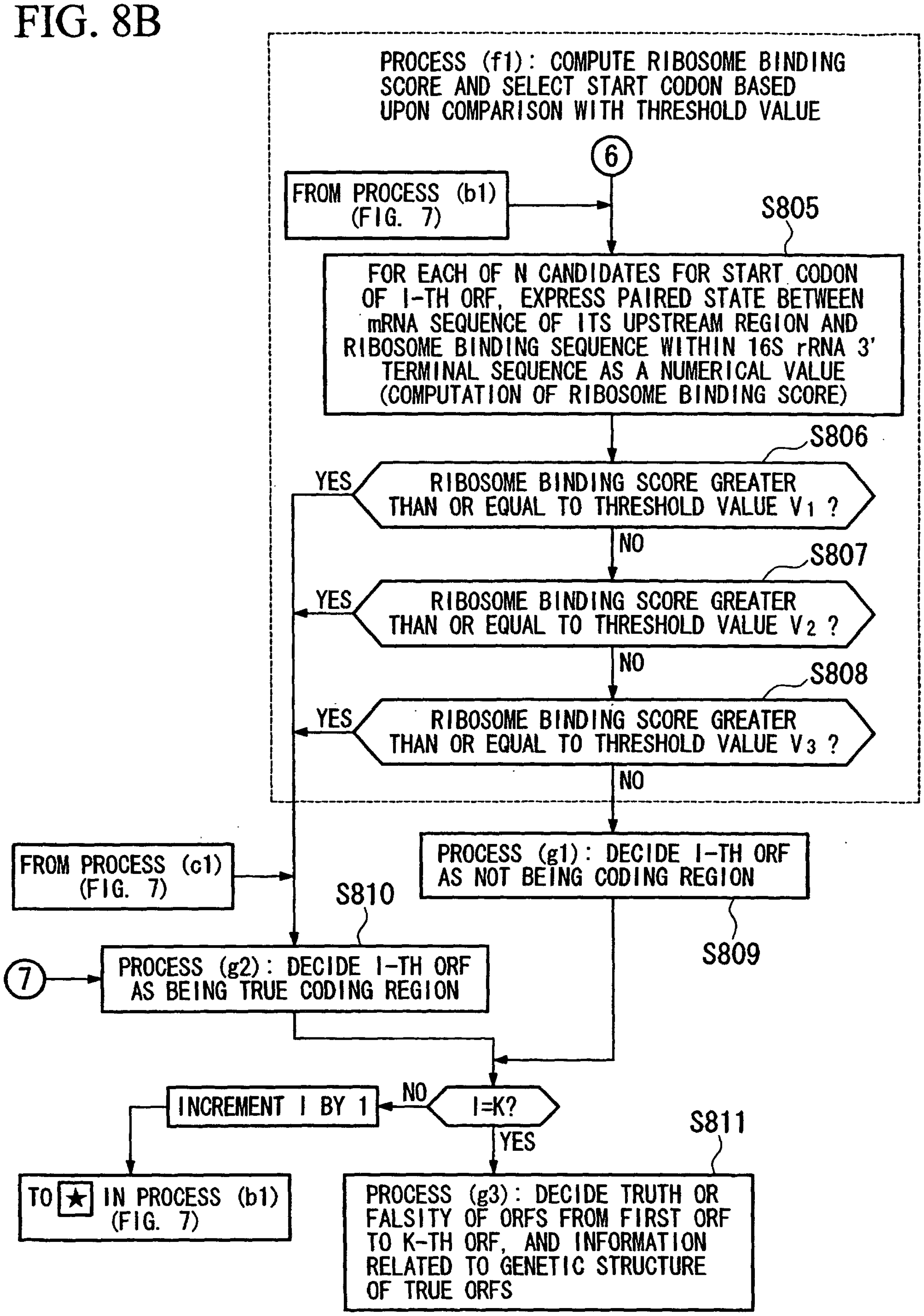
PROCESS (f1): COMPUTE RIBOSOME BINDING SCORE AND SELECT START CODON BASED UPON COMPARISON WITH THRESHOLD VALUE
6
FROM PROCESS (b1) (FIG. 7)
S805
FOR EACH OF N CANDIDATES FOR START CODON OF I-TH ORF, EXPRESS PAIRED STATE BETWEEN mRNA SEQUENCE OF ITS UPSTREAM REGION AND RIBOSOME BINDING SEQUENCE WITHIN 16S rRNA 3' TERMINAL SEQUENCE AS A NUMERICAL VALUE (COMPUTATION OF RIBOSOME BINDING SCORE)
S806
YES
RIBOSOME BINDING SCORE GREATER THAN OR EQUAL TO THRESHOLD VALUE V1 ?
NO
S807
YES
RIBOSOME BINDING SCORE GREATER THAN OR EQUAL TO THRESHOLD VALUE V2 ?
NO
S808
YES
RIBOSOME BINDING SCORE GREATER THAN OR EQUAL TO THRESHOLD VALUE V3 ?
NO
FROM PROCESS (c1) (FIG. 7)
PROCESS (g1): DECIDE I-TH ORF AS NOT BEING CODING REGION
S810
S809
7
PROCESS (g2): DECIDE I-TH ORF AS BEING TRUE CODING REGION
INCREMENT I BY 1
NO
I=K?
YES
S811
TO ★ IN PROCESS (b1) (FIG. 7)
PROCESS (g3): DECIDE TRUTH OR FALSITY OF ORFS FROM FIRST ORF TO K-TH ORF, AND INFORMATION RELATED TO GENETIC STRUCTURE OF TRUE ORFS

FIG. 9A

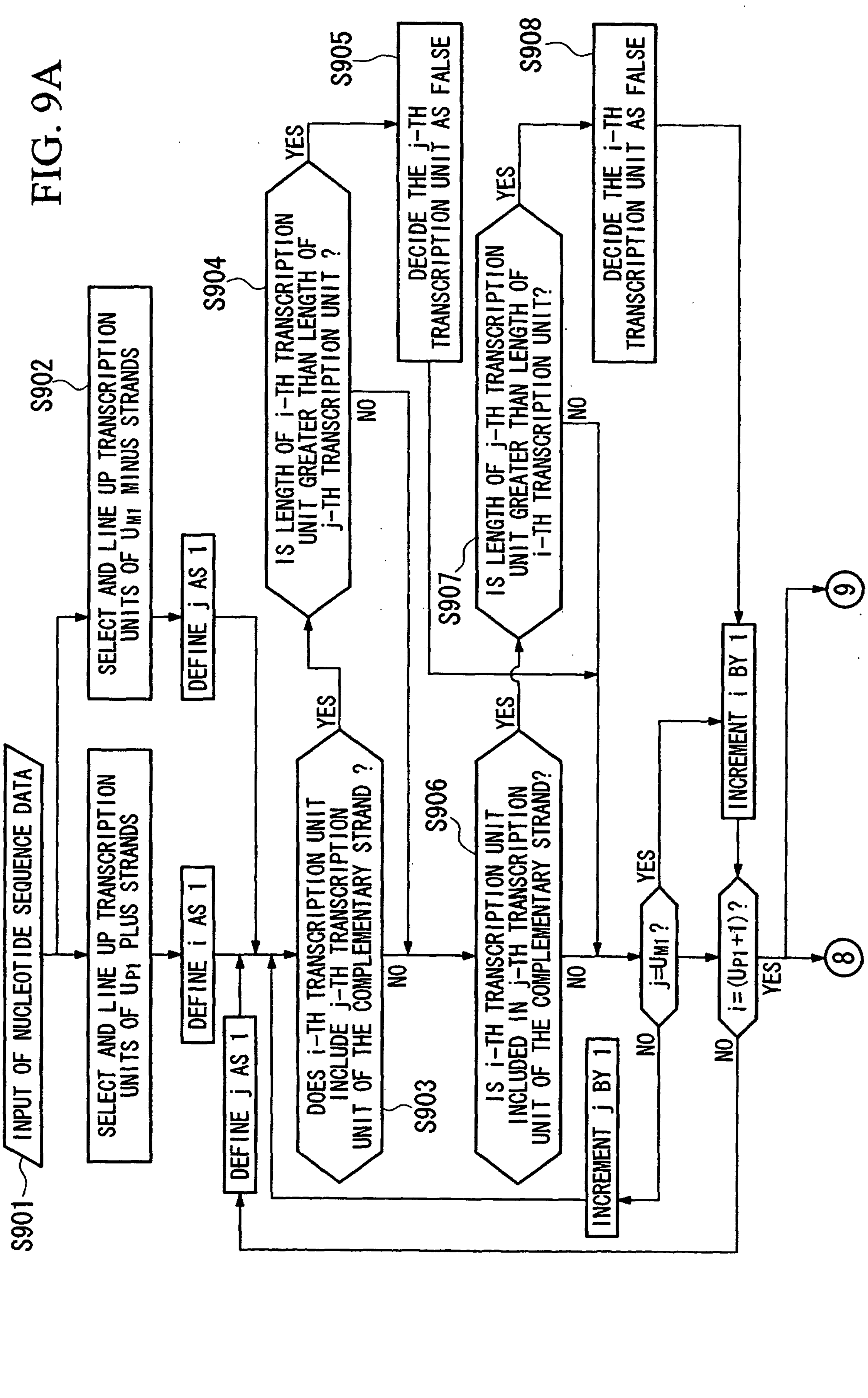
S901
INPUT OF NUCLEOTIDE SEQUENCE DATA
S902
SELECT AND LINE UP TRANSCRIPTION UNITS OF UP1 PLUS STRANDS
SELECT AND LINE UP TRANSCRIPTION UNITS OF UM1 MINUS STRANDS
DEFINE i AS 1
DEFINE j AS 1
DEFINE j AS 1
S903
DOES i-TH TRANSCRIPTION UNIT INCLUDE j-TH TRANSCRIPTION UNIT OF THE COMPLEMENTARY STRAND ?
YES
NO
S904
IS LENGTH OF i-TH TRANSCRIPTION UNIT GREATER THAN LENGTH OF j-TH TRANSCRIPTION UNIT ?
YES
NO
S905
DECIDE THE j-TH TRANSCRIPTION UNIT AS FALSE
S906
IS i-TH TRANSCRIPTION UNIT INCLUDED IN j-TH TRANSCRIPTION UNIT OF THE COMPLEMENTARY STRAND?
YES
NO
S907
IS LENGTH OF j-TH TRANSCRIPTION UNIT GREATER THAN LENGTH OF i-TH TRANSCRIPTION UNIT?
YES
NO
S908
DECIDE THE i-TH TRANSCRIPTION UNIT AS FALSE
j=UM1 ?
YES
NO
INCREMENT j BY 1
INCREMENT i BY 1
i=(UP1+1) ?
YES
NO
8
9

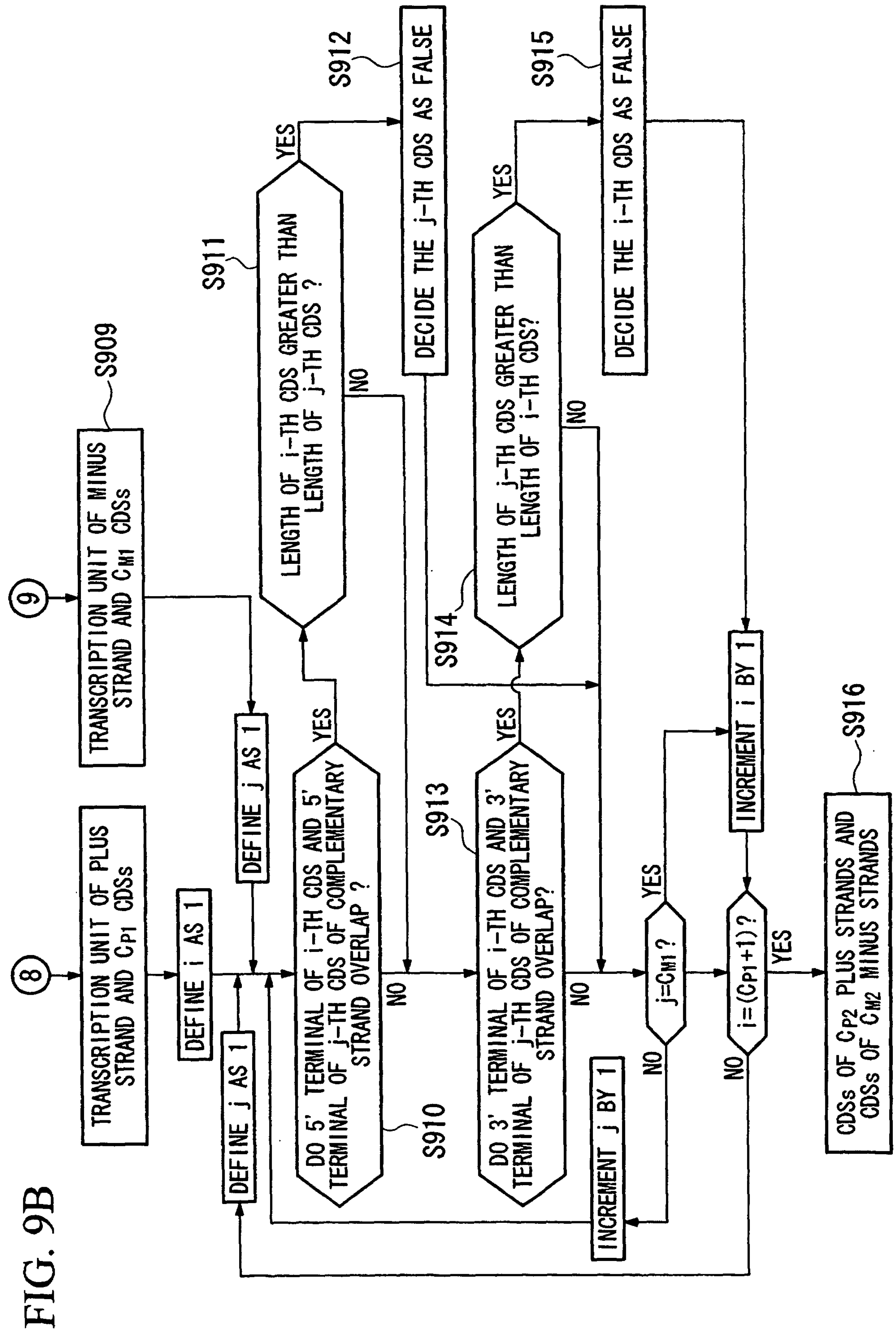

8
TRANSCRIPTION UNIT OF PLUS STRAND AND $C_{P1}$ CDSs
9
TRANSCRIPTION UNIT OF MINUS STRAND AND $C_{M1}$ CDSs
S909
DEFINE i AS 1
DEFINE j AS 1
DEFINE j AS 1
DO 5' TERMINAL OF i-TH CDS AND 5' TERMINAL OF j-TH CDS OF COMPLEMENTARY STRAND OVERLAP ?
S910
YES
NO
LENGTH OF i-TH CDS GREATER THAN LENGTH OF j-TH CDS ?
S911
YES
NO
DECIDE THE j-TH CDS AS FALSE
S912
DO 3' TERMINAL OF i-TH CDS AND 3' TERMINAL OF j-TH CDS OF COMPLEMENTARY STRAND OVERLAP?
S913
YES
NO
LENGTH OF j-TH CDS GREATER THAN LENGTH OF i-TH CDS?
S914
YES
NO
DECIDE THE i-TH CDS AS FALSE
S915
$j=C_{M1}$?
YES
NO
INCREMENT j BY 1
INCREMENT i BY 1
$i=(C_{P1}+1)$?
YES
NO
CDSs OF $C_{P2}$ PLUS STRANDS AND CDSs OF $C_{M2}$ MINUS STRANDS
S916


FIG. 9B

FIG. 10

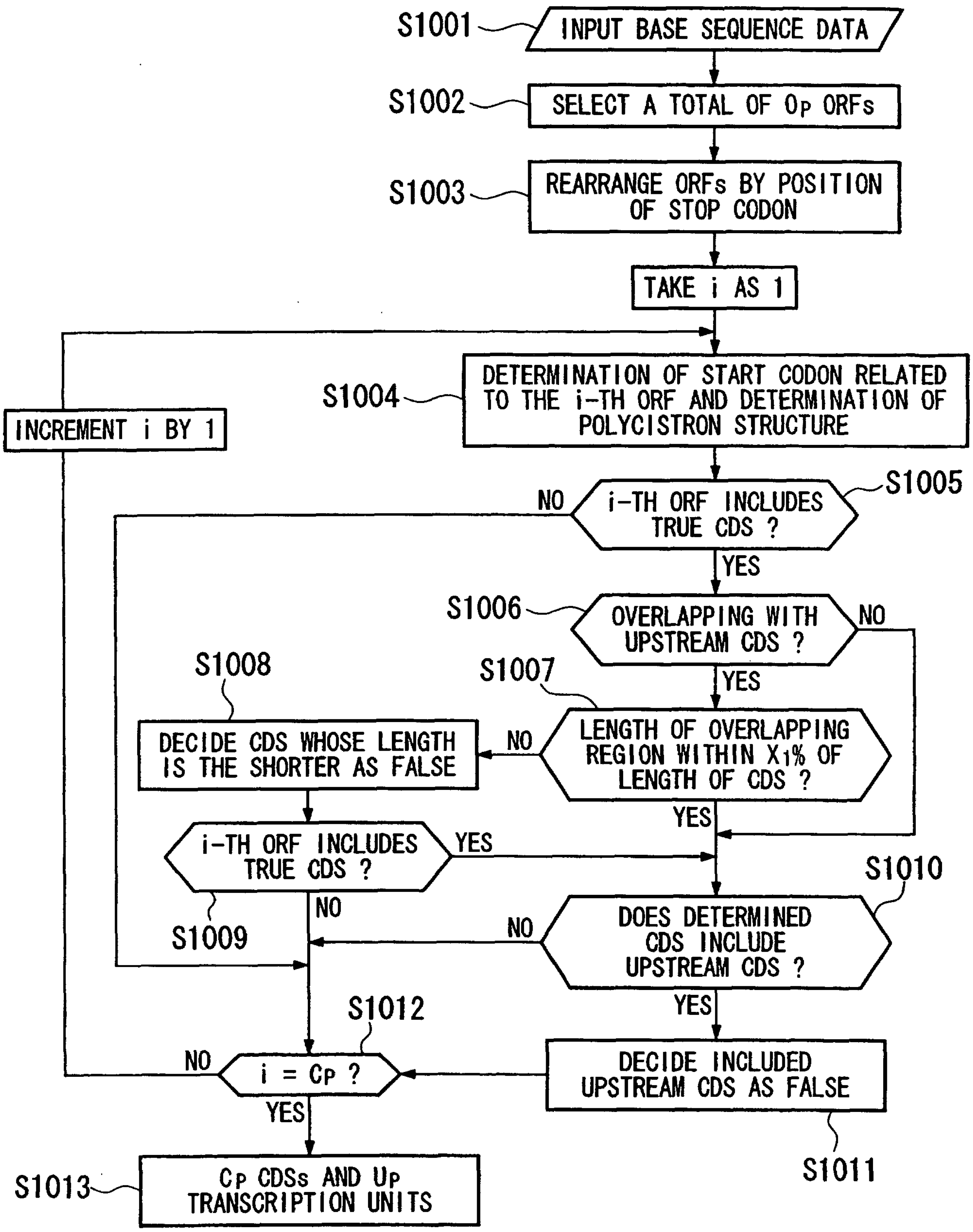
S1001
INPUT BASE SEQUENCE DATA
S1002
SELECT A TOTAL OF Op ORFs
S1003
REARRANGE ORFs BY POSITION OF STOP CODON
TAKE i AS 1
S1004
DETERMINATION OF START CODON RELATED TO THE i-TH ORF AND DETERMINATION OF POLYCISTRON STRUCTURE
INCREMENT i BY 1
S1005
i-TH ORF INCLUDES TRUE CDS ?
NO
YES
S1006
OVERLAPPING WITH UPSTREAM CDS ?
NO
YES
S1007
LENGTH OF OVERLAPPING REGION WITHIN X1% OF LENGTH OF CDS ?
NO
YES
S1008
DECIDE CDS WHOSE LENGTH IS THE SHORTER AS FALSE
S1009
i-TH ORF INCLUDES TRUE CDS ?
YES
NO
S1010
DOES DETERMINED CDS INCLUDE UPSTREAM CDS ?
NO
YES
S1011
DECIDE INCLUDED UPSTREAM CDS AS FALSE
S1012
i = Cp ?
NO
YES
S1013
Cp CDSs AND Up TRANSCRIPTION UNITS

FIG. 11A

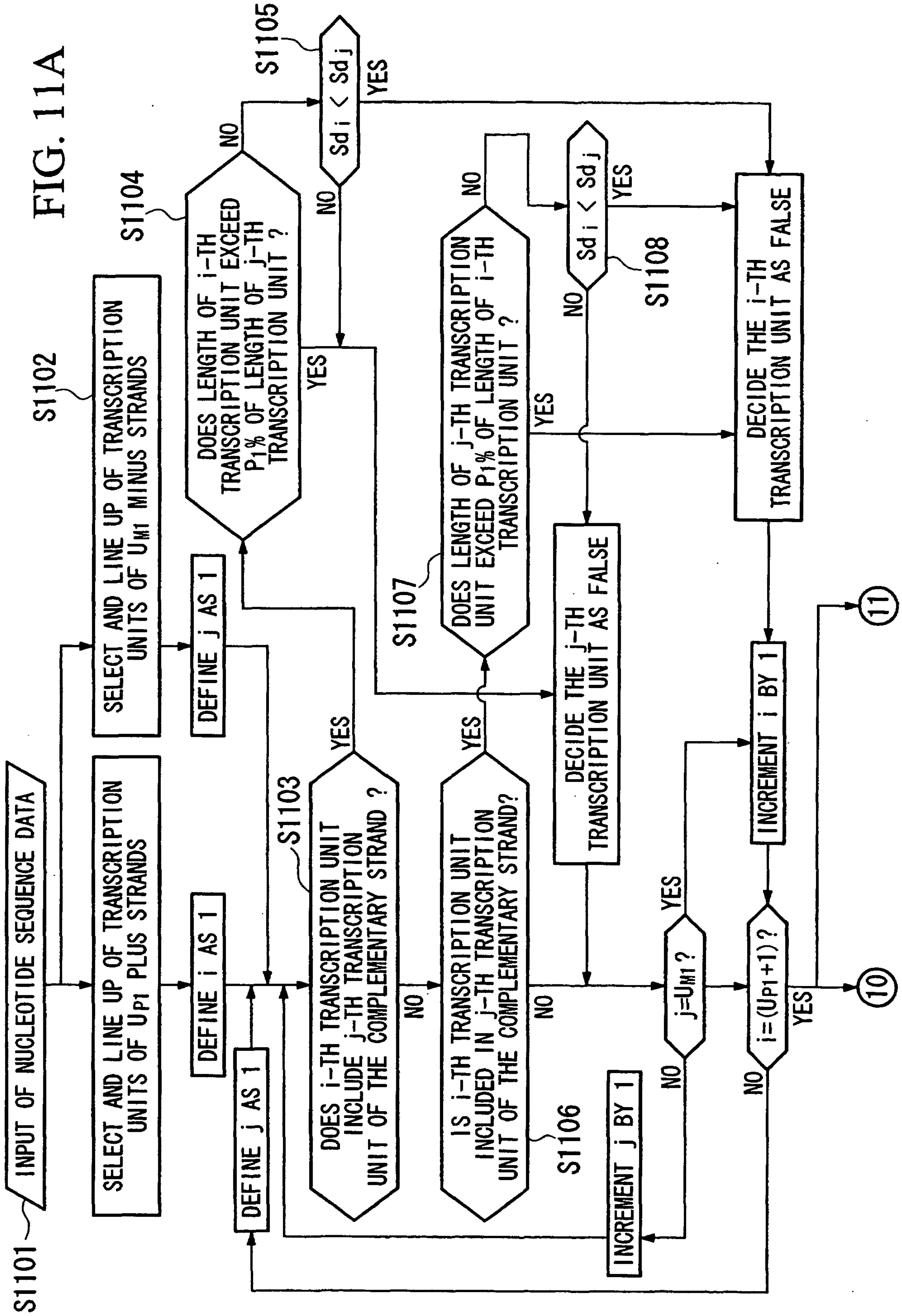
S1101
INPUT OF NUCLEOTIDE SEQUENCE DATA
S1102
SELECT AND LINE UP OF TRANSCRIPTION UNITS OF UP1 PLUS STRANDS
SELECT AND LINE UP OF TRANSCRIPTION UNITS OF UM1 MINUS STRANDS
DEFINE i AS 1
DEFINE j AS 1
DEFINE j AS 1
S1103
DOES i-TH TRANSCRIPTION UNIT INCLUDE j-TH TRANSCRIPTION UNIT OF THE COMPLEMENTARY STRAND ?
S1104
DOES LENGTH OF i-TH TRANSCRIPTION UNIT EXCEED P1% OF LENGTH OF j-TH TRANSCRIPTION UNIT ?
S1105
Sd i < Sd j
S1106
IS i-TH TRANSCRIPTION UNIT INCLUDED IN j-TH TRANSCRIPTION UNIT OF THE COMPLEMENTARY STRAND?
S1107
DOES LENGTH OF j-TH TRANSCRIPTION UNIT EXCEED P1% OF LENGTH OF i-TH TRANSCRIPTION UNIT ?
S1108
Sd i < Sd j
DECIDE THE j-TH TRANSCRIPTION UNIT AS FALSE
DECIDE THE i-TH TRANSCRIPTION UNIT AS FALSE
j=UM1 ?
INCREMENT j BY 1
INCREMENT i BY 1
i=(UP1+1) ?
YES
NO
10
11

FIG. 11B

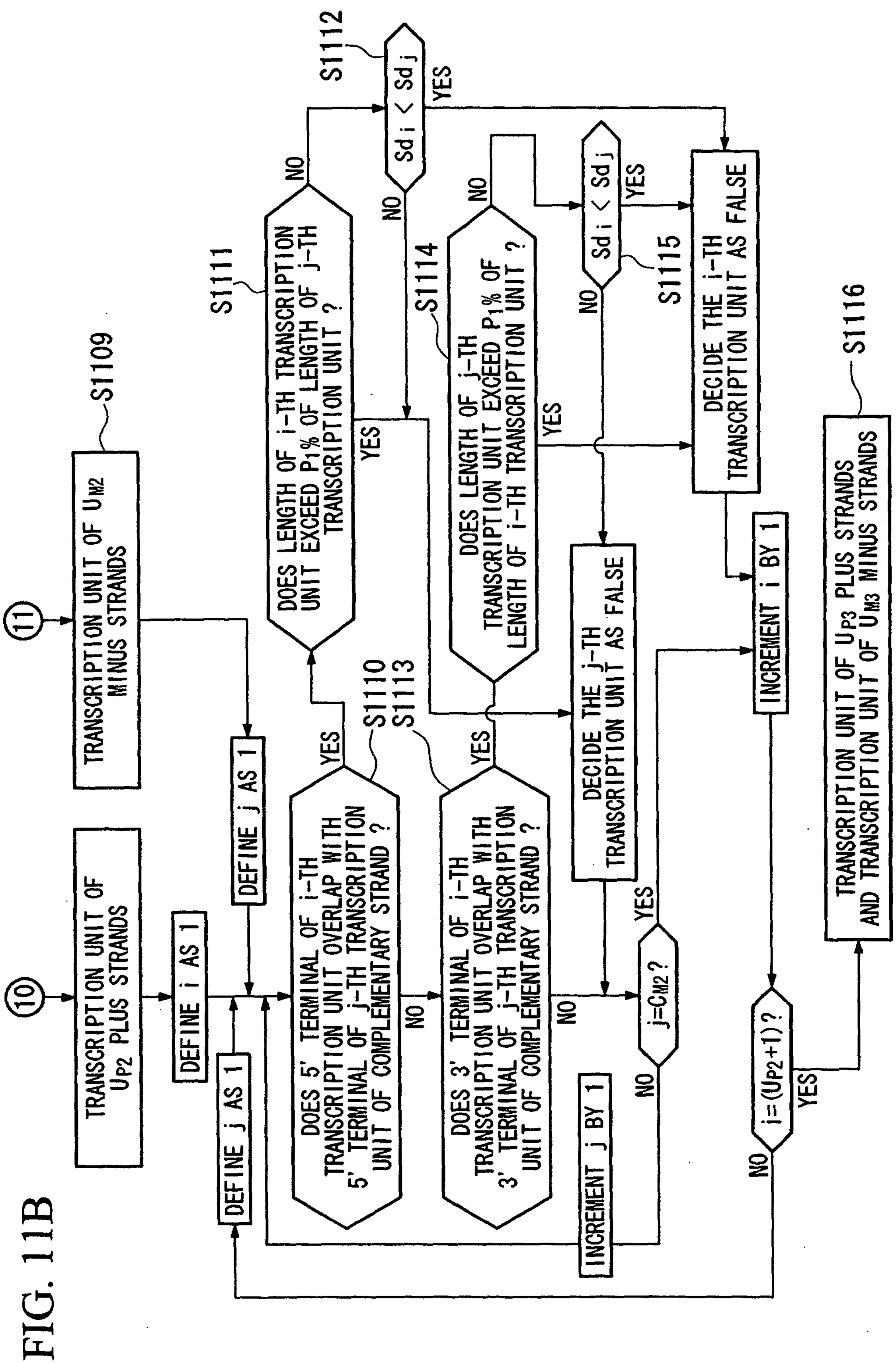
10
TRANSCRIPTION UNIT OF $U_{P2}$ PLUS STRANDS
11
TRANSCRIPTION UNIT OF $U_{M2}$ MINUS STRANDS
S1109
DEFINE i AS 1
DEFINE j AS 1
DEFINE j AS 1
DOES 5' TERMINAL OF i-TH TRANSCRIPTION UNIT OVERLAP WITH 5' TERMINAL OF j-TH TRANSCRIPTION UNIT OF COMPLEMENTARY STRAND ?
S1110
YES
NO
DOES 3' TERMINAL OF i-TH TRANSCRIPTION UNIT OVERLAP WITH 3' TERMINAL OF j-TH TRANSCRIPTION UNIT OF COMPLEMENTARY STRAND ?
S1113
YES
NO
DOES LENGTH OF i-TH TRANSCRIPTION UNIT EXCEED $P_1$% OF LENGTH OF j-TH TRANSCRIPTION UNIT ?
S1111
YES
NO
Sd i < Sd j
S1112
YES
NO
DOES LENGTH OF j-TH TRANSCRIPTION UNIT EXCEED $P_1$% OF LENGTH OF i-TH TRANSCRIPTION UNIT ?
S1114
YES
NO
Sd i < Sd j
S1115
YES
NO
DECIDE THE i-TH TRANSCRIPTION UNIT AS FALSE
DECIDE THE j-TH TRANSCRIPTION UNIT AS FALSE
j=$C_{M2}$ ?
YES
NO
INCREMENT j BY 1
INCREMENT i BY 1
i=($U_{P2}$+1) ?
YES
NO
TRANSCRIPTION UNIT OF $U_{P3}$ PLUS STRANDS AND TRANSCRIPTION UNIT OF $U_{M3}$ MINUS STRANDS
S1116

FIG. 12

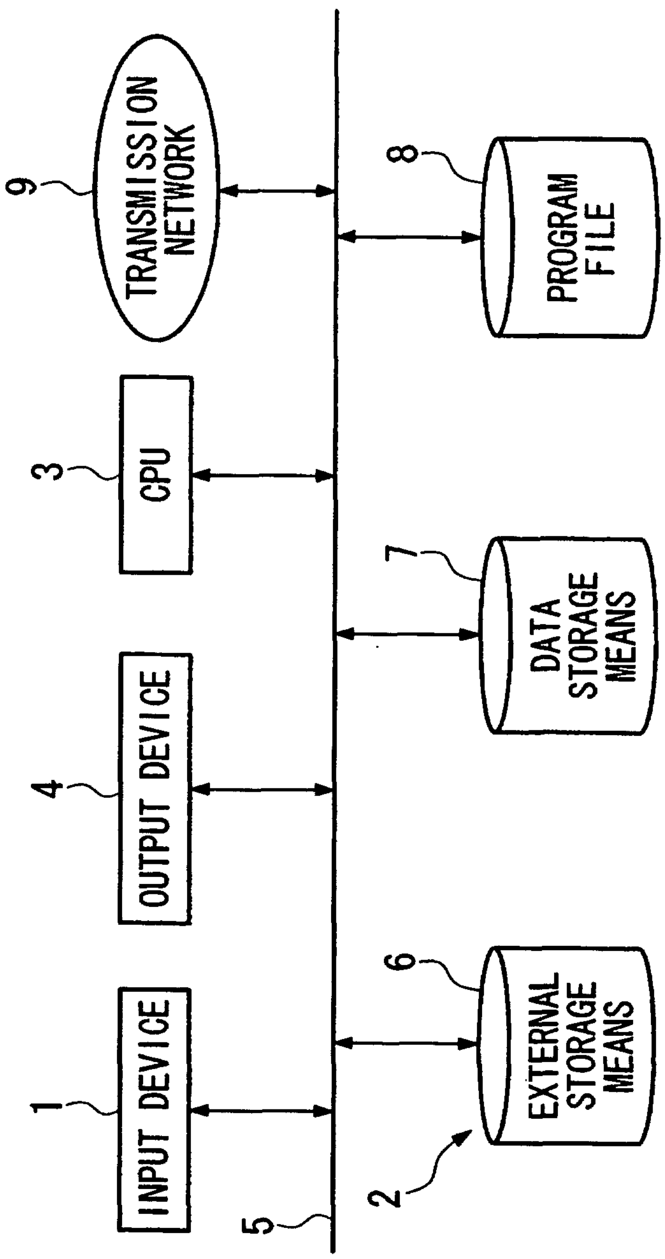
INPUT DEVICE
1
OUTPUT DEVICE
4
CPU
3
TRANSMISSION NETWORK
9
EXTERNAL STORAGE MEANS
6
2
DATA STORAGE MEANS
7
PROGRAM FILE
8
5

FIG. 13

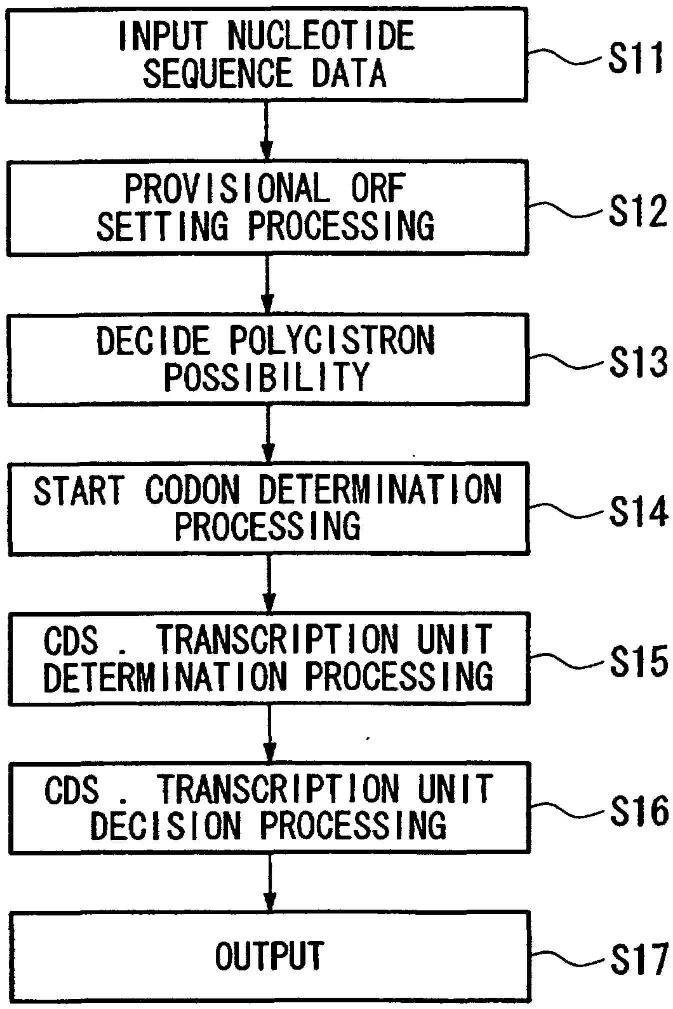
INPUT NUCLEOTIDE SEQUENCE DATA
S11
PROVISIONAL ORF SETTING PROCESSING
S12
DECIDE POLYCISTRON POSSIBILITY
S13
START CODON DETERMINATION PROCESSING
S14
CDS . TRANSCRIPTION UNIT DETERMINATION PROCESSING
S15
CDS . TRANSCRIPTION UNIT DECISION PROCESSING
S16
OUTPUT
S17

FIG. 14

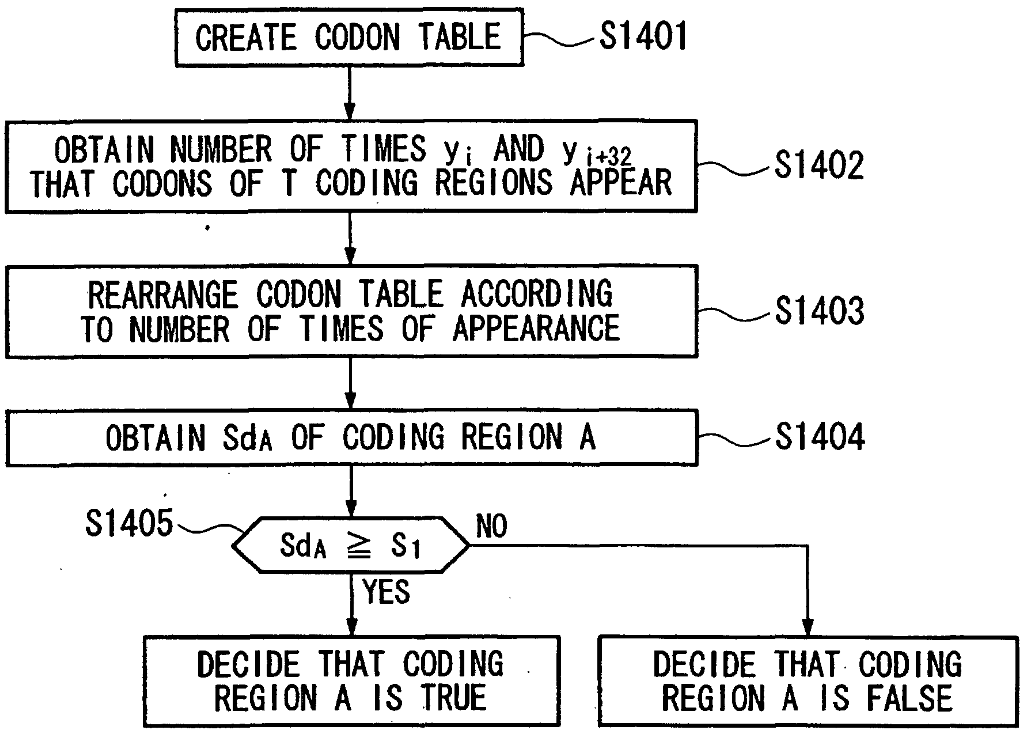
CREATE CODON TABLE
S1401
OBTAIN NUMBER OF TIMES $y_i$ AND $y_{i+32}$ THAT CODONS OF T CODING REGIONS APPEAR
S1402
REARRANGE CODON TABLE ACCORDING TO NUMBER OF TIMES OF APPEARANCE
S1403
OBTAIN $Sd_A$ OF CODING REGION A
S1404
S1405
$Sd_A \geqq S_1$
NO
YES
DECIDE THAT CODING REGION A IS TRUE
DECIDE THAT CODING REGION A IS FALSE

FIG. 15

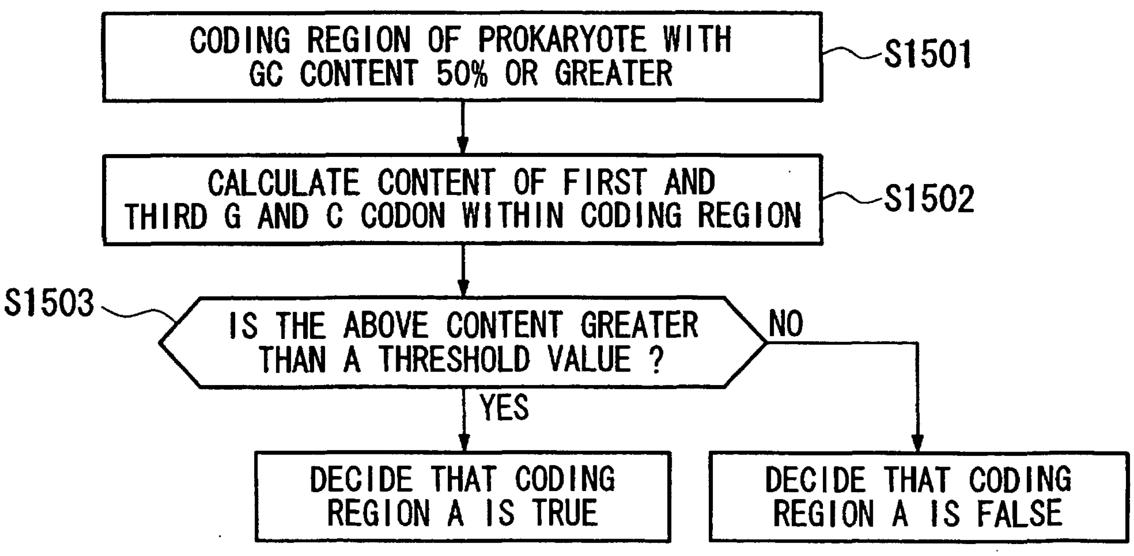
CODING REGION OF PROKARYOTE WITH GC CONTENT 50% OR GREATER
S1501
CALCULATE CONTENT OF FIRST AND THIRD G AND C CODON WITHIN CODING REGION
S1502
S1503
IS THE ABOVE CONTENT GREATER THAN A THRESHOLD VALUE ?
NO
YES
DECIDE THAT CODING REGION A IS TRUE
DECIDE THAT CODING REGION A IS FALSE

## INTERNATIONAL SEARCH REPORT

International application No. PCT/JP02/10851

A. CLASSIFICATION OF SUBJECT MATTER
Int.Cl[7] C12N15/00, G06F17/30

According to International Patent Classification (IPC) or to both national classification and IPC

B. FIELDS SEARCHED

Minimum documentation searched (classification system followed by classification symbols)
Int.Cl[7] C12N15/00, G06F17/30

Documentation searched other than minimum documentation to the extent that such documents are included in the fields searched

Electronic data base consulted during the international search (name of data base and, where practicable, search terms used)
MEDLINE, CAPLUS, BIOSIS, WPIDS(STN)

C. DOCUMENTS CONSIDERED TO BE RELEVANT

| Category* | Citation of document, with indication, where appropriate, of the relevant passages | Relevant to claim No. |
|---|---|---|
| P,X | Suzek B.E. et al., "A probabilistic method for identifying start codons in bacterial genomes", Bioinformatics, (2001 Dec.), Vol.17, No.12, Pages 1123 to 1130, abstract; page 1123, right column, Par. No. [0003]; page 1124, left column, Par. No. [0003] | 1-6,25-29 |
| X<br>Y | Arques, Didier G., et al., "A complementary circular code in the protein coding genes", Journal of Theoretical Biology, (1996), Vol.182, No.1, pages 45 to 58; page 48 "3.2 Complementary Property", tables 1(a), 2(b) | 7-13,30-36<br>18-24,41-47 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 January, 2003 (13.01.03) | 28 January, 2003 (28.01.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

**INTERNATIONAL SEARCH REPORT**

International application No. PCT/JP02/10851

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | Shields D.C. et al., "GCWIND: a microcomputer program for identifying open reading frames according to codon positional G+C content", Computer Applications in the Biosciences, (1992), Vol.8, No.5, pages 521 to 523; page 522, left column, Par. No. [0002] | 14-17,37-40<br>18-29,41-47 |
| Y | Frishman, D. et al., "Combining diverse evidence for gene recognition in completely sequenced bacterial genomes", Nucleic Acids Res., (1998), Vol.26, No.12, pages 2941 to 2947; page 2941, left column, Par. No. [0002] to right column, Par. No. [0002]; page 2942, left column, Par. No. [0005] to right column, Par. No. [0005]; table 1 | 1-6,18-29, 41-47 |
| Y | Frishman, D. et al., "Starts of bacterial genes: Estimating the reliability of computer predictions", Gene, (1999), Vol.234, No.2, pages 257 to 265; page 257, left column, Par. No. [0001] to page 258, left column, Par. No. [0002]; page 258, right column, Par. No. [0004]; Fig. 3 | 1-6,18-29, 41-47 |
| Y | Salzberg, S.L., et al., "Microbial gene indentification using interpolated Markov models", Nucleic Acids Res., (1998), Vol.26, No.2, pages 544 to 548, abstract; page 547, left column, Par. No. [0003] | 1-6,18-29, 41-47 |

Form PCT/ISA/210 (continuation of second sheet) (July 1998)

## INTERNATIONAL SEARCH REPORT

International application No.
PCT/JP02/10851

**Box I Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:
(See extra sheet)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1998)

**INTERNATIONAL SEARCH REPORT**

International application No.
PCT/JP02/10851

Continuation of Box No.II of continuation of first sheet(1)

Claims 1 to 6 and 25 to 29 relate to methods and programs of identifying a prokaryotic gene structure based on the presence/absence of an overlap of presumed open reading frames (ORF) and a ribosome binding site around a presumed start codon.

Claims 7 to 13 and 30 to 36 relate to methods and programs of judging the truth or falsehood of the above presumption by searching for a codon combination showing a high appearance frequency of the codon but a low appearance frequency of the complementary sequence and comparing the appearance frequencies of the codons in the presumed translational domain.

Claims 14 to 17 and 37 to 40 relate to methods and programs for identifying a prokaryotic gene structure based on GC contents in a gene.

Among these three invention concepts, no technical relevancy is observed except being means to be used for solving a well known problem of identifying and confirming a prokaryotic gene structure. Thus, these inventive concepts have no novel technical feature in common.

Claims 18 to 24 and 41 to 47 depend on any of these three inventive concepts in an alternative form and these claims also involve the three inventive concepts, thereby failing to fulfill the requirement of unity of invention.

Such being the case, it is recognized that this international application fails to fulfill the requirement of unity of invention under Article 13 of the Law (PCT Rule 13.1, 13.2 and 13.3).

Form PCT/ISA/210 (extra sheet) (July 1998)